(19) Europäisches Patentamt
European Patent Office
Office européen des brevets

(11) **EP 4 291 253 B1**

(12) **EUROPÄISCHE PATENTSCHRIFT**

(45) Veröffentlichungstag und Bekanntmachung des Hinweises auf die Patenterteilung:
**10.09.2025 Patentblatt 2025/37**

(21) Anmeldenummer: **22705771.8**

(22) Anmeldetag: **10.02.2022**

(51) Internationale Patentklassifikation (IPC):
**A61L 2/18** (2006.01)   **A61G 9/00** (2006.01)
**A61G 9/02** (2006.01)   **A61L 2/07** (2006.01)

(52) Gemeinsame Patentklassifikation (CPC):
**A61L 2/18; A61G 9/02; A61L 2/07; A61L 2/183; A61L 2/186;** A61G 2203/30; A61G 2203/34; A61L 2202/14; A61L 2202/17; A61L 2202/23

(86) Internationale Anmeldenummer:
**PCT/EP2022/053202**

(87) Internationale Veröffentlichungsnummer:
**WO 2022/171721 (18.08.2022 Gazette 2022/33)**

(54) **VERFAHREN ZUR BEHANDLUNG VON BEHÄLTERN FÜR MENSCHLICHE AUSSCHEIDUNGEN**

METHOD FOR TREATING CONTAINERS FOR HUMAN EXCRETIONS

MÉTHODE DE TRAITEMENT DE RÉCIPIENTS POUR DÉJECTIONS HUMAINES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.02.2021 DE 102021201293**

(43) Veröffentlichungstag der Anmeldung:
**20.12.2023 Patentblatt 2023/51**

(73) Patentinhaber: **MEIKO Maschinenbau GmbH & Co. KG**
**77652 Offenburg (DE)**

(72) Erfinder:
• **GAUS, Bruno**
**77654 Offenburg (DE)**
• **HEPPNER, Juergen**
**77746 Schutterwald (DE)**
• **NAEGER, Thomas**
**77652 Offenburg (DE)**
• **PEUKERT, Thomas**
**77815 Bühl (Baden) (DE)**
• **RINGWALD, Bernd**
**77799 Ortenberg (DE)**
• **SCHNEIDER, Vera**
**77652 Offenburg (DE)**

(74) Vertreter: **Altmann Stößel Dick Patentanwälte PartG mbB**
**Theodor-Heuss-Anlage 2**
**68165 Mannheim (DE)**

(56) Entgegenhaltungen:
EP-A2- 0 978 267     DE-A1- 102014 003 954
US-A1- 2013 319 460

**Beschreibung**

Technisches Gebiet

Die vorliegende Erfindung betrifft ein Verfahren zur Behandlung mindestens eines Behälters für menschliche Ausscheidungen und ein Reinigungs- und Desinfektionsgerät zur Behandlung mindestens eines Behälters für menschliche Ausscheidungen. Derartige Reinigungs- und Desinfektionsgeräte und Verfahren werden beispielsweise im Krankenhaus- oder Pflegebereich eingesetzt, um Behälter wie beispielsweise Steckbecken, Urinflaschen, Bettpfannen, Nachtgeschirr, Nierenschalen, Waschschüsseln, Stuhleimer, Messbecher oder andere Behälter zu reinigen, welche zur Aufnahme menschlicher oder tierischer Ausscheidungen geeignet sind, insbesondere mit einem Volumen von mindestens 100 ml, insbesondere mindestens 500 ml oder sogar von mindestens 1000 ml, beispielsweise 1000-5000 ml.

Technischer Hintergrund

Aus dem Stand der Technik sind eine Vielzahl von Reinigungsgeräten und Reinigungsverfahren zur Behandlung von Behältern für menschliche Ausscheidungen bekannt. Die zu reinigenden Behälter können größere Flüssigkeitsmengen oder Mengen an Feststoffabfällen enthalten, welche üblicherweise während der Reinigung entsorgt werden müssen. Herkömmliche Spülmaschinen sind somit für die Reinigung derartiger Behälter in der Regel nicht geeignet. Zudem können derartige Behälter infektiöse Abfälle enthalten oder anderweitig kontaminiert sein, so dass, neben einer Entleerung, auch üblicherweise eine Desinfektion erforderlich ist. Derartige Reinigungsgeräte zur Behandlung von Behältern für menschliche Ausscheidungen werden dementsprechend häufig auch als Reinigungs- und Desinfektionsgeräte (RDG) bezeichnet. Neben den genannten Behältern sind diese grundsätzlich auch zur Reinigung anderer medizinischer Gegenstände geeignet, wie sie beispielsweise in Krankenhäusern oder Altenpflegeeinrichtungen eingesetzt werden. Üblicherweise besteht das Reinigungsgut jedoch aus Urinflaschen, Steckbecken, Nierenschalen, Waschschüsseln oder ähnlichen Behältern, deren Reinigung die Entsorgung größerer Abfallmengen mit sich bringen kann.

Beispiele von Reinigungs- und Desinfektionsgeräten sind in DE 103 48 344 A1 oder auch in WO 2013/037723 A1 gezeigt. Auf die dort beschriebenen Konstruktionen von Reinigungs- und Desinfektionsgeräten kann exemplarisch grundsätzlich auch im Rahmen der vorliegenden Erfindung Bezug genommen werden, wobei die dargestellten Reinigungs- und Desinfektionsgeräte im Rahmen der vorliegenden Erfindung modifiziert und/oder ergänzt eingesetzt werden können. Auch andere Ausgestaltungen sind jedoch grundsätzlich möglich. So umfasst beispielsweise das in DE 103 48 344 A1 beschriebene Reinigungs- und Desinfektionsgerät eine Vorrichtung zur Rückkühlung von Reinigungsgut. Dabei erfolgt innerhalb einer Kammer ein Abspülen des Reinigungsguts, woran sich ein Vorreinigungsspülschritt anschließt. Danach wird das in der Kammer enthaltene Reinigungsgut mit einem einen Klarspülerzusatz enthaltenden Wasser endgereinigt, bevor ein Desinfektionsschritt des Reinigungsguts in der Kammer durch Einleitung von Wasserdampf in diese Kammer erfolgt. In die mit Wasserdampf befüllte Kammer wird zwangsweise Luft bei geschlossener Tür eingebracht, wodurch ein Niederschlag von Wasserdampf innerhalb der Kammer sowie eine Abkühlung und eine Trocknung des in der Kammer enthaltenen Reinigungsguts bewirkt wird.

In einem Reinigungs- und Desinfektionsgerät läuft somit üblicherweise zur Behandlung des Reinigungsguts ein Programm ab, das üblicherweise in einer Steuerung des Geräts hinterlegt ist. Nach der Beladung des RDGs wählt üblicherweise ein Anwender ein Reinigungsprogramm aus und startet dieses üblicherweise durch Betätigen einer entsprechenden Taste, beispielsweise an einer Folientastatur. Der Anwender muss dabei in der Regel subjektiv entscheiden, welche Art und/oder Stärke der Verschmutzung und/oder Verkeimung an den zu reinigenden Gegenständen vorliegt. Hierbei kann jedoch grundsätzlich eine fehlerhafte Auswahl eines Reinigungsprogramms dazu führen, dass das Reinigungsgut nach der Behandlung noch mit Keimen belastet ist.

Eine weitere technische Herausforderung in vielen Reinigungs- und Desinfektionsgeräten besteht darin, dass die Dampfdesinfektion mit Dampf unter bestimmten Umständen einer Keimbelastung nicht ausreicht, um ausreichende Keimabtötung bestimmter Keime zu gewährleisten. Zwar können den zuvor beim Waschen eingesetzten Reinigungsmitteln grundsätzlich Desinfektionsmittel beigemischt werden, oder diese Reinigungsmittel haben selbst eine keimabtötende Wirkung. Dennoch wäre es wünschenswert, eine universellere und dennoch zuverlässigere Desinfektionswirkung in Reinigungs- und Desinfektionsgeräten zu gewährleisten. So sind insbesondere im Bereich von Krankenhäusern und Pflegeeinrichtungen Keime bekannt, die mit herkömmlichen Desinfektionsmethoden nicht oder nur unzureichend abgetötet werden. In diesem Zusammenhang sind beispielsweise Bakterien vom Typ Clostridioides difficile (*C. difficile*) zu nennen. Auch andere Keime können diesbezüglich jedoch auch eine Rolle spielen. Insbesondere ist auch eine sporizide Wirkung mit den bekannten Verfahren zumeist nicht oder nicht vollständig erreichbar. Es wäre deshalb wünschenswert neben der abreichernden Reinigung mir einem Reinigungsfluid einen valide sporizid wirksamen Verfahrensbaustein zur Verfügung zu stellen um eventuell auf dem Spülgut zurückgebliebene Sporen zu inaktivieren.

Weiterhin besteht eine technische Herausforderung darin, dass viele chemische Desinfektionswirkstoffe in der Praxis nur schwer zu handhaben sind. So können diese beispielsweise, entsprechend der Natur der Desinfektion, äußerst aggressiv, korrosiv, brandgefährlich oder instabil sein.

Grundsätzlich sind aus anderen Bereichen der Naturwissenschaften und Technik Desinfektionsmittel und Desinfektionsverfahren in zahlreichen verschiedenen Varianten bekannt. Lediglich exemplarisch wird auf die Desinfektionsverfahren verwiesen, welche in WO 2019/219220 A1 und in WO 2019/219959 A1 beschrieben sind und bei denen *in situ* ein Desinfektionswirkstoff gebildet wird. Die dort beschriebenen Desinfektionsverfahren werden insbesondere im Bereich der Hautdesinfektion, der Desinfektion von Verpackungen und der Desinfektion von Schläuchen von Endoskopen eingesetzt.

US 2018/0058053 A1 beschreibt eine Toilettenanlage mit verschiedenen Ausführungsformen eines Reinigungssystems. Die Toilettenanlage hat eine Toilettenschüssel, einen Toilettenbehälter, ein Spülventil, eine Randeinlassöffnung und einen Randdurchlauf, der sich von einem Auslass des Spülventils zur Randeinlassöffnung erstreckt. Das Reinigungssystem umfasst einen Vorratsbehälter zur Aufnahme eines flüssigen Reinigungsmittels mit einer Auslassöffnung, die in Fluidverbindung mit dem Innenraum des Vorratsbehälters steht und ein Gehäuse zur Aufnahme des Vorratsbehälters. Das Reinigungssystem hat weiterhin eine Zufuhrleitung, die in Fluidverbindung mit dem Innenraum des Vorratsbehälters steht, eine Durchflusssteuerungsvorrichtung, die in der Lage ist, den Durchfluss durch die Zufuhrleitung zu steuern und ein Steuersystem, das durch ein Aktuatormerkmal aktiviert werden kann. Bei Aktivierung des Aktuatormerkmals ist das Steuersystem so ausgelegt, dass es einen Reinigungszyklus einleitet, indem es die Durchflusssteuervorrichtung für eine erste Zeitspanne betätigt, die ausreicht, um eine Dosis eines flüssigen Reinigungsmittels aus der Zufuhrleitung in einen Innenraum des Spülventils in einer geschlossenen Position zu liefern, wobei das Spülventil für die Lieferung von Fluid an die Randeinlassöffnung eingerichtet ist, und weiter das Spülventil zum Öffnen betätigt, um Spülwasser einzuführen, um die Dosis des flüssigen Reinigungsmittels durch die Randeinlassöffnung in die Toilettenschüssel einzubringen.

WO 2019/036828 A1 offenbart eine dampfreinigende Toilette mit Spülung umfassend: eine Toilette mit Spülung, einen Toilettentank und eine Toilettenabdeckung. Innerhalb des Toilettentanks ist ein Wasserspeichertank und ein Dampfgeneratortank vorgesehen, der gegenüberliegend und getrennt von dem Wasserspeichertank angeordnet ist. Ein Dampfgenerator ist innerhalb des Dampfgeneratortanks vorgesehen und umfasst: einen Wassereinlasstank, ein Heizrohr, einen Dampftank und eine Steueranordnung. Am Wassereinlasstank ist ein Wasserzuführrohr und am Dampftank ein Dampfauslassrohr vorgesehen. Der Toilettenrand am oberen Abschnitt des Toilettenkörpers der Spültoilette ist mit einem Dampfeinlassrohr zum Zirkulieren von Dampf aus dem Dampfauslassrohr innerhalb einer Dampfzirkulationsleitung versehen.

US 2017/0260728 A1 beschreibt eine Toilettenvorrichtung zum Bereitstellen eines intelligenten Bidetdosiersystems. Es wird eine dynamische Abgabe gemäß der Position des Bidet-Stabs, der Art der abgegebenen Substanz, Benutzerpräferenzen, Benutzergeschlecht und Benutzeridentifikation beschrieben. Reinigungsmittel, Tenside, Feuchtigkeitsmittel, Medikamente, Deodorants und Duftstoffe werden durch einen Bidet-Stab an einen Benutzer abgegeben und sind in Reservoirs vorgehalten, die in einem Tankbereich der Toilette umfasst sind. In weiteren Ausführungsformen wird die Meldung der Niveaus der Reservoirsubstanzen automatisiert.

Aufgabe der Erfindung

In Anbetracht der oben beschriebenen technischen Herausforderungen von herkömmlichen Reinigungs- und Desinfektionsgeräten wäre es daher wünschenswert, ein Verfahren zur Behandlung mindestens eines Behälters für menschliche Ausscheidungen und ein Reinigungs- und Desinfektionsgerät zur Behandlung mindestens eines Behälters für menschliche Ausscheidungen bereitzustellen, welche die Nachteile bekannter Vorrichtungen und Verfahren der genannten Art zumindest weitgehend vermeiden. Insbesondere soll eine einfache, universelle und zumindest weitgehend von einer Erfahrung des Bedienpersonals unabhängige und dennoch sichere Desinfektionswirkung erzielt werden.

Allgemeine Beschreibung der Erfindung

Diese Aufgabe wird adressiert durch ein Verfahren zur Behandlung mindestens eines Behälters für menschliche Ausscheidungen und ein Reinigungs- und Desinfektionsgerät zur Behandlung mindestens eines Behälters für menschliche Ausscheidungen mit den Merkmalen der unabhängigen Patentansprüche. Vorteilhafte Weiterbildungen, welche einzeln oder in beliebiger Kombination realisierbar sind, sind in den abhängigen Ansprüchen dargestellt.

Im Folgenden werden die Begriffe "haben", "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben den durch diese Begriffe eingeführten Merkmalen, keine weiteren Merkmale vorhanden sind, oder auf Situationen, in welchen ein oder mehrere weitere Merkmale vorhanden sind. Beispielsweise kann sich der Ausdruck "A hat B", "A weist B auf", "A umfasst B" oder "A schließt B ein" sowohl auf die Situation beziehen, in welcher, abgesehen von B, kein weiteres Element in A vorhanden ist (d.h. auf eine Situation, in welcher A ausschließlich aus B besteht), als auch auf die Situation, in welcher, zusätzlich zu B, ein oder mehrere weitere Elemente in A vorhanden sind, beispielsweise Element C, Elemente C und D oder sogar weitere Elemente.

Weiterhin wird darauf hingewiesen, dass die Begriffe "mindestens ein" und "ein oder mehrere" sowie grammatikalische Abwandlungen dieser Begriffe, wenn diese in Zusammenhang mit einem oder mehreren Elementen oder Merkmalen verwendet werden und ausdrücken sollen, dass das Element oder Merkmal einfach oder mehrfach vorgesehen sein kann, in der Regel lediglich einmalig verwendet werden, beispielsweise bei der erstmaligen Einführung des Merkmals oder Elementes. Bei einer nachfolgenden erneuten Erwähnung des Merkmals oder Elementes wird der entsprechende Begriff "mindestens ein" oder "ein oder mehrere" in der Regel nicht mehr verwendet, ohne Einschränkung der Möglichkeit, dass das Merkmal oder Element einfach oder mehrfach vorgesehen sein kann.

Weiterhin werden im Folgenden die Begriffe "vorzugsweise", "insbesondere", "beispielsweise" oder ähnliche Begriffe in Verbindung mit optionalen Merkmalen verwendet, ohne dass alternative Ausführungsformen hierdurch beschränkt werden. So sind Merkmale, welche durch diese Begriffe eingeleitet werden, optionale Merkmale, und es ist nicht beabsichtigt, durch diese Merkmale den Schutzumfang der Ansprüche und insbesondere der unabhängigen Ansprüche einzuschränken. So kann die Erfindung, wie der Fachmann erkennen wird, auch unter Verwendung anderer Ausgestaltungen durchgeführt werden. In ähnlicher Weise werden Merkmale, welche durch "in einer Ausführungsform der Erfindung" oder durch "in einem Ausführungsbeispiel der Erfindung" eingeleitet werden, als optionale Merkmale verstanden, ohne dass hierdurch alternative Ausgestaltungen oder der Schutzumfang der unabhängigen Ansprüche eingeschränkt werden soll. Weiterhin sollen durch diese einleitenden Ausdrücke sämtliche Möglichkeiten, die hierdurch eingeleiteten Merkmale mit anderen Merkmalen zu kombinieren, seien es optionale oder nicht-optionale Merkmale, unangetastet bleiben.

In einem ersten Aspekt der vorliegenden Erfindung wird ein Verfahren zur Behandlung mindestens eines Behälters für menschliche Ausscheidungen vorgeschlagen. Das Verfahren umfasst die nachfolgend näher beschriebenen und definierten Schritte, welche vorzugsweise in der genannten Reihenfolge durchgeführt werden können. Grundsätzlich ist jedoch auch eine andere Reihenfolge möglich. Weiterhin ist es möglich, zwei oder mehr der beschriebenen Verfahrensschritte gleichzeitig oder zeitlich überlappend durchzuführen. Zudem können einer, mehrere oder alle der genannten Verfahrensschritte, einzeln oder auch in Gruppen, auch einfach oder mehrfach wiederholt durchgeführt werden. Zudem kann das Verfahren zusätzliche, nicht genannte Verfahrensschritte umfassen.

Das Verfahren umfasst die folgenden Schritte:

a. mindestens einen Entleerungsschritt, umfassend ein Entleeren des Behälterinhalts innerhalb mindestens einer Reinigungskammer in mindestens einen Abfluss, wobei der Abfluss mindestens einen Geruchsverschluss umfasst;

b. mindestens einen Waschschritt, umfassend mindestens eine Beaufschlagung des Behälters mit mindestens einer Reinigungsflüssigkeit in der Reinigungskammer; und

c. mindestens einen Desinfektionsschritt, umfassend mindestens ein Mischen mindestens zweier reaktiver Komponenten zur Erzeugung mindestens eines Desinfektionswirkstoffs und Beaufschlagung des Behälters mit dem Desinfektionswirkstoff.

Das Verfahren kann allgemein in mindestens einem Reinigungs- und Desinfektionsgerät durchgeführt werden, insbesondere gemäß einer oder mehreren der nachfolgend noch näher beschriebenen Ausgestaltungen. Der Begriff "Reinigungs- und Desinfektionsgerät", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche eingerichtet ist, um eine Behandlung des Behälters, beinhaltend mindestens eine Reinigung und mindestens eine Desinfektion, durchzuführen. Die mindestens eine Reinigung und die mindestens eine Desinfektion können insbesondere in getrennten Programmschritten eines Programmablaufs erfolgen. Beispielsweise können das Verfahren und/oder das Reinigungs- und Desinfektionsgerät ein Programm umfassen bzw. unterstützen, in welchem mindestens ein Reinigungsschritt und mindestens ein Desinfektionsschritt als getrennte Programmschritte vorgesehen sind. Insbesondere kann das Reinigungs- und Desinfektionsgerät einen Steckbeckenspüler umfassen oder als Steckbeckenspüler ausgestaltet sein oder eingerichtet sein.

Der Begriff "Behandlung", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Reinigung beziehen, welche, wie nachfolgend noch näher beschrieben wird, eine Beaufschlagung des Behälters mit mindestens einem Reinigungsfluid beinhaltet. Die Reinigung kann insbesondere derart ausgestaltet werden, dass diese den Behälter zumindest weitgehend von anhaftenden Verunreinigungen befreit. Weiterhin beinhaltet die Behandlung, zusätzlich zu einer Reinigung, auch eine Desinfektion.

Der Begriff "Desinfektion" sowie grammatikalische Abwandlungen davon, wie sie hier verwendet werden, sind ebenfalls weite Begriffe, denen ihre gewöhnliche und gängige Bedeutung beigemessen werden soll, wie der Fachmann sie versteht. Die Begriffe sind nicht beschränkt auf eine spezielle oder angepasste Bedeutung. Die Begriffe können sich, ohne Beschränkung, insbesondere auf eine Keimreduktion beziehen. Allgemein kann die Desinfektion ein Verfahren beinhalten, welches eine Keimreduktion in einem festgelegten Testverfahren mit bestimmten Prüfkörpern um einen Faktor von mindestens $10^{-5}$ bewirkt, bei dem also beispielsweise von ursprünglich 1.000.000 vermehrungsfähigen Keimen, so genannten koloniebildende Einheiten (KbE), nicht mehr als zehn überleben. Hierbei können zur Prüfung der Desinfektionswirkung und Keimabtötung beispielsweise normierte Testverfahren und/oder normierte KbE oder Testorganismen verwendet werden, wie beispielsweise in der Europäischen Norm CEN TC 216 WG1 und WG3 beschrieben.

Die Desinfektionswirkung kann beispielsweise überprüft und/oder überwacht werden, beispielsweise stichprobenartig. Alternativ oder zusätzlich können jedoch auch die Verfahrensbedingungen überwacht werden, beispielsweise durch entsprechende Sensoren und/oder eine Steuerung. Die Verfahrensbedingungen können so eingestellt werden, dass sich die gewünschte Desinfektionswirkung einstellt. So können beispielsweise, um die gewünschte Desinfektion zu erreichen, technische Bedingungen einzuhalten sein, beispielsweise bestimmte Bedingungen ausgewählt aus der Gruppe bestehend aus Temperaturprofilen, Wärmeäquivalenten, Konzentrationen chemischer Wirkstoffe und Einwirkdauern. Diese technischen Bedingungen zur Erzielung der Desinfektionswirkung können beispielsweise auch normiert sein, beispielsweise in Normen, die Mindeststandards und Testverfahren für bestimmte Gerätegruppen und/oder Anwendungen vorgeben.

Eine über die Desinfektion hinausgehende Keimreduktion wird auch als Sterilisation bezeichnet. Bei einer Sterilisation werden typischerweise Keimreduktionen von mindestens $10^{-6}$ verlangt.

Die Keimreduktion kann beispielsweise durch eine thermische Behandlung und/oder durch eine chemische Behandlung des mindestens einen Behälters erfolgen, beispielsweise durch eine Behandlung mit mindestens einem Desinfektionswirkstoff, beispielsweise einer desinfizierenden flüssigen Verbindung, einem desinfizierenden Gas und/oder einem desinfizierenden Gas, beispielsweise Dampf. Die Desinfektion kann optional bis hin zu einer Sterilisation des Behälters führen.

Der Begriff "Behälter für menschliche Ausscheidungen", wie er hier verwendet wird, ist ebenfalls ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf einen Behälter beziehen, welcher mindestens einen Aufnahmebereich aufweist, beispielsweise einen Hohlraum oder eine Vertiefung, in welchem eine größere Menge an menschlichen oder auch tierischen Ausscheidungen aufnehmbar ist, beispielsweise eine Menge von mindestens 50 ml, insbesondere mindestens 100 ml, vorzugsweise mindestens 200 ml oder sogar mindestens 500 ml oder mindestens 1000 ml. Beispielsweise kann eine Maximalmenge aufnehmbar sein, welche im Bereich von 100 ml bis 5 l liegt, beispielsweise 1000 ml bis 5000 ml. Zusätzlich zu dem mindestens einen Aufnahmebereich, beispielsweise dem mindestens einen Hohlraum, kann der Behälter weiterhin mindestens eine Öffnung aufweisen. Diese Öffnung kann von vorneherein vorhanden sein und/oder kann auch zu einem späteren Zeitpunkt geschaffen werden. Weiterhin kann auch während und/oder vor der Behandlung mindestens eine Öffnung geschaffen werden, beispielsweise durch ein mechanisches Öffnen des Behälters und/oder durch ein Aufschneiden und/oder Aufreißen des Behälters, beispielsweise im Rahmen des vorgeschlagenen Verfahrens. Weiterhin kann die Öffnung eingerichtet sein, um reversibel oder irreversibel geöffnet und/oder verschlossen zu werden. Der Behälter kann mindestens eine Behälterwand aufweisen, welche starr oder auch verformbar, insbesondere flexibel, ausgestaltet sein kann. So kann der Behälter beispielsweise ein Gefäß mit einer starren Behälterwand, beispielsweise aus einem oder mehreren der Materialien Glas, Kunststoff, Keramik und Metall, aufweisen. Alternativ oder zusätzlich kann der Behälter auch mindestens eine verformbare Behälterwand, beispielsweise mindestens einen Folienbeutel, insbesondere einen Folienbeutel aus einem Kunststoffmaterial, aufweisen. So kann der Behälter für menschliche Ausscheidungen beispielsweise auch ganz oder teilweise als zunächst geschlossenes Behältnis in Gestalt eines Folienbeutels ausgebildet sein. Allgemein kann der Behälter beispielsweise ausgewählt sein aus der Gruppe bestehend aus: einer Urinflasche; einem Steckbecken; einer Bettpfanne; einem Urinbeutel; einer Nierenschale; einem Stuhleimer; einem Messbecher.

Wie oben ausgeführt, umfasst das Verfahren in Verfahrensschritt a. zunächst ein Entleeren des Behälterinhalts innerhalb mindestens einer Reinigungskammer, beispielsweise einer Reinigungskammer des Reinigungs- und Desinfektionsgeräts, in mindestens einen Abfluss, beispielsweise einen Abfluss des Reinigungs- und Desinfektionsgeräts. Hierbei kann das Entleeren insbesondere durch eine Änderung einer Lage und/oder Orientierung des Behälters erfolgen, beispielsweise indem der Inhalt des Behälters vollständig oder teilweise in den Abfluss gekippt wird. Diese Änderung der Lage und/oder Orientierung kann, wie später noch beschrieben wird, beispielsweise dadurch erfolgen, dass der Behälter an einer Tür des Reinigungs- und Desinfektionsgeräts befestigt ist, welche schwenkbar ist, so dass beim Schwenken die Lageänderung erfolgt. Das Entleeren kann somit durch die eigene Gewichtskraft des Behälterinhalts erfolgen. Alternativ oder zusätzlich ist auch eine andere Art der Orientierungsänderung möglich, beispielsweise durch eine separate Schwenkvorrichtung. Der Inhalt kann beispielsweise durch eine Öffnung des Behälters entleert werden. Alternativ oder zusätzlich kann jedoch, wie oben ausgeführt, auch eine andere Ausgestaltung vorgesehen sein, beispielsweise ein Aufschneiden eines Beutels, insbesondere ein automatisches Aufschneiden. In diesem Fall ist auch eine Lageänderung grundsätzlich nicht erforderlich, kann jedoch gleichwohl vorgesehen sein.

Der Begriff "Reinigungskammer", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine vollständig oder teilweise geschlossene Kammer beziehen, in welche der Behälter einbringbar ist und in welcher die Behandlung oder ein Teil der Behandlung des Behälters erfolgt. Insbesondere kann das Reinigungs- und Desinfektionsgerät als Einkammer-Reinigungsgerät ausgestaltet sein, wobei sämtliche Behandlungsschritte in derselben Reinigungskammer erfolgen. In der Reinigungskammer kann somit eine Beaufschlagung mit dem Reinigungsfluid erfolgen, sowie optional die Desinfektion des Behälters und/oder eine Entleerung des Behälters. Beispielsweise kann das Reinigungs- und Desinfektionsgerät eingerichtet sein, um mindestens ein Reinigungsprogramm mit mindestens den oben genannten Verfahrensschritten durchzuführen. Die Reinigungskammer kann insbesondere vollständig geschlossen sein und kann beispielsweise, wie unten noch näher ausgeführt, mindestens eine Tür aufweisen.

Wie oben ausgeführt, erfolgt das Entleeren des Behälterinhalts innerhalb mindestens einer Reinigungskammer in mindestens einen Abfluss. Der Begriff "Abfluss", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche eingerichtet ist, um flüssige Abfälle einer Entsorgung zuzuführen. Insbesondere kann der Abfluss mindestens eine Öffnung im Bodenbereich der Reinigungskammer umfassen. Weiterhin kann der Abfluss mindestens ein Abflussrohr umfassen, welches mit der Öffnung verbunden ist. Dieses Abflussrohr kann beispielsweise eine Mündung an der Öffnung in dem Boden der Reinigungskammer aufweisen. Diese Mündung kann insbesondere derart geöffnet sein, dass der Behälterinhalt ohne Hindernisse und allein aufgrund seiner Gewichtskraft in den Abfluss abfließen kann. Beispielsweise kann die Reinigungskammer im Bodenbereich zumindest teilweise trichterförmig und/oder geneigt auf den Abfluss zulaufen. Der Abfluss kann beispielsweise direkt oder indirekt mit einem gebäudeseitigen Entsorgungssystem und/oder einem Abwassersystem verbunden sein. Der Abfluss, insbesondere das Abflussrohr, kann insbesondere einen Querschnitt von mindestens 30 mm aufweisen, insbesondere von mindestens 50 mm, besonders bevorzugt von mindestens 70 mm oder sogar mindestens 100 mm. Auf diese Weise können auch die oben genannten Flüssigkeitsmengen durch den Abfluss entsorgt werden.

Wie oben ausgeführt, umfasst der Abfluss mindestens einen Geruchsverschluss. Der Begriff "Geruchsverschluss", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche eingerichtet ist, um Gase aus mindestens einem Abflussrohr vom Inneren der Reinigungskammer fernzuhalten. Beispielsweise kann der Geruchsverschluss mindestens einen Siphonbogen aufweisen. Das Reinigungs- und Desinfektionsgerät kann insbesondere eingerichtet sein, um den Behälter in dem Reinigungsprogramm in den Abfluss hinein zu entleeren. Wie oben ausgeführt, kann diese Entleerung in dem Reinigungsprogramm eine automatische Entleerung umfassen und/oder eine Entleerung, welche beispielsweise während eines Einbringens des Behälters in die Reinigungskammer erfolgt, beispielsweise während eines Schließens einer Tür, wobei beispielsweise eine Halterung für den Behälter mit der Tür verbunden ist und während des Schließens der Tür geschwenkt wird. Das Schließen der Tür kann dabei ganz oder teilweise durch einen Antrieb angetrieben werden, beispielsweise durch einen Motor, eine Mechanik, eine Hydraulik oder eine Pneumatik und/oder kann auch ganz oder teilweise durch Muskelkraft angetrieben werden. Der Behälterinhalt kann über den Abfluss abfließen, und der Geruchsverschluss kann zumindest teilweise verhindern, dass Gase oder Dämpfe aus dem Abfluss zurück in die Reinigungskammer strömen.

Wie oben ausgeführt, umfasst Verfahrensschritt b. mindestens einen Waschschritt, bei dem in der Reinigungskammer eine Beaufschlagung des Behälters mit mindestens einer Reinigungsflüssigkeit erfolgt, insbesondere eine Beaufschlagung mindestens einer Oberfläche des Behälters. Somit kann unter einem Waschschritt allgemein ein Vorgang verstanden werden, bei welchem der Behälter mit mindestens einem Reinigungsfluid in Form einer Reinigungsflüssigkeit in Kontakt gebracht wird. Der Begriff "Reinigungsfluid", wie er hier verwendet wird, ist somit als Oberbegriff ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Flüssigkeit und/oder ein Gas beziehen, welche eine reinigende Wirkung auf den Behälter aufweisen, beispielsweise indem durch das Reinigungsfluid anhaftende Verunreinigungen von mindestens einer Oberfläche des Behälters abgespült werden. Hierbei kann es sich um mindestens eine äußere Oberfläche des Behälters handeln und/oder auch um mindestens eine innenliegende Oberfläche, beispielsweise mindestens eine Innenoberfläche mindestens eines Behälterinnenraums, welche beispielsweise über mindestens eine Düse mit der mindestens einen Reinigungsflüssigkeit beaufschlagt werden kann.

Das Reinigungsfluid kann insbesondere ein wässriges Reinigungsfluid sein, wobei Wasser verwendet werden kann, optional unter Zusatz eines oder mehrerer Reinigungsmittel und/oder Hilfsstoffe und/oder Desinfektionsmittel. Derartige Reinigungsfluide sind aus dem Stand der Technik grundsätzlich bekannt. Das Reinigungs- und Desinfektionsgerät kann beispielsweise mindestens einen Tank aufweisen, über welchen das Reinigungsfluid der Beaufschlagungsvorrichtung bereitgestellt werden kann. Alternativ oder zusätzlich kann das Reinigungs- und Desinfektionsgerät auch einen oder mehrere Anschlüsse zur Bereitstellung des Reinigungsfluids aufweisen, beispielsweise einen Frischwasseranschluss und/oder einen Heißwasseranschluss, welche beispielsweise mit einer gebäudeseitigen Versorgung verbunden werden können. Weiterhin kann, wie unten noch näher ausgeführt wird, das Reinigungs- und Desinfektionsgerät auch beispielsweise einen oder mehrere Dampferzeuger aufweisen, zur Beaufschlagung des Behälters mit Wasserdampf. Weiterhin kann das Reinigungs- und Desinfektionsgerät auch einen oder mehrere Dosiertanks aufweisen, in welchen Zusatzstoffe des Reinigungsfluids gelagert werden können, beispielsweise indem diese dosiert mit anderen Komponenten des Reinigungsfluids gemischt werden, beispielsweise durch eine dosierte Einmischung in Wasser. Beispielsweise können ein oder mehrere Reinigertanks und/oder ein oder mehrere Tanks für Hilfsstoffe und/oder ein oder mehrere Tanks für Desinfektionsmittel in dem Reinigungs- und Desinfektionsgerät und/oder an dem Reinigungs- und Desinfektionsgerät vorgesehen sein. Der Begriff "Reinigungsflüssigkeit" bezieht sich, als spezielle Form des Reinigungsfluids, auf ein Reinigungsfluid in flüssiger Form.

Die Beaufschlagung kann insbesondere, wie nachfolgend noch näher beschrieben wird, mittels mindestens einer Beaufschlagungsvorrichtung des Reinigungs- und Desinfektionsgeräts erfolgen. Beispielsweise kann die Beaufschlagung in Form eines Besprühens und/oder eines Betropfens und/oder eines Bestrahlens des Behälters mit der Reinigungsflüssigkeit erfolgen. Insbesondere kann die Beaufschlagungsvorrichtung mindestens eine Düse aufweisen, welche mindestens einen Fluidstrahl, speziell mindestens einen Flüssigkeitsstrahl, erzeugt, der auf dem Behälter auftrifft. Beispielsweise kann die Beaufschlagungsvorrichtung mindestens eine Düse umfassen, welche den Behälter aus einer oder mehreren Raumrichtungen gezielt mit einem oder mehreren Strahlen der Reinigungsflüssigkeit besprüht und/oder bestrahlt.

Wie weiterhin oben ausgeführt, umfasst das Verfahren mit dem Verfahrensschritt c. mindestens einen Desinfektionsschritt. In dem mindestens einen Desinfektionsschritt, oder, falls mehrere Desinfektionsschritte vorgesehen sind, in mindestens einem dieser Desinfektionsschritte, erfolgt mindestens einmal ein Mischen mindestens zweier reaktiver Komponenten zur Erzeugung mindestens eines Desinfektionswirkstoffs. Weiterhin erfolgt eine Beaufschlagung des Behälters mit dem Desinfektionswirkstoff, beispielsweise mindestens einer Oberfläche des Behälters. Hierbei kann es sich um dieselbe mindestens eine Oberfläche handeln, die auch in Verfahrensschritt b. beaufschlagt werden kann, oder auch um mindestens eine andere Oberfläche. Wiederum kann es sich um mindestens eine äußere Oberfläche des Behälters handeln und/oder auch um mindestens eine innenliegende Oberfläche, beispielsweise mindestens eine Innenoberfläche mindestens eines Behälterinnenraums.

Der Begriff "reaktive Komponenten", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf chemische Verbindungen beziehen, welche eingerichtet sind, um miteinander, alleine oder unter Einwirkung eines oder mehrerer Zusatzstoffe wie beispielsweise Katalysatoren, eine chemische Reaktion durchzuführen. So können beispielsweise die reaktiven Komponenten mindestens eine Komponente A und mindestens eine Komponente B umfassen, welche als Edukte miteinander reagieren können, alleine oder unter Mitwirkung eines oder mehrerer Zusatzstoffe, wobei beispielsweise mindestens eine Komponente oder Verbindung AB oder auch C als Produkt entstehen kann, sowie gegebenenfalls weitere Produkte oder Nebenprodukte. Die Reaktion kann homogen oder auch heterogen ausgestaltet sein. Die Reaktion kann von alleine ablaufen, sobald die Edukte miteinander in Kontakt gebracht werden, oder kann eine zusätzliche Initialisierung erfordern, wie beispielsweise eine chemische, thermische, fotochemische oder auch katalytische Initialisierung. So kann beispielsweise der Ablauf der Reaktion davon abhängig sein, ob bestimmte Umgebungsbedingungen vorliegen. Diese Umgebungsbedingungen können beispielsweise durch eine Temperatur, durch einen pH-Wert, durch Anwesenheit eines oder mehrerer Katalysatoren oder auch durch andere Parameter oder Kombinationen der genannten und/oder anderer Parameter bedingt und bestimmt sein. So kann beispielsweise gezielt ein pH-Wert derart eingestellt werden, dass zunächst keine Reaktion abläuft, woraufhin dann, beispielsweise auf dem Behälter, der pH-Wert so eingestellt wird, dass die Reaktion ablaufen kann. Beispielsweise können die reaktiven Komponenten gemischt werden und auf den Behälter aufgebracht werden, wobei das Mischen vor dem Aufbringen oder auch während oder nach dem Aufbringen erfolgen kann, in einem Zustand, in welchem der pH-Wert den Ablauf der Reaktion verhindert. Auf dem Behälter kann dann, beispielsweise durch ein gezieltes Aufbringen von Säure und/oder Lauge, der pH-Wert so eingestellt werden, dass die Reaktion initiiert wird. Alternativ oder zusätzlich kann die Initiierung auch beispielsweise durch gezieltes Beleuchten mit UV-Licht oder auch auf andere Weise erfolgen. Wiederum alternativ oder zusätzlich kann beispielsweise auch eine Initiierung durch mechanisches Einwirken erfolgen, beispielsweise durch Einwirkung von Ultraschall. Mittels Ultraschall können beispielsweise Separierungen zwischen den reaktiven Komponenten zerstört oder aufgelöst werden, beispielsweise Verkapselungen, so dass die reaktiven Komponenten durch Ultraschalleinwirkung in Kontakt gebracht und zur Reaktion gebracht werden können. Die Reaktion kann einstufig oder auch mehrstufig ausgestaltet sein.

Der Begriff "Desinfektionswirkstoff", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine chemische Verbindung, eine chemische Substanz oder auch eine Mischung mehrerer chemischer Substanzen beziehen, welche eine keimabtötende Wirkung aufweisen, insbesondere eine desinfizierende Wirkung oder auch eine sterilisierende Wirkung. Insbesondere kann der Desinfektionswirkstoff mindestens eine Wirkung aufweisen, ausgewählt aus der Gruppe bestehend aus: einer sporiziden Wirkung; einer viruziden Wirkung, insbesondere einer vollviruziden Wirkung; einer fungiziden Wirkung; einer bakteriziden Wirkung. Weiterhin kann der Desinfektionswirkstoff auch eine abtötende Wirkung haben auf mindestens einen Parasiten ausgewählt aus der Gruppe bestehend aus einzellige humanpathogene Parasiten, bevorzugt Euglenozoa, Parabasalidea, Diplomonadida, Entamoebidae, Heterolobosea oder Alveolata, und mehrzellige humanpathogene Parasiten, bevorzugt Plathelminthes, Platyzoa, Nematoda, Nematomorpha, Annelida, Pentastomida, Arachnida oder Insecta.

Wie weiterhin oben ausgeführt, beinhaltet Verfahrensschritt c. mindestens ein Mischen der mindestens zwei reaktiven Komponenten. Der Begriff "Mischen", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann sich, ohne Beschränkung, insbesondere auf einen Vorgang beziehen, bei welchem die mindestens zwei reaktiven Komponenten miteinander in Kontakt gebracht werden, so dass diese miteinander reagieren können, um direkt in einem Schritt oder indirekt in mehreren Schritten, alleine oder auch unter Mitwirkung eines oder mehrerer Hilfsstoffe, den Desinfektionswirkstoff zu bilden. Verschiedene Möglichkeiten sind denkbar und werden nachfolgend auch noch näher erläutert.

So können die reaktiven Komponenten beispielsweise im gleichen Aggregatszustand oder auch in unterschiedlichen Aggregatszuständen vorliegen, wobei die reaktiven Komponenten nach dem Mischen homogen oder heterogen gemischt sein können oder auch mindestens eine gemeinsame Grenzfläche miteinander aufweisen können. So kann beispielsweise beim Mischen der reaktiven Komponenten eine Flüssigkeit, insbesondere eine Flüssigkeit ausgewählt aus einer flüssigen Mischung, einer Lösung, einer Suspension, einer Emulsion oder einer Dispersion, entstehen, welche die reaktiven Komponenten umfasst, welche unmittelbar oder auch nach einem Zeitversatz zu dem mindestens einen Desinfektionswirkstoff weiter reagieren können. Die reaktiven Komponenten können dabei in Reinform miteinander in Kontakt gebracht werden. Alternativ oder zusätzlich kann auch mindestens eine der reaktiven Komponenten in mindestens einem Hilfsstoff und/oder Trägerstoff aufgenommen sein, beispielsweise in mindestens einem Lösungsmittel. Der Hilfsstoff oder Trägerstoff, wobei beide Begriffe auch synonym verwendet werden können, kann beispielsweise fest, flüssig oder auch gasförmig ausgestaltet sein. Dabei kann für die mindestens zwei reaktiven Komponenten derselbe Trägerstoff verwendet werden, oder es können auch verschiedene Trägerstoffe eingesetzt werden.

Das Mischen kann somit beispielsweise umfassen, dass die reaktiven Komponenten jeweils in Form von Flüssigkeiten oder enthalten in Flüssigkeiten miteinander in Kontakt gebracht werden oder zusammengeführt werden, beispielsweise in einer Mischkammer, einem Mischbehälter, einem Mischgefäß, einer Pumpe, einer Mischstrecke und/oder in einem Strahl. Alternativ oder zusätzlich kann das Mischen jedoch auch umfassen, dass mindestens eine der reaktiven Komponenten in mindestens eine andere der reaktiven Komponenten eingebracht wird, beispielsweise jeweils in flüssiger Form. Wiederum alternativ oder zusätzlich kann das Mischen auch beispielsweise umfassen, dass mindestens eine der reaktiven Komponenten auf mindestens eine Oberfläche, beispielsweise mindestens eine Behälteroberfläche des Behälters, aufgebracht wird, und dass entweder anschließend mindestens eine zweite der reaktiven Komponenten auf die Oberfläche aufgebracht wird und/oder dass die Oberfläche einer Atmosphäre ausgesetzt wird, beispielsweise einer Gas- oder Dampfatmosphäre, welche mindestens eine zweite der reaktiven Komponenten umfasst. Wiederum alternativ oder zusätzlich kann das Mischen auch beispielsweise dadurch erfolgen, dass die reaktiven Komponenten einmalig oder mehrfach abwechselnd in die Reinigungskammer eingebracht werden und/oder auf den Behälter aufgebracht werden. So kann, wie oben ausgeführt, das Mischen innerhalb oder außerhalb der Reinigungskammer erfolgen. Ein Mischen innerhalb der Reinigungskammer kann beispielsweise, wie ausgeführt, durch abwechselndes auftragen der reaktiven Komponenten auf den Behälter oder auf andere Weise erfolgen. Wiederum alternativ oder zusätzlich kann der mindestens eine Trägerstoff auch beispielsweise ganz oder teilweise in fester Form vorliegen, und mindestens eine der reaktiven Komponenten kann in dem Trägerstoff eingebettet sein. Beispielsweise sind Tabs aus einem löslichen Trägerstoff denkbar, mit mindestens zwei Kammern, in denen sich die reaktiven Komponenten befinden. Bei Kontakt mit mindestens einem Lösungsmittel, beispielsweise Wasser, beispielsweise vor oder während der Beaufschlagung des Behälters, können sich die Tabs dann beispielsweise auflösen, und die reaktiven Komponenten können sich mischen. Verschiedene Möglichkeiten sind denkbar und werden nachfolgend noch näher beschrieben.

Wie weiterhin oben ausgeführt, wird der Behälter mit dem mindestens einen Desinfektionswirkstoff beaufschlagt. Wie nachfolgend noch näher ausgeführt wird, beinhaltet diese Beaufschlagung allgemein, dass der Behälter an mindestens einer Oberfläche, vorzugsweise großflächig, in Kontakt mit dem mindestens einen Desinfektionswirkstoff gebracht wird.

Hierfür bestehen mehrere Möglichkeiten, die nachfolgend auch noch näher ausgeführt werden. So kann der Desinfektionswirkstoff selbst auf den Behälter aufgebracht werden. Alternativ kann jedoch auch mindestens eine der reaktiven Komponenten auf den Behälter aufgebracht werden, und es kann auf dem Behälter selbst die oben beschriebene chemische Reaktion der reaktiven Komponenten erfolgen, bei welcher der mindestens eine Desinfektionswirkstoff gebildet wird. So ist es also möglich, dass der mindestens eine Desinfektionswirkstoff ganz oder teilweise räumlich getrennt von dem Behälter gebildet wird und anschließend auf den Behälter aufgebracht wird, beispielsweise auf die oben beschriebene mindestens eine innen- oder außenliegende Oberfläche des Behälters, und/oder dass der mindestens eine Desinfektionswirkstoff ganz oder teilweise auf dem Behälter selbst gebildet wird, beispielsweise auf der mindestens einen oben beschriebenen innen- oder außenliegenden Oberfläche des Behälters. Unabhängig davon beinhaltet die Beaufschlagung des Behälters mit dem Desinfektionswirkstoff, dass der mindestens eine Behälter ganz oder teilweise zu mindestens einem Zeitpunkt in Kontakt mit dem mindestens einen Desinfektionswirkstoff ist.

Beide Varianten, also die Bildung des Desinfektionswirkstoffs außerhalb des Behälters und ein anschließendes Aufbringen des Desinfektionswirkstoffs auf den Behälter oder die Bildung des Desinfektionswirkstoffs auf dem Behälter selbst beinhalten Vorteile, welche entsprechend der gegebenen Situation genutzt werden können.

Bei einer Bildung des Desinfektionswirkstoffs außerhalb des Behälters kann beispielsweise ein Mischungsverhältnis exakt eingestellt werden, und die Reaktion zur Bildung des Desinfektionswirkstoffs kann beispielsweise exakter kontrolliert werden.

Wird der Desinfektionswirkstoff hingegen ganz oder teilweise direkt auf dem Behälter gebildet, beispielsweise indem die Reaktion der mindestens zwei reaktiven Komponenten auf der mindestens einen Oberfläche des Behälters stattfindet, so kann dies insbesondere den Vorteil beinhalten, dass die Komponenten getrennt voneinander aufgebracht werden können, nacheinander oder gleichzeitig. Bei schnellen Reaktionen mit anschließender schneller Zersetzung des Desinfektionswirkstoffs kann auf diese Weise die Lebensdauer des Desinfektionswirkstoffs, der unmittelbar auf der Oberfläche entsteht, besser genutzt werden, da der Desinfektionswirkstoff nicht erst auf den Behälter aufgebracht werden muss, wobei wertvolle Zeit verloren gehen könnte. Weiterhin kann auch eine unerwünschte Gasbildung, welche beispielsweise bei einem Zerfall des Desinfektionswirkstoffs auftreten kann, bei einer Bildung des Desinfektionswirkstoffs unmittelbar auf der Oberfläche des Behälters besser kontrolliert werden, sodass Gase beispielsweise entweder gar nicht oder reduziert entstehen oder der Desinfektionswirkstoff bereits vor seinem Zerfall und vor der Gasbildung wieder von der Oberfläche des Behälters abgespült werden kann.

Das Verfahren kann also allgemein eine *in-situ*-Bildung des Desinfektionswirkstoffs aus den mindestens zwei reaktiven Komponenten innerhalb des Reinigungs- und Desinfektionsgeräts, welches für das Verfahren verwendet wird, beinhalten. Der Begriff "*in situ*" kann sich dabei darauf beziehen, dass der Desinfektionswirkstoff in dem Reinigungs- und Desinfektionsgerät selbst gebildet wird. Dabei sind mehrere Möglichkeiten gegeben, nämlich eine Bildung innerhalb des Reinigungs- und Desinfektionsgeräts, jedoch außerhalb der Reinigungskammer, eine Bildung innerhalb der Reinigungskammer des Reinigungs- und Desinfektionsgeräts, jedoch vor Beaufschlagung des Behälters, oder auch eine Bildung auf der Oberfläche des Behälters. Auch Kombinationen sind denkbar.

Das Verfahren kann weiterhin folgenden Schritt umfassen:
d. mindestens einen Klarspülschritt, umfassend mindestens eine Beaufschlagung des Behälters mit mindestens einer Klarspülflüssigkeit.

Der Klarspülschritt d. kann vor und/oder auch nach dem Desinfektionsschritt c. erfolgen. Auch mehrere Klarspülschritte sind möglich.

Der Begriff "Klarspülschritt", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere allgemein auf eine Beaufschlagung mindestens eines Stücks Reinigungsgut mit mindestens einer Klarspülflüssigkeit beziehen. Die Klarspülflüssigkeit kann dabei ein Reinigungsfluid sein, welches mindestens einen Klarspülerzusatz aufweist. Die Klarspülflüssigkeit kann insbesondere zu unterscheiden sein von der in dem Waschschritt verwendeten Reinigungsflüssigkeit, welche auch als Waschflüssigkeit oder Waschfluid bezeichnet werden kann. So kann beispielsweise die Waschflüssigkeit Wasser mit mindestens einem Reinigerzusatz aufweisen, wohingegen die Klarspülflüssigkeit Wasser mit mindestens einem Klarspülerzusatz umfasst, welcher ein Trocknen des Behälters beschleunigt und/oder welcher ein rückstandfreies Abperlen oder Ablaufen der Klarspülflüssigkeit von der Behälteroberfläche begünstigt.

Das Verfahren kann weiterhin folgenden Verfahrensschritt umfassen:
e. mindestens einen, insbesondere dem Klarspülschritt und/oder dem Desinfektionsschritt nachgelagerten, Dampfdesinfektionsschritt, umfassend mindestens eine Beaufschlagung des Behälters mit Dampf, insbesondere Wasserdampf.

Beispielsweise kann das Reinigungs- und Desinfektionsgerät mindestens einen Dampferzeuger aufweisen, in welchem der Dampf erzeugt wird, beispielsweise der Wasserdampf. Dieser Dampferzeuger kann beispielsweise mindestens einen Wasserspeicher aufweisen. Alternativ oder zusätzlich kann der Dampferzeuger auch beispielsweise mindestens eine Heizvorrichtung aufweisen, beispielsweise mindestens eine Heizwendel. Der Dampferzeuger kann beispielsweise über mindestens eine Dampfleitung mit der Reinigungskammer verbunden werden. Zur Beaufschlagung in Verfahrensschritt e. kann das Reinigungs- und Desinfektionsgerät beispielsweise mindestens eine Dampfdüse aufweisen, welche beispielsweise getrennt von der Beaufschlagungsvorrichtung für den Waschschritt und/oder den optionalen Klarspülschritt ausgebildet sein kann. Alternativ oder zusätzlich können die Dampfdüse und die Beaufschlagungsvorrichtung für den Waschschritt und/oder den optionalen Klarspülschritt auch ganz oder teilweise zusammengefasst sein.

Wie oben ausgeführt, kann das Mischen in Schritt c. auf verschiedene Weisen ausgestaltet sein, welche auch miteinander kombinierbar sind. Insbesondere kann das Mischen in Schritt c. mindestens eine Vermischung der reaktiven Komponenten umfassen, also ein Inkontaktbringen der reaktiven Komponenten miteinander, ausgewählt aus der Gruppe bestehend aus:

- die reaktiven Komponenten werden vermischt, wobei der Desinfektionswirkstoff in der Mischung gebildet wird und die Mischung auf den Behälter aufgebracht wird;
- die reaktiven Komponenten werden auf den Behälter aufgebracht und auf dem Behälter vermischt, wobei der Desinfektionswirkstoff in der Mischung auf dem Behälter gebildet wird;
- mindestens eine erste der reaktiven Komponenten wird auf den Behälter aufgebracht, und der Behälter mit der darauf aufgebrachten ersten reaktiven Komponente wird in der Reinigungskammer einer Atmosphäre ausgesetzt, welche mindestens eine zweite der reaktiven Komponenten umfasst, so dass sich die reaktiven Komponenten auf dem Behälter vermischen, wobei der Desinfektionswirkstoff in der Mischung auf dem Behälter entsteht.

Beispiele werden nachfolgend noch näher beschrieben.

Wie weiterhin oben ausgeführt, können die reaktiven Komponenten, mindestens eine der reaktiven Komponenten oder auch der Desinfektionswirkstoff jeweils unabhängig voneinander in reiner Form vorliegen oder auch in Kombination mit einem oder mehreren weiteren Stoffen, beispielsweise einem oder mehreren Trägerstoffen. So kann beispielsweise der Desinfektionswirkstoff in mindestens einem Trägerstoff enthalten sein, insbesondere in mindestens einem Lösungsmittel, insbesondere Wasser. Der Trägerstoff kann grundsätzlich insbesondere fluide, also flüssig und/oder gasförmig, sein. Auch Aerosole und/oder Nebel können eingesetzt werden. Der Trägerstoff kann insbesondere beispielsweise einen wässrigen Trägerstoff umfassen, da Wasser oder wasserhaltige Lösungsmittel in ihrer Entsorgung, insbesondere über den Abfluss, in der Regel geringere Herausforderungen darstellen als andere Lösungsmittel.

Der Desinfektionswirkstoff und der Trägerstoff, insbesondere das Lösungsmittel, können eine Wirklösung bilden, welche optional auch noch einen oder mehrere weitere Zusatzstoffe umfassen kann. Der Begriff "Wirklösung", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere allgemein auf ein fluides Medium beziehen, insbesondere eine Flüssigkeit, welche den mindestens einen Desinfektionswirkstoff sowie weiterhin mindestens einen Trägerstoff umfassen kann, sowie optional einen oder mehrere Zusatzstoffe. Beispielsweise kann dieser Trägerstoff mindestens ein Lösungsmittel umfassen. Die Wirklösung kann beispielsweise eine Lösung des mindestens einen Desinfektionswirkstoffs in dem mindestens einen Trägerstoff, eine Dispersion des mindestens einen Desinfektionswirkstoffs in dem mindestens einen Trägerstoff, eine Suspension des mindestens einen Desinfektionswirkstoffs in dem mindestens einen Trägerstoff oder eine Emulsion des mindestens einen Desinfektionswirkstoffs in dem mindestens einen Trägerstoff sein oder umfassen. Der Behälter kann allgemein mit der mindestens einen Wirklösung beaufschlagt werden, insbesondere in Verfahrensschritt c.

Wie oben ausgeführt, kann Verfahrensschritt c. insbesondere ein Mischen mindestens einer der reaktiven Komponenten mit dem mindestens einen Trägerstoff umfassen. Hierbei kann es sich um denselben Trägerstoff handeln, in welchem auch der Desinfektionswirkstoff enthalten sein kann, beispielsweise um dasselbe Lösungsmittel. Alternativ kann es sich jedoch auch beispielsweise lediglich um mindestens eine Komponente dieses Trägerstoffs des Desinfektionswirkstoffs handeln, beispielsweise mindestens eine Komponente eines mehrkomponentigen Lösungsmittels.

Weitere mögliche Ausgestaltungen betreffen die reaktiven Komponenten. So können die reaktiven Komponenten insbesondere mindestens ein Oxidationsmittel und mindestens ein Anion einer Säure aufweisen. Der Begriff "Oxidationsmittel", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf einen Stoff beziehen, der andere Stoffe akzeptieren kann und dabei selbst reduziert wird. Alternativ oder zusätzlich kann sich der Begriff auf einen Stoff beziehen, der mindestens ein Elektron aufnehmen kann, welcher also als Elektronenakzeptor fungieren kann. Der Begriff "Anion einer Säure", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf ein einfach oder mehrfach negativ geladenes Ion, also ein Anion, beziehen, welches dadurch entstehen kann, dass eine Säure ein oder mehrere positiv geladene Wasserstoffionen oder Protonen, also $H^+$-Ionen, abgibt.

Insbesondere kann für mögliche reaktive Komponenten, sowie auch für mögliche Desinfektionswirkstoffe, die auch im Rahmen der vorliegenden Erfindung einsetzbar sind, auf die oben beschriebenen Dokumente WO 2019/219220 A1 und WO 2019/219959 A1 verwiesen werden. Auch andere reaktive Komponenten und/oder Desinfektionswirkstoffe sind jedoch grundsätzlich möglich. Alternativ oder zusätzlich zu Peroxinitritsäure können beispielsweise als Desinfektionswirkstoff auch andere Wirkstoffe eingesetzt werden, mit entsprechenden reaktiven Komponenten. So könnten beispielsweise chlorbasierte Desinfektionswirkstoffe eingesetzt werden und/oder Peroxycarbonsäuren.

Durch die Reaktion der mindestens zwei reaktiven Komponenten kann das Verfahren, wie oben ausgeführt, eine in-situ-Erzeugung des Desinfektionswirkstoffs in dem Reinigungs- und Desinfektionsgerät umfassen. Somit entsteht der Desinfektionswirkstoff vorzugsweise erst während des Verfahrens, insbesondere in dem Reinigungs- und Desinfektionsgerät. Hierdurch lassen sich auch instabile, aggressive oder brandgefährliche Desinfektionswirkstoffe einsetzen, die ohne die in-situ-Erzeugung nicht oder nur schwer einsetzbar wären. Dadurch lassen sich das Wirkungsspektrum im Hinblick auf eine Keimabtötung vergrößern und/oder die keimabtötende Wirkung verbessern. Auch ist die Lagerung und die Bevorratung mit vergleichsweise harmlosen Ausgangsstoffen sichere und leichter um zusetzten, als dies für instabile, aggressive oder brandgefährliche Desinfektionswirkstoffe der Fall wäre.

Insbesondere kann der Desinfektionswirkstoff mindestens einen Wirkstoff umfassen, ausgewählt aus der Gruppe bestehend aus: einer reaktiven Stickstoffverbindung (reactive nitrogen oxide species, RNOS), insbesondere einer reaktiven Stickstoffverbindung ausgewählt aus der Gruppe bestehend aus: Peroxinitritsäure (ONOOH); Peroxinitrit (ONOO-); einer reaktiven Sauerstoffverbindung (reactive oxygen species, ROS), insbesondere $H_2O_2$; einer Peroxycarbonsäure, insbesondere Peroxyessigsäure ($CH_3COOOH$); einem Anion einer einer Peroxycarbonsäure, insbesondere Peroxyessigsäure ($CH_3COOO^-$); und einer Chlorverbindung, insbesondere einer Chlorverbindung ausgewählt aus der Gruppe bestehend aus hypochloriger Säure (HClO), einem Anion der hypochlorigen Säure ($ClO^-$), chloriger Säure ($HClO_2$), einem Anion der chlorigen Säure ($ClO_2^-$), Chlorsäure ($HClO_3$), einem Anion der Chlorsäure ($ClO_3^-$), einem Chloroxid, insbesondere Chlordioxid.

Die reaktiven Komponenten oder zumindest eine der reaktiven Komponenten können beispielsweise Wasserstoffperoxid ($H_2O_2$) und/oder Ozon ($O_3$) umfassen oder diese in einer chemischen Reaktion generieren, insbesondere als Oxidationsmittel.

Mindestens eine der reaktiven Komponenten, insbesondere eine andere reaktive Komponente, kann weiterhin mindestens eine Komponente umfassen, ausgewählt aus der Gruppe bestehend aus: Nitrat ($NO_3^-$), Nitrit ($NO_2^-$); einer Carbonsäure, insbesondere Essigsäure ($CH_3COOH$); einem Anion einer Carbonsäure, insbesondere Essigsäure ($CH_3COO^-$); Hypochlorit ($ClO^-$); Chlorit ($ClO_2^-$); Chlorat ($ClO_3^-$).

Die reaktiven Komponenten können insbesondere mindestens eine Kombination umfassen, insbesondere als Kombination aus mindestens einer ersten reaktiven Komponente und mindestens einer zweiten reaktiven Komponente, ausgewählt aus der Gruppe bestehend aus:

- Wasserstoffperoxid ($H_2O_2$) und Nitrit ($NO_2^-$);
- Wasserstoffperoxid ($H_2O_2$) und Nitrat ($NO_3^-$),
- einer Carbonsäure und einem Oxidationsmittel, insbesondere Wasserstoffperoxid, insbesondere Essigsäure ($CH_3COOH$) und Wasserstoffperoxid ($H_2O_2$);
- Hypochlorit ($ClO^-$) und einer Säure, insbesondere Hypochlorit ($ClO^-$) und einer Säure ausgewählt aus der Gruppe bestehend aus Essigsäure, Schwefelsäure, Zitronensäure, Phosphorsäure und Salpetersäure;
- Chlorit ($ClO_2^-$) und einer Säure, insbesondere Chlorit ($ClO_2^-$) und einer Säure ausgewählt aus der Gruppe bestehend aus Essigsäure, Schwefelsäure, Zitronensäure, Phosphorsäure und Salpetersäure;
- Chlorat ($ClO_3^-$) und einer Säure, insbesondere Chlorat ($ClO_3^-$) und einer Säure ausgewählt aus der Gruppe bestehend aus Essigsäure, Schwefelsäure, Zitronensäure, Phosphorsäure und Salpetersäure.

So lässt sich beispielsweise der Desinfektionswirkstoff Peroxinitritsäure (ONOOH) durch eine Reaktion von Wasserstoffperoxid ($H_2O_2$) und Nitrit ($NO_2^-$) als reaktiven Komponenten, insbesondere in saurem Medium, darstellen durch die Reaktion:

$$H_2O_2 + NO_2^- + H_3O^+ \rightarrow ONOOH + 2\ H_2O.$$

Bezüglich möglicher Konzentrationen und Parameter der Mischung der reaktiven Komponenten kann exemplarisch ebenfalls auf die oben beschriebenen Dokumente WO 2019/219220 A1 und WO 2019/219959 A1 verwiesen werden. Insbesondere kann bezüglich der Konzentrationen der reaktiven Komponenten, bezüglich des pH-Werts des Desinfektionswirkstoffs und/oder einer den Desinfektionswirkstoff enthaltenden Wirklösung auf dem Behälter sowie auch bezüglich der bevorzugten Zeiten der Mischung auf diese Dokumente verwiesen werden. Auch andere Zusammensetzungen und/oder Konzentrationen und/oder Parameter sind jedoch möglich.

Weiterhin lässt sich beispielsweise, alternativ oder zusätzlich, der Desinfektionswirkstoff Peroxyessigsäure ($CH_3COOOH$) durch eine Reaktion von Essigsäure ($CH_3COOH$) und Wasserstoffperoxid ($H_2O_2$) als reaktiven Komponenten darstellen durch die Reaktion:

$$CH_3COOH + H_2O_2 \rightarrow CH_3COOOH + H_2O$$

Weiterhin lässt sich beispielsweise, ebenfalls alternativ oder zusätzlich zu einer oder beiden der oben genannten Optionen, der Desinfektionswirkstoff Chlordioxid ($ClO_2$) durch eine Reaktion von Chlorat ($ClO_3^-$) und einer Säure, insbesondere Zitronensäure ($CH_3COOH$) und Wasserstoffperoxid ($H_2O_2$) als reaktiven Komponenten darstellen durch die Reaktion:

$$ClO_3^- + H^+ \rightarrow ClO_2 + H_2O$$

Weiterhin lässt sich beispielsweise, ebenfalls alternativ oder zusätzlich zu einer oder mehreren der oben genannten Optionen, der Desinfektionswirkstoff hypochlorige Säure (HClO) durch eine Reaktion von Hypochlorit ($ClO^-$) und einer Säure, beispielsweise Zitronensäure, Phosphorsäure, Schwefelsäure, Salpetersäure oder Essigsäure, als reaktiven Komponenten darstellen durch die Reaktion:

$$ClO^- + H^+ \rightarrow HClO$$

Die Hypochloritionen lassen sich beispielsweise in einer ersten reaktiven Konponente oder Lösung bereitstellen, welche ein Salz der Hypochlorsäure in alkalischer Lösung umfasst. Diese erste reaktive Komponente kann beispielsweise mit einer zweiten reaktiven Komponente gemischt werden, welche die Säure umfasst.

Auch andere Darstellungen von Desinfektionswirkstoffen aus mehreren reaktiven Komponenten sind möglich.

Im Gegensatz zur direkten Einbringung der Desinfektionswirkstoffe lassen sich die reaktiven Komponenten derart wählen, dass diese vergleichsweise einfach zu handhaben sind. So sind beispielsweise Peroxyessigsäure, Peroxinitritsäure oder Chlordioxid instabil, brandgefährlich und extrem reaktiv, sodass diese kaum zu handhaben und in einem Reinigungs- und Desinfektionsgerät eingesetzt werden können. Durch eine in-situ-Erzeugung, insbesondere in in der Reinigungskammer, aus zwei oder mehr reaktiven Komponenten, welche für sich genommen einfacher handhabbar sind, entstehen die aggressiven und gegebenenfalls kurzlebigen Desinfektionswirkstoffe erst innerhalb der Reinigungskammer, insbesondere auf der Oberfläche des zu reinigenden Behälters, und entfalten gezielt dort ihre Wirkung.

So kann allgemein beispielsweise mindestens eine der reaktiven Komponenten Wasserstoffperoxid, also $H_2O_2$, und/oder Ozon, also $O_3$, umfassen, insbesondere als Oxidationsmittel. Auch andere Oxidationsmittel sind grundsätzlich einsetzbar. Wasserstoffperoxid ist jedoch allgemein in Reinigungs- und Desinfektionsgeräten gut handhabbar und stellt ein sehr wirksames Oxidationsmittel dar. Auch die genannten Säuren sind leicht zu handhaben. Ebenso sind Nitrate, Nitrite, Chlorate, Chlorite oder Hypochlorite leicht in Form entsprechender Salze, beispielsweise Natriumsalze, bereitstellbar, beispielsweise in Form von wässrigen Lösungen dieser Salze. Beispielsweise können Nitritsalze entsprechend der Formel $M_xNO_2$ bereitgestellt werden, beispielsweise $NaNO_2$.

Zusätzlich zu den reaktiven Komponenten können weitere Stoffe enthalten sein. Hierbei kann es sich beispielsweise um Hilfsstoffe handeln, wie beispielsweise oberflächenaktive Substanzen, Tenside, Schaumbildner, Duftstoffe oder um Kombinationen der genannten Hilfsstoffe. Weiterhin können, alternativ oder zusätzlich, zusätzliche Hilfsstoffe enthalten sein, wie beispielsweise Säuren, Basen oder Puffer.

Verfahrensschritt c. kann insbesondere so durchgeführt werden, dass zwischen dem Mischen der mindestens zwei reaktiven Komponenten und der Beaufschlagung des Behälters, sofern die Mischung nicht ohnehin auf der Behälteroberfläche erfolgt, eine Zeitdauer von nicht mehr als 75 s, insbesondere von nicht mehr als 20 s, insbesondere nicht mehr als 15 s vergeht. Das Zeitfenster kann beispielsweise 1 s bis 20 s betragen. Beispielsweise kann zwischen dem Mischen der mindestens zwei reaktiven Komponenten und der Beaufschlagung des Behälters eine Zeit von 2-3 s vergehen, beispielsweise bei der oben beschriebenen Reaktion der reaktiven Komponenten Wasserstoffperoxid und Nitrat- und/oder Nitrit-Ionen.

Weiterhin kann der Desinfektionswirkstoff als Wirklösung vorliegen oder in einer Wirklösung vorliegen, beispielsweise als echte Lösung, als Dispersion, als Suspension oder als Emulsion. Als Trägerstoff, insbesondere als Lösungsmittel, kann beispielsweise Wasser verwendet werden. Wie oben ausgeführt, kann dieser Trägerstoff beispielsweise auch als Trägerstoff für eine oder mehrere der reaktiven Komponenten, insbesondere für Wasserstoffperoxid und/oder Nitrat- und/oder Nitrit-Ionen fungieren, welche außerhalb der Oberfläche des Behälters oder auch erst auf der Oberfläche des Behälters vermischt werden. Insbesondere bei Verwendung von Wasser als Trägerstoff für den Desinfektionswirkstoff und/oder für eine oder beide der reaktiven Komponenten können beispielsweise die Bedingungen derart eingestellt werden, dass in der Wirklösung mit dem darin enthaltenen Trägerstoff und dem Desinfektionswirkstoff ein pH-Wert von 2,1 bis 6,8 vorliegt.

Insbesondere bei der oben beschriebenen Reaktion der reaktiven Komponenten Wasserstoffperoxid und Nitrat- und/oder Nitrit-Ionen kann beispielsweise die Konzentration der Nitrat- und/oder Nitrit-Ionen, insbesondere in der gemischten Wirklösung mit beiden reaktiven Komponenten vor der Reaktion, in einem Bereich von 5 bis 500 mM (entsprechend 5 bis 500 × 0.001 mol/l) liegen, insbesondere in einem Bereich von 8 bis 200 mM, insbesondere in einem Bereich von 10 bis 100 mM, beispielsweise 50 mM.

Allgemein kann für die Wirksamkeit beispielsweise die Konzentration des Desinfektionswirkstoffs auf der Oberfläche des Behälters über die Zeit betrachtet werden und daraus der sogenannte Habersche Wirksamkeitsparameter H definiert werden mit:

$$H = \int_{t_1}^{t_2} c_{\mathrm{D}}\, dt \qquad\qquad (1)$$

Dabei ist $c_{\mathrm{D}}$ die Konzentration des Desinfektionswirkstoffs auf der Oberfläche des Behälters, und H ermittelt sich aus dem integral dieser Konzentration über die Zeit t im Anwendungsintervall ($t_1$, $t_2$).

Speziell für die oben beschriebene Verwendung von Peroxinitritsäure lässt sich der Habersche Wirksamkeitsparameter H beispielsweise berechnen zu

$$H = \int_{t_1}^{t_2} [ONOOH]\, dt \qquad\qquad (2)$$

Dabei ist [$ONOOH$] die Konzentration der Peroxinitritsäure auf der Oberfläche des Behälters.

Allgemein kann das Verfahren insbesondere derart durchgeführt werden, dass der Habersche Wirksamkeitsparameter H im Bereich 10 mM·s bis 400 mM mM·s liegt, insbesondere im Bereich 10 mM·s bis 200 mM·s, insbesondere im Bereich 20 mM·s bis 100 mM·s, insbesondere für die Verwendung von Peroxinitritsäure als Desinfektionswirkstoff. Der gewünschte Wirksamkeitsparameter bei dem Verfahren kann insbesondere über die Konzentration des Desinfektionswirkstoffs und/oder über die Einwirkzeit eingestellt werden. Eine Überwachung kann beispielsweise über mindestens einen Sensor erfolgen, wie nachfolgend noch näher beschrieben wird.

Der Behälter kann beispielsweise für eine vorgegebene Einwirkzeit mit dem mindestens einen Desinfektionswirkstoff und/oder der mindestens einen Wirklösung, welche den mindestens einen Trägerstoff, den mindestens einen Desinfektionswirkstoff sowie optional einen oder mehrere Zusatzstoffe umfassen kann, beaufschlagt werden. Diese Einwirkzeit kann beispielsweise beginnen mit einem Inkontaktbringen des Behälters mit dem Desinfektionswirkstoff und/oder der Wirklösung, und kann beispielsweise enden durch ein Abspülen des Desinfektionswirkstoffs oder auch auf andere Weise. Die Einwirkzeit kann unendlich oder auch begrenzt sein. Ein Abspülen kann auch beispielsweise ersetzt und/oder ergänzt werden durch die Beaufschlagung des Behälters mit Dampf, beispielsweise gemäß Verfahrensschritt e. Die Einwirkzeit kann beispielsweise begrenzt werden, beispielsweise auf 90 Sekunden oder weniger, auf 50 Sekunden oder weniger, auf 30 Sekunden oder weniger oder auch auf 15 Sekunden oder weniger. Beispielsweise kann die Einwirkzeit 1 s bis 90 s betragen. Beispielsweise kann die Einwirkzeit 1 s bis 10 Sekunden betragen, insbesondere 2 Sekunden bis 3 Sekunden. Verfahrensschritt c. kann also allgemein auch mindestens einen Einwirkschritt umfassen, bei dem über eine vorgegebene Einwirkdauer der Desinfektionswirkstoff auf den Behälter einwirkt.

Weitere optionale Möglichkeiten betreffen die Beaufschlagung in Verfahrensschritt c. So kann die Beaufschlagung in Verfahrensschritt c. insbesondere mindestens eine Beaufschlagung mit mindestens einer Beaufschlagungsart umfassen, ausgewählt aus der Gruppe bestehend aus: Besprühen; Bestrahlen; Betropfen; Begasen; Bedampfen; Vernebeln, insbesondere Kaltvernebeln. Erfolgt die Mischung der reaktiven Komponenten erst in der Reinigungskammer, beispielsweise in der Atmosphäre der Reinigungskammer und/oder auf der Oberfläche des Behälters, so können die reaktiven Komponenten auch jeweils mit unterschiedlicher Beaufschlagungsart aufgebracht werden. So kann beispielsweise eine der reaktiven Komponenten auf den Behälter aufgesprüht werden, während eine andere der reaktiven Komponenten beispielsweise vernebelt wird und als Niederschlag aus dem Nebel auf den Behälter aufgebracht wird. Die Mischung der reaktiven Komponenten kann dann beispielsweise ganz oder teilweise auf der Behälteroberfläche oder auch ganz oder teilweise in der Atmosphäre der Reinigungskammer erfolgen. Die Mischung kann weiterhin unterstützt werden durch zusätzliche Vorrichtungen, beispielsweise durch mindestens ein Gebläse zum Umwälzen des Nebels.

Wie weiterhin oben ausgeführt, kann das Verfahren zusätzliche, nicht genannte Verfahrensschritte umfassen, zusätzlich zu den Verfahrensschritten a. bis c. und optional auch zusätzlich zu den optionalen Verfahrensschritten d. und/oder e. Insbesondere kann das Verfahren weiterhin mindestens einen Verdrängungsschritt umfassen. In dem Verdrängungsschritt können Gase aus der Reinigungskammer, insbesondere Stickoxid-haltige Gase, abgeleitet werden, insbesondere zwangsweise. Der Verdrängungsschritt kann insbesondere nach dem Verfahrensschritt c. durchgeführt werden, beispielsweise unmittelbar nach dem Verfahrensschritt c. oder in einem vorgegebenen zeitlichen Abstand, beispielsweise in einem zeitlichen Abstand von 1 s bis 90 s. Beispielsweise kann der Verdrängungsschritt in einem zeitlichen Abstand von 5 Sekunden bis 90 Sekunden, insbesondere in einem zeitlichen Abstand von 10 Sekunden bis 60 Sekunden, nach Beendigung des Verfahrensschritts c. durchgeführt werden, beispielsweise jedoch vor Durchführung des optionalen Verfahrensschritts e. Alternativ oder zusätzlich kann der Verdrängungsschritt jedoch auch nach dem Verfahrensschritt d. und/oder nach dem Verfahrensschritt e. durchgeführt werden. Letztere Möglichkeit kann insbesondere den Vorteil bieten, dass Zeit für die Durchführung des Verfahrens eingespart werden kann, da dann beispielsweise eine Verdrängung des Dampfs der Dampfdesinfektion e. zeitlich zusammengefasst werden kann mit dem Schritt der Verdrängung von Dämpfen des Desinfektionsschritts c., so dass eine mehrfache Durchführung des Verdrängungsschritts entfallen kann. Weiterhin kann, alternativ oder zusätzlich, bei einer Durchführung des Verdrängungsschritts nach der Dampfdesinfektion e. und/oder nach dem Klarspülschritt d. der Vorteil bestehen, dass Dämpfe des Desinfektionsschritts c. zusätzlich durch Wasser und/oder Wasserdampf aus dem Klarspülschritt d. und/oder aus der Dampfdesinfektion e. verdünnt werden. Dennoch ist eine mehrfache Durchführung des optionalen Verdrängungsschritts ebenfalls möglich. Somit können allgemein einer oder mehrere Verdrängungsschritte durchgeführt werden, beispielsweise nach einem oder mehreren der Verfahrensschritte c., d. und e..

Der Verdrängungsschritt kann jedoch auch mehrfach durchgeführt werden, beispielsweise einmal nach Durchführung des Verfahrensschritts c. und mindestens ein weiteres Mal nach Durchführung des optionalen Verfahrensschritts e. So können beispielsweise in einem ersten Verdrängungsschritt, welcher dem Verfahrensschritt c. nachgelagert ist, Gase aus der Reinigungskammer abgeleitet werden, welche Stickoxide (NOx) enthalten, wohingegen in einem zweiten Verdrängungsschritt, welcher dem Verfahrensschritt e. nachgelagert ist, Dampf aus der Reinigungskammer abgeleitet wird. So kann dementsprechend das Verfahren beispielsweise die Verfahrensschritte a. bis c. sowie optional d. umfassen, gefolgt von einem ersten Verdrängungsschritt, auf welchen wiederum der Verfahrensschritt e. folgt, auf welchen wiederum der zweite Verdrängungsschritt folgt. Auf diese Weise können der Desinfektionsschritt c. und der optionale Dampfdesinfektionsschritt e. klar getrennt werden, und es kann zwischen diesen Desinfektionsschritten c. und e. sowie optional auch nach dem Dampfdesinfektionsschritt e. jeweils mindestens ein Verdrängungsschritt erfolgen. Damit können beispielsweise schädliche Gase aus Verfahrensschritt c. sowie auch optional für das Bedienungspersonal unangenehme Dämpfe und Feuchtigkeitsschwaden vor einem Öffnen einer Tür der Reinigungskammer in den Verdrängungsschritten abgeleitet werden, und es kann verhindert werden, dass diese in die Raumluft abgegeben werden.

Der Verdrängungsschritt kann insbesondere derart erfolgen, dass in dem Verdrängungsschritt die Gase aus der Reinigungskammer durch mindestens einen Bypass in den Abfluss stromabwärts des Geruchsverschlusses abgeleitet werden. Somit kann das Reinigungs- und Desinfektionsgerät also mindestens einen Bypass, beispielsweise mindestens eine Rohrleitung, aufweisen, welcher die Reinigungskammer und den Abfluss stromabwärts des Geruchsverschlusses, insbesondere des Siphons, verbindet. Der Bypass kann beispielsweise mindestens ein Rückschlagventil aufweisen und/oder mindestens eine andere Art von Ventil, welches verhindert, dass Gase aus dem Abfluss zurück in die Reinigungskammer strömen können.

Der Verdrängungsschritt kann insbesondere zwangsweise erfolgen. Dies kann beispielsweise dadurch realisiert werden, dass der Druck in der Reinigungskammer erhöht wird und/oder dass mindestens ein Verdrängungsmedium in die Reinigungskammer eingeleitet wird, insbesondere unter Überdruck. Zu diesem Zweck kann das Reinigungs- und Desinfektionsgerät beispielsweise mindestens eine Zuleitung für das Verdrängungsmedium aufweisen, insbesondere mit mindestens einem Ventil, und/oder mindestens ein Gebläse, welches beispielsweise zwangsweise Luft in die Reinigungskammer einleitet. Alternativ oder zusätzlich kann in dem Verdrängungsschritt auch eine Absaugung von Gasen aus der Reinigungskammer erfolgen.

Wie oben ausgeführt, kann der mindestens eine Verdrängungsschritt insbesondere mehrfach durchgeführt werden. Insbesondere kann der Verdrängungsschritt zumindest einmal nach dem Desinfektionsschritt c. und vorzugsweise vor dem optionalen Dampfdesinfektionsschritt e. durchgeführt werden. Weiterhin kann der Verdrängungsschritt optional zumindest einmal nach dem optionalen Dampfdesinfektionsschritt e. durchgeführt werden.

Alternativ oder zusätzlich zu dem Verdrängungsschritt kann das Verfahren auch mindestens einen Umwandlungsschritt umfassen. Bei dem Umwandlungsschritt können insbesondere Gase aus der Reinigungskammer, beispielsweise Stickoxid-haltige Gase, mindestens einer Umwandlungsvorrichtung zur chemischen und/oder physikalischen und/oder biologischen Aufbereitung zumindest eines Teils der Gase zugeführt werden. Die Umwandlungsvorrichtung kann beispielsweise mindestens eine Vorrichtung aufweisen, ausgewählt aus der Gruppe bestehend aus einem Katalysator, einem Filter und einer Gaswäsche. Der Filter kann beispielsweise Aktivkohle umfassen, da Aktivkohle beispielsweise Stickoxide und insbesondere $NO_2$ gut binden kann. Der Umwandlungsschritt kann auch derart durchgeführt werden, dass dieser mindestens einen Schritt umfasst, in welchem der Katalysator und/oder der Filter regeneriert und/oder wiederverwendbar gemacht werden. Dies kann beispielsweise durch Einwirkung von Temperatur erfolgen, beispielsweise auf ein Zeolithmaterial als Katalysator und/oder auf das Filtermaterial, und/oder auf andere Weise, beispielsweise auf chemische Weise.

So können beispielsweise Stickoxid-haltige Gase durch einen Katalysator, beispielsweise einen Platin-haltigen und/oder einen Palladium-haltigen Katalysator, in feuchter Atmosphäre in gasförmigen Stickstoff, Kohlendioxid und Wasser umgewandelt werden. Der Umwandlungsschritt kann in der Reinigungskammer selbst erfolgen, oder in dem Umwandlungsschritt können Gase aus der Reinigungskammer abgeleitet werden, beispielsweise durch Verdrängung und/oder Absaugen, und der Umwandlungsvorrichtung, beispielsweise dem Filter und/oder Katalysator und/oder der Gaswäsche, zugeführt werden. Nach der Aufbereitung durch die Umwandlungsvorrichtung können die Gase beispielsweise weitergeleitet werden in mindestens einer Weise, ausgewählt aus der Gruppe bestehend aus: die Gase werden zurück in die Reinigungskammer geleitet, insbesondere in einem Umwälzprozess oder Umlaufverfahren; die Gase werden in eine Umgebung abgelassen; die Gase werden in ein Abluftsystem abgeleitet; die Gase werden in den Abfluss stromabwärts des Geruchsverschlusses abgeleitet. Die erste Möglichkeit kann beispielsweise im Umwälzbetrieb erfolgen, beispielsweise indem die Gase wiederholt über die Umwandlungsvorrichtung geleitet werden. Das Umwälzverfahren kann beispielsweise so lange durchgeführt werden, bis die aufgearbeiteten Gase bestimmte Qualitätsanforderungen erfüllen, beispielsweise bis mindestens eine Gaskomponente eine Konzentrationsschwelle nicht mehr überschreitet, beispielsweise in der Reinigungskammer. Die Qualitätsanforderungen können beispielsweise durch mindestens einen Sensor erfasst werden. So können die Gase nach dem Desinfektionsschritt beispielsweise auch im Umlaufverfahren an einem oder mehreren Katalysatoren abgebaut werden. Alternativ oder zusätzlich können diese beispielsweise in einer Gaswäsche an Wasser gebunden werden. Das Wasser kann dann beispielsweise in einem nachfolgenden Reinigungsverfahren verwendet werden, beispielsweise in einem nachfolgenden Waschschritt und/oder einem Waschschritt eines nachfolgenden spült Zyklus. Alternativ oder zusätzlich kann das Wasser auch entsorgt werden, beispielsweise über den Abfluss.

Der mindestens eine optionale Umwandlungsschritt kann kontinuierlich oder diskontinuierlich durchgeführt werden. Er kann auch alternativ oder zusätzlich zu dem beschriebenen Verdrängungsschritt durchgeführt werden. Bezüglich möglicher Zeitpunkte, zu welchen der Umwandlungsschritt in dem Verfahren durchgeführt werden kann, kann auf die möglichen Zeitpunkte des Verdrängungsschritts verwiesen werden. So kann der Umwandlungsschritt beispielsweise insbesondere nach dem Verfahrensschritt c. Durchgeführt werden, beispielsweise unmittelbar nach dem Verfahrensschritt c. oder in einem vorgegebenen zeitlichen Abstand, beispielsweise in einem zeitlichen Abstand von 1 s bis 90 s. Alternativ oder zusätzlich kann der Umwandlungsschritt jedoch auch nach dem Verfahrensschritt d. und/oder nach dem Verfahrensschritt e. durchgeführt werden. Bezüglich der Optionen kann auf die Beschreibung des Verdrängungsschritts verwiesen werden.

Wie oben ausgeführt, kann das Verfahren noch weitere Verfahrensschritte umfassen. So kann das Verfahren beispielsweise mindestens einen Trocknungsschritt umfassen. Dieser Trocknungsschritt kann beispielsweise dem Desinfektionsschritt und/oder dem Dampfdesinfektionsschritt nachgelagert sein. Wie oben ausgeführt, kann beispielsweise das Verfahren zunächst die Verfahrensschritte a. bis c. sowie optional den Verfahrensschritt d. umfassen, wobei Verfahrensschritt d. vorzugsweise dem Verfahrensschritt c. vorgelagert ist. Anschließend kann, nach Durchführung des Verfahrensschritts c., ein erster Verdrängungsschritt durchgeführt werden, gefolgt optional von dem Dampfdesinfektionsschritt e. sowie optional dem mindestens einen zweiten Verdrängungsschritt und dem Trocknungsschritt. Dabei können der zweite Verdrängungsschritt und der Trocknungsschritt als getrennte Verfahrensschritte ausgestaltet sein oder können auch ganz oder teilweise als gemeinsame Verfahrensschritte ausgestaltet sein.

Wie nachfolgend noch weiter ausgeführt wird, können bei dem Verfahren einer oder mehrere Sensoren verwendet werden. Insbesondere können einer oder mehrere Sensoren eingesetzt werden, welche einen oder mehrere der Verfahrensschritte a.-c. und/oder optional einen oder mehrere der optionalen Verfahrensschritte d. und/oder e. überwachen und/oder einen oder mehrere Parameter, welche in diesen Verfahrensschritten eine Rolle spielen und welche die Durchführung dieser Verfahrensschritte charakterisieren. Insbesondere können einer oder mehrere Sensoren eingesetzt werden, welche den mindestens einen Desinfektionsschritt oder auch einen oder mehrere Teilschritte desselben überwachen. Beispielsweise können das Mischen der reaktiven Komponenten und/oder die Beaufschlagung des Behälters mit dem Desinfektionswirkstoff durch einen oder mehrere Sensoren überwacht werden. Diese Überwachung kann beispielsweise für eine Steuerung des Verfahrens, insbesondere des Desinfektionsschritts, insbesondere für eine Regelung, verwendet werden. Alternativ oder zusätzlich können mittels dieser Überwachung auch Fehlfunktionen detektiert werden, welche insbesondere einen Einfluss auf die Desinfektion des Behälters haben könnten. Bei einer Detektion einer Fehlfunktion kann das Verfahren beispielsweise eine Ausgabe eines Warnsignals an einen Benutzer umfassen.

So kann allgemein, wie ausgeführt, das Verfahren die Verwendung mindestens eines Sensors umfassen. Der Sensor kann insbesondere in mindestens einer Weise eingerichtet sein, ausgewählt aus der Gruppe bestehend aus:

a) der Sensor ist eingerichtet, um mindestens eine Eigenschaft mindestens einer Komponente zu detektieren, ausgewählt aus der Gruppe bestehend aus: dem Desinfektionswirkstoff; mindestens einer den Desinfektionswirkstoff enthaltenden Wirklösung; mindestens einer der reaktiven Komponenten; mindestens einer der reaktiven Komponenten, gemischt mit mindestens einem Trägerstoff, insbesondere Wasser; mindestens einem bei einer Reaktion der reaktiven Komponenten entstehenden Nebenprodukt; und/oder
b) der Sensor ist eingerichtet, um mindestens eine Eigenschaft mindestens eines Reaktionsprodukts innerhalb der Reinigungskammer oder auf der Oberfläche des Behälters zu detektieren, ausgewählt aus der Gruppe bestehend aus: einer entstehenden Säure; einem aus der Reaktion entstehenden Gas; einem Reaktionsnebenprodukt.

Ist der Sensor gemäß Variante a) eingerichtet, so kann die Detektion dieser mindestens einen Eigenschaft der mindestens einen Komponente innerhalb der Reinigungskammer oder auch außerhalb der Reinigungskammer erfolgen, beispielsweise in einem von der mindestens einen Komponente durchströmten Leitungssystem, wie nachfolgend noch näher ausgeführt wird.

Wie oben ausgeführt, kann der Sensor genutzt werden, beispielsweise mittels mindestens einer Steuerung, um das Verfahren zu beeinflussen. Insbesondere kann in dem Desinfektionsschritt mindestens ein Parameter des Desinfektionsschritts entsprechend mindestens eines Sensorsignals des Sensors beeinflusst werden. Der Parameter kann beispielsweise ausgewählt sein aus der Gruppe bestehend aus: einem Mischungsverhältnis der reaktiven Komponenten; einer Konzentration mindestens einer der reaktiven Komponenten; einer Konzentration des Desinfektionswirkstoffs.

Wie weiterhin nachfolgend noch näher ausgeführt wird, können bei dem Verfahren weiterhin ein oder mehrere Filterelemente eingesetzt werden. Wie nachfolgend noch näher ausgeführt wird, kann das Verfahren insbesondere unter Verwendung mindestens eines Reinigungs- und Desinfektionsgeräts durchgeführt werden. Das mindestens eine Filterelement kann dementsprechend insbesondere Bestandteil des mindestens einen Reinigungs- und Desinfektionsgeräts sein. Das Filterelement kann insbesondere in mindestens einem von mindestens einer Komponente durchströmten Leitungssystem angeordnet sein, beispielsweise mindestens einer Leitung dieses Leitungssystems, beispielsweise eines Leitungssystems des Reinigungs- und Desinfektionsgeräts. Beispielsweise kann es sich dabei um ein Desinfektionsleitungssystem handeln. Die mindestens eine Komponente kann insbesondere ausgewählt sein aus der Gruppe bestehend aus: dem Desinfektionswirkstoff; mindestens einer den Desinfektionswirkstoff enthaltenden Wirklösung; mindestens einer der reaktiven Komponenten; mindestens einer der reaktiven Komponenten, gemischt mit mindestens einem Trägerstoff, insbesondere Wasser; mindestens einem bei einer Reaktion der reaktiven Komponenten entstehenden Nebenprodukt.

Das Filterelement kann insbesondere auch genutzt werden um die mindestens eine Komponente aufzubereiten und beispielsweise um Ausfällungen, Partikelbildung oder ähnliche unerwünschte Effekte zu vermeiden. Weiterhin kann das Filterelement auch Bestandteil einer Überwachung der Funktionsweise des Verfahrens sein, beispielsweise des Desinfektionsschritts. So kann beispielsweise das Verfahren weiterhin eine Erfassung mindestens eines Drucks in dem Leitungssystem stromaufwärts und stromabwärts des Filterelements mittels mindestens eines Drucksensors umfassen, insbesondere mindestens einen Differenzdruck. Verstopft beispielsweise das Filterelement, beispielsweise aufgrund von Alterungseffekten und/oder auch der Bildung unerwünschter Bestandteile in der den Filter durchströmenden Komponente, so kann dies anhand des Drucks erkannt werden. Das Verfahren kann insbesondere in mindestens einer der folgenden Weisen eingerichtet sein:

- der Desinfektionsschritt wird entsprechend des mindestens einen Drucksignals gesteuert; und/oder
- das Drucksignal wird überwacht und bei Abweichungen des Drucksignals von mindestens einem vorgegebenen Normwert, mindestens einem vorgegebenen Normverlauf oder mindestens einem vorgegebenen Normbereich wird mindestens eine Information an einen Benutzer ausgegeben, insbesondere mindestens eine Warnung.

Steigt beispielsweise eine Viskosität aufgrund einer von einem Normbereich abweichenden Zusammensetzung des Desinfektionswirkstoffs, aufgrund einer von einem Normbereich abweichenden Konzentration oder ähnlicher Effekte, so kann dies an dem Filterelement aufgrund des Drucks erkannt werden, beispielsweise indem der Differenzdruck ansteigt. Der Desinfektionsschritt kann dann beispielsweise mittels dieses Drucksignals gesteuert werden, beispielsweise indem die Konzentration gezielt beeinflusst wird. Alternativ oder zusätzlich können beispielsweise Warnungen ausgegeben werden, wenn eine Abweichung von dem Normbereich vorliegt. Auch eine Protokollierung derartiger Effekte kann erfolgen.

In einem weiteren Aspekt der vorliegenden Erfindung wird ein Reinigungs- und Desinfektionsgerät zur Behandlung mindestens eines Behälters für menschliche Ausscheidungen vorgeschlagen. Das Reinigungs- und Desinfektionsgerät ist eingerichtet, um das vorgeschlagene Verfahren in einer oder mehreren der oben beschriebenen Ausgestaltungen und/oder gemäß einer oder mehrerer der nachfolgend noch näher beschriebenen Ausführungsformen durchzuführen. Dementsprechend kann für mögliche Definitionen und Optionen des Reinigungs- und Desinfektionsgeräts weitgehend auf die Beschreibung des Verfahrens verwiesen werden.

Das Reinigungs- und Desinfektionsgerät umfasst, wie oben ausgeführt, mindestens eine Reinigungskammer. Weiterhin umfasst das Reinigungs- und Desinfektionsgerät mindestens einen Abfluss mit mindestens einem Geruchsverschluss, insbesondere einem Siphonbogen. Weiterhin umfasst das Reinigungs- und Desinfektionsgerät mindestens eine Beaufschlagungsvorrichtung zur Beaufschlagung des Behälters in der Reinigungskammer mit mindestens einem Reinigungsfluid. Der Begriff "Beaufschlagungsvorrichtung", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche eingerichtet ist, um den Behälter mit dem mindestens einen Reinigungsfluid zu beaufschlagen. Beispielsweise kann eine Beaufschlagung in Form eines Besprühens und/oder eines Betropfens und/oder eines Bestrahlens des Behälters mit dem Reinigungsfluid erfolgen. Insbesondere kann die Beaufschlagungsvorrichtung mindestens eine Düse aufweisen, welche mindestens einen Fluidstrahl erzeugt, der auf dem Behälter auftrifft.

Beispielsweise kann die Beaufschlagungsvorrichtung mindestens eine Düse umfassen, welche den Behälter aus einer oder mehreren Raumrichtungen gezielt mit einem oder mehreren Strahlen des Reinigungsfluids besprüht und/oder bestrahlt.

Wie oben ausgeführt, ist der Begriff "Reinigungsfluid" als Oberbegriff zu verstehen und kann flüssige Reinigungsfluide sowie auch gasförmige Reinigungsfluide umfassen. Flüssige Reinigungsfluide werden auch als Reinigungsflüssigkeiten bezeichnet. Sind mehrere Reinigungsfluide zur Behandlung des Behälters in dem Reinigungs- und Desinfektionsgerät vorgesehen, so können für diese unterschiedlichen Reinigungsfluide unterschiedliche Beaufschlagungsvorrichtungen oder auch gemeinsame Beaufschlagungsvorrichtungen vorgesehen sein. So kann beispielsweise die mindestens eine Beaufschlagungsvorrichtung mindestens ein Waschsystem aufweisen, beispielsweise mit mindestens einer Waschdüse, welches für den oben beschriebenen Waschschritt einsetzbar ist, bei dem beispielsweise der Behälter mit mindestens einer Reinigungsflüssigkeit in Form mindestens einer Waschflüssigkeit beaufschlagt wird.

Für den mindestens einen optionalen Klarspülschritt kann die Beaufschlagungsvorrichtung beispielsweise optional mindestens ein Klarspülsystem aufweisen, beispielsweise mit mindestens einer Klarspüldüse. Das Waschsystem und das Klarspülsystem können ganz oder teilweise getrennt ausgebildet sein, können jedoch auch ganz oder teilweise zusammengefasst sein. Insbesondere kann das Waschsystem mindestens einen Waschtank und mindestens ein Waschleitungssystem aufweisen. Das Klarspülsystem kann mindestens einen Klarspültank und mindestens ein Klarspülleitungssystem aufweisen.

Weiterhin kann die Beaufschlagungsvorrichtung für den mindestens einen optionalen Dampfdesinfektionsschritt mindestens ein Dampfsystem aufweisen, welches beispielsweise mindestens eine Dampfdüse, mindestens einen Dampferzeuger und mindestens eine Dampfleitung aufweisen kann. Auch hier kann beispielsweise das Dampfsystem ganz oder teilweise getrennt von dem Waschsystem und dem Klarspülsystem ausgebildet sein oder kann auch ganz oder teilweise mit einem oder beiden dieser Systeme zusammengefasst sein. So kann beispielsweise das Klarspülleitungssystem ganz oder teilweise auch als Dampfleitung genutzt werden, ebenso wie beispielsweise die Klarspüldüse ganz oder teilweise als Dampfdüse genutzt werden kann. Auch eine getrennte Ausbildung ist möglich.

Zudem kann die Beaufschlagungsvorrichtung für die Durchführung des mindestens einen Desinfektionsschritts c. mindestens ein Desinfektionssystem aufweisen. Dieses Desinfektionssystem kann beispielsweise, wie nachfolgend noch näher beschrieben wird, mindestens zwei Vorratsbehälter für die reaktiven Komponenten sowie mindestens eine Desinfektionsdüse zur Beaufschlagung des Behälters mit einer oder beiden der reaktiven Komponenten aufweisen, wobei die Beaufschlagung in flüssiger Form und/oder in gasförmiger Form erfolgen kann. Weiterhin kann das Desinfektionssystem mindestens ein Desinfektionsleitungssystem aufweisen, welches beispielsweise die reaktiven Komponenten sowie optional einen oder mehrere Trägerstoffe der mindestens einen Desinfektionsdüse zuführen kann.

Das Reinigungs- und Desinfektionsgerät weist weiterhin mindestens zwei Vorratsbehälter zur Aufnahme der mindestens zwei reaktiven Komponenten auf. Hierbei kann es sich insbesondere um Vorratsbehälter für Flüssigkeiten handeln. Diese Vorratsbehälter können ganz oder teilweise geschlossen ausgestaltet sein, beispielsweise als Kanister und/oder andere Arten von Vorratsbehältern.

Das Reinigungs- und Desinfektionsgerät weist weiterhin mindestens eine Steuerung zum Ansteuern mindestens eines Reinigungsprogramms auf. Der Begriff "Reinigungsprogramm", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Abfolge von Programmschritten beziehen, welche der Reinigung von Reinigungsgut dient und bei welcher das Reinigungsgut auf unterschiedliche Weisen behandelt wird. Mindestens einer der Programmschritte beinhaltet dabei eine Beaufschlagung mit mindestens einem Reinigungsfluid. Dabei können mehrere Programmschritte mit unterschiedlicher Beaufschlagung mit unterschiedlichen Reinigungsfluiden vorgesehen sein. Für mögliche Ausgestaltungen kann beispielsweise auf das Verfahren gemäß der vorliegenden Erfindung verwiesen werden.

Der Begriff "Steuerung", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung, insbesondere eine elektronische Vorrichtung, beziehen, welche eingerichtet ist, um mindestens eine Funktion einer anderen Vorrichtung anzusteuern, zu kontrollieren, zu regeln oder in sonst irgendeiner Weise zu beeinflussen. Die Steuerung kann zentral oder auch dezentral ausgestaltet sein und/oder kann beispielsweise mindestens eine Datenverarbeitungsvorrichtung umfassen, welche programmtechnisch eingerichtet ist, um das Reinigungsprogramm zu steuern. Beispielsweise kann die Datenverarbeitungsvorrichtung eingerichtet sein, um einen, mehrere oder alle Programmparameter des Reinigungsprogramms einzustellen, beispielsweise in einer vorgegebenen Reihenfolge und/oder mit einem vorgegebenen Zeitschema. Die Steuerung kann zentral und/oder einstückig ausgestaltet sein oder kann auch dezentral ausgestaltet sein und mehrere Steuerungskomponenten umfassen. Beispielsweise kann die Steuerung einen oder mehrere Prozessoren umfassen, die optional drahtlos oder drahtgebunden miteinander verbunden sein können, um Informationen und/oder Befehle auszutauschen. Die mindestens eine Steuerung, beispielsweise die mindestens eine optionale Datenverarbeitungsvorrichtung, kann allgemein ganz oder teilweise in ein gemeinsames Modul mit der mindestens einen Reinigungskammer integriert sein, beispielsweise in ein gemeinsames Gehäuse. Alternativ oder zusätzlich kann die mindestens eine Steuerung, beispielsweise die mindestens eine Datenverarbeitungsvorrichtung, auch ganz oder teilweise außerhalb eines die Reinigungskammer umfassenden Moduls des Reinigungs- und Desinfektionsgeräts angeordnet sein, beispielsweise außerhalb eines die Reinigungskammer umschließenden Gehäuses, beispielsweise als externe Steuerung. Die mindestens eine Steuerung kann eine oder mehrere Steuerungskomponenten umfassen, beispielsweise mehrere Datenverarbeitungsvorrichtungen, die beispielsweise über mindestens eine Schnittstelle und/oder mindestens eine Datenverbindung miteinander verbunden sein können, beispielsweise um Daten und/oder allgemeine Informationen und/oder Steuerbefehle auszutauschen. Allgemein kann die Steuerung auch mindestens eine Datenverarbeitungsvorrichtung ganz oder teilweise umfassen, welche neben einer Steuerung des Reinigungs- und Desinfektionsgeräts mindesten einem weiteren Zweck dient, beispielsweise einen PC. Allgemein kann die Steuerung also eine oder mehrere Steuerungskomponenten umfassen, welche jeweils ganz oder teilweise als Hardwarekomponenten und/oder ganz oder teilweise als Softwarekomponenten realisiert sein können. Sind mehrere Steuerungskomponenten vorgesehen, so können diese als unterschiedliche Hardwarekomponenten ausgestaltet sein, die räumlich getrennt angeordnet sein können, beispielsweise in Form mehrerer Datenverarbeitungsvorrichtungen. Alternativ oder zusätzlich kann die Steuerung mehrere Steuerungskomponenten umfassen, welche als Softwarekomponenten in ein und derselben Hardware, beispielsweise in ein und derselben Datenverarbeitungsvorrichtung implementiert sein können.

Die Steuerung kann insbesondere eingerichtet sein, um zumindest die Verfahrensschritte b. und c., sowie optional die Verfahrensschritte d. und/oder e., durchzuführen, insbesondere als Programmschritte des Reinigungsprogramms.

Die Steuerung kann insbesondere eingerichtet sein, um den Desinfektionsschritt zu steuern. Hierbei kann die Steuerung beispielsweise gezielt an eine gewünschte Keimabtötung angepasst werden, beispielsweise automatisch und/oder aufgrund entsprechender Einstellungen durch das Bedienpersonal. So kann Bedienpersonal beispielsweise mittels der Steuerung den Desinfektionsschritt beeinflussen, beispielsweise eine Dauer und/oder eine Intensität des Desinfektionsschritts. Beispielsweise kann hierbei eine Art der Verkeimung und deren Bekämpfung einstellbar sein. Beispielsweise kann gezielt eingestellt werden, ob die Desinfektion gegen eine bestimmte Art von Erregern wirksam ist, wie beispielsweise gegen den typischen Krankenhauskeim *C. Difficile,* oder auch ob die Desinfektion eine sporizide Wirkung entfalten soll, beispielsweise eine vollsporizide Wirkung. Hierfür kann beispielsweise mittels der Steuerung eine Art und/oder Konzentration des Desinfektionswirkstoffs und/oder eine Dauer des Desinfektionsschritts einstellbar sein. Explizit kann dann über die Steuerung der vorher festgelegte und verifizierte Habersche Wirksamkeitsparameter H beeinfluss und eingestellt werden. Das Desinfektionsverfahren ist somit verifizierbar und sicher (entsprechend den A0-Werten).

Das Reinigungs- und Desinfektionsgerät kann insbesondere mindestens ein Desinfektionssystem aufweisen, welches beispielsweise Bestandteil der Beaufschlagungsvorrichtung sein kann. Insbesondere kann das Reinigungs- und Desinfektionsgerät mindestens eine Mischvorrichtung aufweisen, wobei die Mischvorrichtung eingerichtet sein kann, um die reaktiven Komponenten zu mischen. Bezüglich möglicher Ausgestaltungen des Mischvorgangs kann auf die obige Beschreibung des Mischens in Verfahrensschritt c. verwiesen werden, wobei die Mischvorrichtung allgemein eine Vorrichtung sein kann, um den beschriebenen Mischvorgang in einer oder mehreren der beschriebenen Ausführungsformen durchzuführen. Insbesondere kann die Mischvorrichtung eingerichtet sein, um die reaktiven Komponenten vor der Beaufschlagung des Behälters zu mischen, insbesondere in mindestens einer Vorrichtung ausgewählt aus der Gruppe bestehend aus: einer Mischkammer; einer Mischstrecke; einer Pumpe, insbesondere einer Kreiselpumpe; einer Düse, insbesondere einer Mischdüse. So kann die Mischvorrichtung allgemein, neben den Vorratsbehältern, die mindestens eine zusätzliche Vorrichtung aufweisen. Die Mischkammer kann insbesondere eine Kammer sein, welche getrennt von den Vorratsbehältern ausgebildet ist und in welcher die reaktiven Komponenten, alleine oder unter Zusatz beispielsweise von einem oder mehreren Trägerstoffen und/oder Zusatzstoffen, zusammengeführt werden können, bevor diese dann beispielsweise auf den Behälter aufgebracht werden, insbesondere über die mindestens eine Düse oder Mischdüse. Die Mischstrecke kann insbesondere mindestens einen fluidischen Leiter umfassen, welcher von den reaktiven Komponenten, alleine oder unter Zusatz beispielsweise von einem oder mehreren Trägerstoffen und/oder Zusatzstoffen, durchströmt werden kann, beispielsweise mindestens ein Strömungsrohr und/oder mindestens eine Düse, beispielsweise eine Strömungsdüse. Alternativ oder zusätzlich kann die Mischstrecke auch mindestens eine Mischvorrichtung umfassen, ausgewählt beispielsweise aus der Gruppe bestehend aus einem statischen Mischer und/oder einer Pumpe, beispielsweise einer Kreiselpumpe. Die Mischdüse kann allgemein mindestens eine Düse sein, welche eingerichtet ist, um mindestens eine der reaktiven Komponenten oder auch die mindestens zwei reaktiven Komponenten, alleine oder auch unter Zusatz beispielsweise eines oder mehrerer Trägerstoffe und/oder Zusatzstoffe, gemeinsam zu verdüsen, so dass diese auf den Behälter auftreffen und beispielsweise auf einer Strecke zwischen der Mischdüse und dem Behälter miteinander in Kontakt kommen und beispielsweise auf dieser Strecke miteinander reagieren können oder auch auf der Oberfläche des Behälters miteinander reagieren können. Dabei können die reaktiven Komponenten im gleichen Aggregatzustand oder auch in unterschiedlichen Aggregatszuständen vorliegen. So kann beispielsweise mittels der Mischdüse mindestens eine der reaktiven Komponenten, alleine oder unter Zusatz eines oder mehrerer Trägerstoffe und/oder Zusatzstoffe, auf den Behälter in flüssiger Form aufgesprüht werden, wohingegen mindestens eine andere der reaktiven Komponenten, alleine oder unter Zusatz eines oder mehrerer Trägerstoffe und/oder Zusatzstoffe, beispielsweise in Gasform, als Aerosol oder als Nebel in die Reinigungskammer eingebracht werden kann und dort beispielsweise an der Oberfläche des Behälters mit der ersten der reaktiven Komponenten reagieren kann.

Das Reinigungs- und Desinfektionsgerät kann weiterhin mindestens einen Aufbereitungsbehälter zur Aufbereitung mindestens einer der reaktiven Komponenten aufweisen. Dieser Aufbereitungsbehälter kann allgemein ein Behälter sein, welcher mit mindestens einem der Vorratsbehälter verbunden ist. Der Aufbereitungsbehälter kann weiterhin mit mindestens einem Vorratsbehälter für mindestens einen Trägerstoff verbunden sein, insbesondere einem Vorratsbehälter für Wasser. Das Reinigungs- und Desinfektionsgerät kann eingerichtet sein, um in dem Aufbereitungsbehälter die mindestens eine reaktive Komponente mit dem Trägerstoff zu mischen. Somit kann der Aufbereitungsbehälter dazu dienen, die reaktive Komponente oder mindestens eine der reaktiven Komponenten in den mindestens einen Trägerstoff einzubringen, wodurch beispielsweise eine Konzentration in gezielter Weise eingestellt werden kann und wodurch auch beispielsweise eine Verdüsung oder Verneblung der mindestens einen reaktiven Komponente erleichtert werden kann.

Das Reinigungs- und Desinfektionsgerät kann insbesondere mindestens eine Dosierpumpe aufweisen, welche eingerichtet ist, um eine vorgebbare Menge der mindestens einen reaktiven Komponente in den Aufbereitungsbehälter einzubringen. Beispielsweise kann die Dosierpumpe durch die Steuerung angesteuert werden, insbesondere auch beispielsweise hinsichtlich des Zeitpunkts der Dosierung und/oder hinsichtlich der vorgegebenen Menge der mindestens einen reaktiven Komponente und/oder des Zusatzstoffs.

Der Aufbereitungsbehälter kann insbesondere über mindestens eine Zuleitung, die mindestens ein Ventil aufweist, mit dem Vorratsbehälter verbunden sein. Das Reinigungs- und Desinfektionsgerät kann insbesondere eingerichtet sein, um den Trägerstoff über die Zuleitung in den Aufbereitungsbehälter einzubringen. Dieses Einbringen kann beispielsweise gravitationsgetrieben oder auch angetrieben durch mindestens eine weitere Dosierpumpe sein. Beispielsweise kann das Reinigungs- und Desinfektionsgerät eingerichtet sein, insbesondere mittels der Steuerung, um das Ventil entsprechend anzusteuern, um beispielsweise eine vorgegebene Menge des Trägerstoffs über die Zuleitung in den Aufbereitungsbehälter einzubringen. Beispielsweise kann dementsprechend eine Zeitsteuerung vorgesehen sein, um die Menge an Trägerstoff zu definieren.

Insbesondere kann die Zuleitung in den Aufbereitungsbehälter hineinragen. So kann beispielsweise eine Mündung der Zuleitung innerhalb einer in dem Aufbereitungsbehälter enthaltenen Menge der reaktiven Komponente eintauchbar sein. Die Steuerung kann beispielsweise eingerichtet sein, um zunächst die mindestens eine reaktive Komponente in den Aufbereitungsbehälter einzubringen, beispielsweise durch entsprechende Ansteuerung der Dosierpumpe. Die Steuerung kann weiterhin eingerichtet sein, um anschließend den mindestens einen Trägerstoff in den Aufbereitungsbehälter einzubringen, beispielsweise durch entsprechende Ansteuerung des Ventils, wobei der Trägerstoff insbesondere derart eingebracht werden kann, dass dieser von innen heraus in die reaktive Komponente einströmt, über die in die reaktive Komponente eintauchende Mündung der Zuleitung. Auf diese Weise kann eine bessere und schnellere Durchmischung der reaktiven Komponente mit dem Trägerstoff erfolgen, was insbesondere für schnell ablaufende Reaktionen vorteilhaft sein kann und was insbesondere zu einer guten Homogenität der Verteilung der reaktiven Komponente in dem Trägerstoff führen kann.

Das Reinigungs- und Desinfektionsgerät kann weiterhin mindestens einen Dampferzeuger zur Erzeugung von Dampf umfassen. Der Dampferzeuger kann insbesondere mindestens einen Vorratsbehälter, insbesondere für Wasser, und mindestens eine Heizvorrichtung zur Erzeugung des Dampfs, aufweisen, wobei der Dampferzeuger beispielsweise über mindestens eine Dampfleitung mit mindestens einer Dampfdüse an der Reinigungskammer verbunden sein kann. Der oben beschriebene Vorratsbehälter für den Trägerstoff kann insbesondere zumindest teilweise identisch mit dem Vorratsbehälter des Dampferzeugers sein. So kann beispielsweise Wasser als Trägerstoff für mindestens eine reaktive Komponente dem Vorratsbehälter des Dampferzeugers entnommen werden.

Mindestens eine und insbesondere mehrere der reaktiven Komponenten können aufbereitet werden, indem diese in mindestens einen Trägerstoff eingebracht werden. So kann allgemein beispielsweise das Reinigungs- und Desinfektionsgerät mindestens zwei der Aufbereitungsbehälter aufweisen, wobei in den Aufbereitungsbehältern unterschiedliche reaktive Komponenten aufbereitbar sind.

Nach der Aufbereitung einer oder mehrerer der reaktiven Komponenten können diese gemischt werden. So kann das Reinigungs- und Desinfektionsgerät insbesondere eingerichtet sein, um die mit dem mindestens einen Trägerstoff gemischten reaktiven Komponenten aus den Aufbereitungsbehältern zusammenzuführen und den Desinfektionswirkstoff zu bilden. Dieses Zusammenführen kann das oben beschriebene Mischen umfassen oder kann Bestandteil des oben beschriebenen Mischens sein, beispielsweise in einer oder mehrerer der beschriebenen Varianten. Das Zusammenführen kann somit außerhalb des Behälters erfolgen, beispielsweise in einer Mischkammer, in einer Mischstrecke, mittels einer Mischdüse, oder einer Kombination der genannten Möglichkeiten, oder auch beispielsweise auf dem Behälter, beispielsweise indem auf der Behälteroberfläche die aufbereiteten reaktiven Komponenten zusammengeführt werden.

Das Reinigungs- und Desinfektionsgerät kann weiterhin mindestens eine Pumpe aufweisen, welche eingerichtet ist, um die mit dem mindestens einen Trägerstoff gemischten reaktiven Komponenten aus den Aufbereitungsbehältern heraus zu pumpen und auf den Behälter aufzubringen. Die Pumpe kann insbesondere Bestandteil der Beaufschlagungsvorrichtung sein. Hierbei kann eine gesonderte Desinfektionspumpe vorgesehen sein, welche beispielsweise einen erforderlichen Druck generiert und/oder welche die aufbereiteten reaktiven Komponenten mindestens einer Desinfektionsdüse zuführt.

Weitere mögliche Ausgestaltungen betreffen die Prozesskontrolle und die Überwachung des Reinigungsprozesses, insbesondere des Desinfektionsschritts. So kann das Reinigungs- und Desinfektionsgerät, wie oben im Zusammenhang mit dem Verfahren ausgeführt, insbesondere mindestens einen Sensor umfassen. Der Sensor kann insbesondere mindestens einen Parameter und/oder mindestens eine messbare Größe erfassen, welche für den Betrieb des Reinigungs- und Desinfektionsgeräts relevant ist. Hierbei kann es sich insbesondere um mindestens eine physikalische und/oder chemische und/oder biologische Größe handeln. Entsprechende Sensoren, beispielsweise elektrische und/oder elektrooptische und/oder elektromechanische Sensoren, sind dem Fachmann grundsätzlich bekannt. Beispiele werden unten noch näher beschrieben.

Insbesondere kann der mindestens eine Sensor in mindestens einer Weise eingerichtet sein, ausgewählt aus der Gruppe bestehend aus:

a) der Sensor ist eingerichtet, um mindestens eine Eigenschaft mindestens einer Komponente zu detektieren, ausgewählt aus der Gruppe bestehend aus: dem Desinfektionswirkstoff; mindestens einer den Desinfektionswirkstoff enthaltenden Wirklösung; mindestens einer der reaktiven Komponenten; mindestens einer der reaktiven Komponenten, gemischt mit mindestens einem Trägerstoff, insbesondere Wasser; mindestens einem bei einer Reaktion der reaktiven Komponenten entstehenden Nebenprodukt;

b) der Sensor ist eingerichtet, um mindestens eine Eigenschaft mindestens eines Reaktionsprodukts innerhalb der Reinigungskammer oder auf der Oberfläche des Behälters zu detektieren, ausgewählt aus der Gruppe bestehend aus: einer entstehenden Säure; einem aus der Reaktion entstehenden Gas; einem Reaktionsnebenprodukt.

Auch Kombinationen der genannten Möglichkeiten sind denkbar.

Entsprechend der genannten Variante a) kann der Sensor also eingerichtet sein, um mindestens eine Eigenschaft mindestens einer Komponente zu detektieren, ausgewählt aus der Gruppe bestehend aus: dem Desinfektionswirkstoff; mindestens einer den Desinfektionswirkstoff enthaltenden Wirklösung; mindestens einer der reaktiven Komponenten; mindestens einer der reaktiven Komponenten, gemischt mit mindestens einem Trägerstoff, insbesondere Wasser; mindestens einem bei einer Reaktion der reaktiven Komponenten entstehenden Nebenprodukt. Das Nebenprodukt kann beispielsweise in flüssiger oder auch in gasförmiger Form entstehen.

Auf diese Weise kann insbesondere überwacht werden, dass beispielsweise ein Mischungsverhältnis zwischen dem mindestens einen Trägerstoff und der mindestens einen reaktiven Komponente und/oder des Desinfektionswirkstoffs korrekt eingestellt ist. Der Sensor kann beispielsweise in mindestens einem Aufbereitungsbehälter, in mindestens einer Zuleitung, innerhalb der Reinigungskammer oder auch an anderen Orten angeordnet sein, an welchen die Überwachung sinnvoll möglich ist.

Der Sensor kann, insbesondere in einer oder beiden der genannten Möglichkeiten a) und/oder b), insbesondere mindestens einen Sensor umfassen, ausgewählt aus der Gruppe bestehend aus: einem Leitfähigkeitssensor; einem Füllstandssensor; einem Drucksensor; einem Durchflusssensor; einem optischen Sensor, insbesondere einem optischen Sensor zur Detektion einer Gelbfärbung; einem Sensor zur Detektion spektroskopischer Eigenschaften; einem Sensor mit einer oder mehreren Leuchtdioden, insbesondere mit definierter Wellenlänge und einem oder mehreren Lichtdetektoren; einem Füllstandssensor; einem Gassensor; einem pH-Sensor. Der optische Sensor kann beispielsweise mindestens einen Absorptionssensor umfassen, wie nachfolgend noch näher ausgeführt wird.

Die spektroskopischen Eigenschaften können beispielsweise mittels mindestens einer Spektralkamera und/oder mindestens einer Hyperspektralkamera erfasst werden. Die Leuchtdioden können einzeln oder kombiniert ausgestaltet sein, beispielsweise in Form von Mehrfarb-Leuchtdioden. Der mindestens eine Lichtdetektor kann auf verschiedene Weisen ausgestaltet sein, beispielsweise indem dieser die entsprechenden Spektrallinieneigenschaften aufweist.

Lediglich exemplarisch können ein oder mehrere der Stoffe ausgewählt aus der Gruppe bestehend aus Stickoxiden, Nitrat, Nitrit, Wasserstoffperoxid, Peroxinitritsäure und Peroxinitrit optisch erfasst werden, beispielsweise indem eine Gelbfärbung von Wasser bei Anwesenheit von Nitrat, Nitrit, Stickoxiden, Peroxinitritsäure oder Peroxinitrit qualitativ und/oder quantitativ optisch erfasst wird.

Insbesondere, jedoch nicht ausschließlich, in dem Fall, dass der Sensor zumindest teilweise in der Weise a) eingerichtet ist, kann der Sensor insbesondere in mindestens einem von der Komponente durchströmten Leitungssystem angeordnet sein. Insbesondere kann es sich hierbei um mindestens eine Leitung mindestens eines Desinfektionsleitungssystems handeln, welche von der mindestens einen Komponente durchströmt wird. Insbesondere kann der Sensor somit in diesem oder auch in anderen Fällen als Durchflusssensor ausgestaltet sein, also als Sensor, welcher mindestens eine Eigenschaft mindestens eines strömenden Mediums erfassen kann. Insbesondere kann es sich um einen optischen Sensor handeln, welcher mindestens eine optische Eigenschaft des mindestens einen strömenden Mediums, insbesondere der mindestens einen Komponente, erfassen kann.

Wie oben ausgeführt, kann der mindestens eine Sensor zur Prozesskontrolle eingerichtet sein und kann beispielsweise optisch, elektrisch, mechanisch oder auf andere Weise mindestens eine Eigenschaft des Desinfektionswirkstoffs, mindestens einer den Desinfektionswirkstoff enthaltenden Wirklösung, mindestens einer der reaktiven Komponenten, mindestens einer der reaktiven Komponenten, gemischt mit mindestens einem Trägerstoff oder einer Kombination der genannten Stoffe, erfassen, welche beispielsweise zur Prozesskontrolle verwendet werden kann. So kann beispielsweise aus optischen, elektrischen oder mechanischen Eigenschaften auf eine Konzentration geschlossen werden. Der mindestens eine Sensor kann, wie nachfolgend noch näher ausgeführt wird, insbesondere mit der mindestens einen Steuerung gekoppelt sein, und es kann beispielsweise eine Regelung vorgesehen sein, um beispielsweise Konzentrationen der genannten Stoffe einzeln oder in Kombination einzustellen und/oder zu regeln.

Der mindestens eine Sensor kann, wie bereits ausgeführt, mindestens einen optischen Sensor umfassen. Insbesondere kann der mindestens eine Sensor mindestens einen optischen Absorptionssensor umfassen. Der optische Absorptionssensor kann insbesondere mindestens eine Lichtquelle und mindestens einen Fotodetektor aufweisen. Die Lichtquelle und der Fotodetektor können beispielsweise auf einander gegenüberliegenden Seiten einer Zelle angeordnet sein, in welcher sich mindestens ein Medium, welches bei dem Verfahren und/oder in dem Reinigungs- und Desinfektionsgerät eingesetzt wird, befindet. Insbesondere kann es sich hierbei um ein fluides Medium handeln, beispielsweise eine Flüssigkeit, welche insbesondere durch die Zelle strömen kann. Insbesondere kann es sich bei dem Medium um die bereits oben genannte mindestens eine Komponente handeln, welche im Rahmen des Desinfektionsschritts eingesetzt wird, insbesondere der Komponente ausgewählt aus der Gruppe bestehend aus: dem Desinfektionswirkstoff; mindestens einer den Desinfektionswirkstoff enthaltenden Wirklösung; mindestens einer der reaktiven Komponenten; mindestens einer der reaktiven Komponenten, gemischt mit mindestens einem Trägerstoff, insbesondere Wasser; mindestens einem bei einer Reaktion der reaktiven Komponenten entstehenden Nebenprodukt. Wie nachfolgend näher ausgeführt wird, kann insbesondere eine den Desinfektionswirkstoff enthaltende Wirklösung mittels des mindestens einen Sensors überwacht werden, beispielsweise des Absorptionssensors, insbesondere in einem strömenden Zustand.

Der mindestens eine optionale Absorptionssensor kann grundsätzlich auf die absorbierenden Eigenschaften oder spektralen Eigenschaften des zu erfassenden Mediums, beispielsweise der mindestens einen Komponente, angepasst werden. So sind grundsätzlich in den meisten Fällen die spektralen Eigenschaften des Mediums und/oder einer Komponente desselben bekannt, beispielsweise in Form von Absorptionskurven. Auf diese Weise kann, beispielsweise unter Verwendung des Lambert-Beer-Gesetzes, aus den Absorptionseigenschaften auf eine oder mehrere Konzentrationen geschlossen werden. Beispielsweise können die mindestens eine Lichtquelle und/oder der mindestens eine Fotodetektor auf diese spektralen Eigenschaften angepasst sein. Beispielsweise kann die Lichtquelle im ultravioletten Spektralbereich imitieren, und der Fotodetektor kann ebenfalls im ultravioletten Spektralbereich sensitiv sein. Auch andere Spektralbereiche sind jedoch, je nach den erfassenden Gegebenheiten, möglich.

Wie oben ebenfalls ausgeführt, kann der Sensor, insbesondere der optische Sensor und/oder auch andere Arten von Sensoren, insbesondere mit der mindestens einen Steuerung verbunden sein. Die Steuerung kann, wie ausgeführt, einteilig oder auch mehrteilig ausgestaltet sein. So kann beispielsweise auch optional mindestens eine Steuerungskomponente speziell für die Verarbeitung der Sensorsignale des mindestens einen Sensors und/oder für mindestens einen mittels des Sensors zu steuernden Prozessschritt angepasst sein, beispielsweise den Desinfektionsschritt. Dementsprechend kann die Steuerung einteilig oder auch mehrteilig aufgebaut sein und kann beispielsweise auch mindestens eine Desinfektionssteuerung umfassen, welche gezielt eingerichtet ist, um den mindestens einen Desinfektionsschritt anzusteuern.

Dementsprechend kann die Steuerung beispielsweise eingerichtet sein, um den Desinfektionsschritt mittels mindestens eines Sensorsignals des Sensors zu steuern, insbesondere zu regeln. So kann die Steuerung insbesondere eingerichtet sein, um mindestens einen Parameter des Desinfektionsschritts entsprechend dem Sensorsignal zu beeinflussen, insbesondere mindestens einen Parameter ausgewählt aus der Gruppe bestehend aus: einem Mischungsverhältnis der reaktiven Komponenten; einer Konzentration mindestens einer der reaktiven Komponenten; einer Konzentration des Desinfektionswirkstoffs.

Der mindestens eine Sensor, insbesondere der mindestens eine optische Sensor und insbesondere der optische Absorptionssensor, kann insbesondere eingerichtet sein, um mindestens eine Eigenschaft des Desinfektionswirkstoffs, der Wirklösung mit dem Desinfektionswirkstoff oder eines Vorprodukts oder einer Teilkomponente, beispielsweise einer oder mehrerer der reaktiven Komponenten, zu erfassen. Diesbezüglich kann auf die Optionen a) und b) oben verwiesen werden. Die mindestens eine Eigenschaft kann beispielsweise durch die Steuerung über mindestens ein Sensorsignal des mindestens einen Sensors als Istwert aufgenommen werden und mit mindestens einem Sollwert verglichen werden, um daraus beispielsweise entsprechende Steuersignale und/oder andere Informationen, beispielsweise über die überwachte Komponente, zu generieren. Das mindestens eine Sensorsignal kann beispielsweise mindestens ein Absorptionssignal sein oder umfassen, welches beispielsweise auf einen Absorptionsgrad der überwachten Komponente schließen lässt. Allgemein kann das mindestens eine Sensorsignal statisch erfasst werden. Alternativ oder zusätzlich kann auch beispielsweise ein zeitlicher Verlauf mindestens eines Signals des mindestens einen Sensors überwacht werden. Beispielsweise kann die Steuerung eingerichtet sein, um den zeitlichen Verlauf des mindestens einen Signals des mindestens einen Sensors mit mindestens einem Sollverlauf zu vergleichen. Allgemein kann die Steuerung eingerichtet sein, um aus dem mindestens einen Sensorsignal und/oder dessen zeitlichem Verlauf beispielsweise auch auf einen oder mehrere Fehler zu schließen, wie beispielsweise ein falsches Mischungsverhältnis, eine falsche und/oder gealterte reaktive Komponente oder andere Arten von Fehlern. Beispielsweise können in der Steuerung entsprechende Fehler hinterlegt sein und bestimmten Sensorsignalen und/oder Verläufen der Sensorsignale zugeordnet sein. Hierbei können auch Methoden des maschinellen Lernens eingesetzt werden, beispielsweise indem bestimmte Sensorsignale oder deren zeitliche Verläufe mit bekannten Fehlern als Trainingsdaten verwendet werden, um dann im realen Einsatz über ein trainiertes Modell auf einen entsprechenden Fehler schließen zu können.

Der mindestens eine Sensor kann, wie oben ausgeführt, an verschiedenen Stellen angeordnet sein. So kann dieser außerhalb der Reinigungskammer angeordnet sein, beispielsweise an einer oder mehreren der folgenden Stellen: an der mindestens einen Aufbereitungskammer; an der mindestens einen Mischvorrichtung; an der mindestens einen Mischkammer; an mindestens einem Bypass; an der mindestens einen Mischstrecke; an der mindestens einen Mischdüse; an mindestens einem der Vorratsbehälter; an mindestens einem Aufbereitungsbehälter; an der mindestens einen Zuleitung. Alternativ oder zusätzlich kann der mindestens eine Sensor auch innerhalb der Reaktionskammer angeordnet sein und kann beispielsweise eingerichtet sein, um die mindestens eine Eigenschaft innerhalb der Reinigungskammer zu erfassen, beispielsweise auf der Oberfläche des Behälters.

Wie weiterhin oben im Zusammenhang mit dem vorgeschlagenen Verfahren ausgeführt, kann das Reinigungs- und Desinfektionsgerät weiterhin mindestens ein Filterelement umfassen. Insbesondere kann das Filterelement in mindestens einem von mindestens einer Komponente durchströmten Leitungssystem angeordnet sein, insbesondere mindestens einer fluiden Komponente. Diese Komponente kann insbesondere ausgewählt sein aus der Gruppe bestehend aus: dem Desinfektionswirkstoff; mindestens einer den Desinfektionswirkstoff enthaltenden Wirklösung; mindestens einer der reaktiven Komponenten; mindestens einer der reaktiven Komponenten, gemischt mit mindestens einem Trägerstoff, insbesondere Wasser; mindestens einem bei einer Reaktion der reaktiven Komponenten entstehenden Nebenprodukt.

Die Funktion des mindestens einen Filterelements kann, wie ebenfalls ausgeführt, auf verschiedene Weise genutzt werden. So kann das mindestens eine Filterelement insbesondere eine aufbereitende Wirkung auf die mindestens eine Komponente ausüben, insbesondere ein Zurückhalten von Partikeln und/oder festen oder hochviskosen Bestandteilen. Alternativ oder zusätzlich kann das Filterelement auch als Sensor wirken, indem beispielsweise, wie oben ausgeführt, ein Druck stromaufwärts und stromabwärts des Filterelements erfasst und/oder überwacht wird. Auf diese Weise können beispielsweise Verunreinigungen und/oder Änderungen in der Zusammensetzung erkannt werden, denn beispielsweise kann eine Änderung der Zusammensetzung zu einer Veränderung der Druckverhältnisse stromaufwärts und stromabwärts des Filterelements führen.

Dementsprechend kann das Reinigungs- und Desinfektionsgerät, wie oben ausgeführt, insbesondere weiterhin mindestens einen Drucksensor umfassen. Der mindestens eine Drucksensor kann insbesondere eingerichtet sein, um einen Druck in dem Leitungssystem stromaufwärts und stromabwärts des Filterelements zu erfassen, insbesondere mindestens einen Differenzdruck. Die Steuerung kann insbesondere eingerichtet sein, um mindestens ein Drucksignal des Drucksensors zu erfassen. Dabei bestehen mehrere Möglichkeiten, wie die Steuerung dieses Drucksignal verarbeiten kann. Insbesondere kann die Steuerung in mindestens einer der folgenden Weisen eingerichtet sein:

- die Steuerung ist eingerichtet, um den Desinfektionsschritt entsprechend des mindestens einen Drucksignals zu steuern, beispielsweise in dem ein Mischungsverhältnis der mindestens zwei reaktiven Komponenten und/oder mindestens eine Konzentration angepasst wird;
- die Steuerung ist eingerichtet, um das Drucksignal zu überwachen und bei Abweichungen des Drucksignals von mindestens einem vorgegebenen Normwert, mindestens einem vorgegebenen Normverlauf oder mindestens einem vorgegebenen Normbereich mindestens eine Information an einen Benutzer auszugeben, insbesondere mindestens eine Warnung.

Wie oben ausgeführt, umfasst das Reinigungs- und Desinfektionsgerät die mindestens eine Beaufschlagungsvorrichtung. Diese Beaufschlagungsvorrichtung kann insbesondere für die Durchführung des mindestens einen Waschschritts eingerichtet sein sowie optional für die Durchführung des mindestens einen Klarspülschritts und/oder des mindestens einen Dampfdesinfektionsschritts. Das Reinigungs- und Desinfektionsgerät kann also eingerichtet sein, um die Beaufschlagungsvorrichtung für den mindestens einen Waschschritt einzusetzen. Das Reinigungs- und Desinfektionsgerät kann weiterhin mindestens eine Desinfektionsbeaufschlagungsvorrichtung aufweisen, wobei die Desinfektionsbeaufschlagungsvorrichtung Bestandteil der Beaufschlagungsvorrichtung ist oder auch getrennt von der Beaufschlagungsvorrichtung ausgebildet sein kann. So kann beispielsweise die Desinfektionsbeaufschlagungsvorrichtung mindestens eine Desinfektionsdüse aufweisen, welche beispielsweise von mindestens einer Waschdüse getrennt ausgebildet sein kann. Auf diese Weise kann beispielsweise eine Vermischung von Waschflüssigkeit mit einem oder mehreren Fluiden des Desinfektionsschritts vermieden werden. Das Reinigungs- und Desinfektionsgerät kann insbesondere eingerichtet sein, um die Desinfektionsbeaufschlagungsvorrichtung bei dem Desinfektionsschritt einzusetzen. Die Desinfektionsbeaufschlagungsvorrichtung kann insbesondere als Bestandteil der mindestens einen Desinfektionsdüse mindestens eine Sprühdüse aufweisen, und das Reinigungs- und Desinfektionsgerät kann eingerichtet sein, um mittels der Sprühdüse mindestens eine Komponente auf den Behälter aufzubringen. Die Komponente kann ausgewählt sein aus der Gruppe bestehend aus den reaktiven Komponenten, dem Desinfektionswirkstoff und mindestens einem Hilfsstoff, insbesondere mindestens einem Trägerstoff. Die Desinfektionsbeaufschlagungsvorrichtung kann insbesondere weiterhin, alternativ oder zusätzlich zu der mindestens einen Sprühdüse, mindestens einen Vernebler aufweisen, welcher beispielsweise eingerichtet sein kann, um mindestens ein fluides Medium zu erzeugen, ausgewählt aus der Gruppe bestehend aus einem Dampf und einem Aerosol. Wird ein Vernebler verwendet, so kann dieser insbesondere auch beispielsweise in Kombination mit mindestens einem Gebläse eingesetzt werden, beispielsweise mindestens einem Umwälzgebläse, welches einen von dem Vernebler erzeugten Nebel in der Reinigungkammer umwälzen kann. Das fluide Medium kann insbesondere mindestens eine Komponente aufweisen, ausgewählt aus der Gruppe bestehend aus den reaktiven Komponenten, dem Desinfektionswirkstoff und mindestens einem Hilfsstoff, insbesondere mindestens einem Trägerstoff.

Wie oben ausgeführt, kann das Reinigungs- und Desinfektionsgerät insbesondere mindestens eine Tür zu der Reinigungkammer aufweisen. Die Tür kann insbesondere als Klapptür ausgestaltet sein, beispielsweise als Klapptür mit einer horizontalen Schwenkachse. Die Klapptür kann insbesondere mindestens einer Halterung für den mindestens einen Behälter aufweisen, so dass dieser beispielsweise mit der Tür verbunden sein kann. Das Reinigungs- und Desinfektionsgerät kann insbesondere derart eingerichtet sein, dass bei einem Schließen der Klapptür ein Inhalt des Behälters in den Abfluss entleert wird, beispielsweise durch ein Kippen des Behälters.

Wie oben ausgeführt, kann das Reinigungs- und Desinfektionsgerät weiterhin mindestens einen Bypass aufweisen. Für mögliche Ausgestaltungen des Bypasses kann auf die obige Beschreibung verwiesen werden. Dabei können Gase aus der Reinigungkammer durch den Bypass in den Abfluss stromabwärts des Geruchsverschlusses ableitbar sein. Das Reinigungs- und Desinfektionsgerät kann weiterhin mindestens eine Verdrängungsvorrichtung aufweisen, welche eingerichtet ist, um Gase aus der Reinigungkammer durch den Bypass in den Abfluss zu abzuleiten. Die Verdrängungsvorrichtung kann insbesondere mindestens eine Vorrichtung aufweisen, ausgewählt aus der Gruppe bestehend aus einem Lüfter, einem Gebläse und einer Druckgasquelle.

Wie weiterhin oben ausgeführt, kann das Reinigung- und Desinfektionsgerät eingerichtet sein, um den mindestens einen Umwandlungsschritt durchzuführen. Dementsprechend kann das Reinigungs- und Desinfektionsgerät aufweisen. Die Umwandlungsvorrichtung kann eingerichtet sein, um Gase aus der Reinigungkammer, insbesondere Stickoxid-haltige Gase, chemisch und/oder physikalisch und/oder biologisch aufzubereiten. Wie oben ausgeführt, kann die Umwandlungsvorrichtung beispielsweise mindestens einen Katalysator und/oder mindestens einen Filter und/oder mindestens eine Gaswäsche aufweisen. Das Reinigungs- und Desinfektionsgerät kann insbesondere eingerichtet sein, um die Gase, nach Aufbereitung durch die Umwandlungsvorrichtung, weiterzuleiten werden in einer Weise, ausgewählt aus der Gruppe bestehend aus: die Gase werden zurück in die Reinigungkammer geleitet; die Gase werden in eine Umgebung abgelassen; die Gase werden in ein Abluftsystem abgeleitet; die Gase werden in den Abfluss stromabwärts des Geruchsverschlusses abgeleitet. Das Reinigung- und Desinfektionsgerät kann beispielsweise eine Pumpe und/oder eine Absaugung aufweisen, welche die Gase aus der Reinigungkammer absaugt, der Umwandlungsvorrichtung zuführt und anschließend optional entweder in die Umgebung ablässt, in den Abfluss ableitet oder zurück in die Reinigungskammer führt.

Das Verfahren und das Reinigungs- und Desinfektionsgerät gemäß einer oder mehreren der vorliegend vorgeschlagenen Ausgestaltungen weisen gegenüber bekannten Verfahren und Vorrichtungen ähnlicher Art zahlreiche Vorteile auf. So lassen sich durch das mehrkomponentige Verfahren unter Verwendung mehrerer reaktiver Komponenten, die erst in dem Reinigungs- und Desinfektionsgerät zusammengeführt und zur Reaktion gebracht werden, in situ Desinfektionswirkstoffe erzeugen. Diese Desinfektionswirkstoffe können kurzlebig sein und dennoch eine hohe Wirksamkeit aufweisen. Gerade Vorrichtungen, die hohen Hygieneanforderungen genügen müssen, wie dies in Reinigungs- und Desinfektionsgeräten der Fall ist, können von kurzlebigen Desinfektionswirkstoffen mit hoher Wirksamkeit profitieren.

Insbesondere die oben beschriebene Verwendung von Peroxinitritsäure, die sich beispielsweise in situ durch eine chemische Reaktion zwischen Wasserstoffperoxid und Nitrit herstellen lässt, bietet hierbei zahlreiche Vorteile für Reinigungs- und Desinfektionsgeräte. So hat diese unter Normalbedingungen, also bei Raumtemperatur und einem pH-Wert von weniger als 10, in der Regel eine sehr kurze Lebensdauer von typischerweise einer Sekunde. Eine Lagerung ist somit nur unter großem Aufwand möglich, beispielsweise durch Einfrieren bei -80 °C und bei pH=14. Durch das vorgeschlagene Verfahren können jedoch kontinuierlich die reaktiven Komponenten zusammengeführt und der Desinfektionswirkstoff frisch, kurz vor, während oder kurz nach der Beaufschlagung des Behälters gebildet werden. Der in situ gebildete Desinfektionswirkstoff Peroxinitritsäure weist jedoch eine hohe antibakterielle Wirkung auf.

Der Desinfektionswirkstoff lässt sich somit aus mindestens zwei reaktiven Komponenten erzeugen, die beispielsweise zeitnah vor der erwünschten Wirkung zusammengebracht werden können. Alternativ kann die Mischung auch bereits vorher erfolgen, und die Mischung kann in vorgemischter Form beispielsweise unter passiven Umgebungsbedingungen gelagert werden. Bei einer Mischung von Nitrit und Wasserstoffperoxid kann beispielsweise durch Einstellen des pH-Wertes eine Deaktivierung erfolgen, beispielsweise indem die Mischung bei pH=14 gelagert wird. Zum gewünschten Zeitpunkt der Wirkung kann der pH-Wert zur Aktivierung verändert werden, beispielsweise durch Zugabe von Säure als Hilfsstoff.

Für das Mischen und die Beaufschlagung des Behälters kommen mehrere Möglichkeiten in Betracht, die auch kombinierbar sind. So können die reaktiven Komponenten beispielsweise unmittelbar vor der Beaufschlagung, beispielsweise vor dem Versprühen, gemischt werden. Diese können beispielsweise über den Vermischungszeitpunkt hinaus weiter reagieren, auch nach der Beaufschlagung des Behälters. Beispielsweise kann eine Mischdüse mit mindestens zwei Zuläufen verwendet werden, um die reaktiven Komponenten zu mischen.

Alternativ oder zusätzlich können die reaktiven Komponenten bei der Beaufschlagung selbst vermischt werden. So können diese sich beispielsweise im Sprühstrahl vermischen, wobei beispielsweise wiederum eine Mischdüse oder mehrere Düsen mit einander überlappenden Sprühstrahlen verwendet werden können. Die reaktiven Komponenten treffen dann bereits vermischt auf den Behälter auf und können dort reagieren oder weiter reagieren. Beispielsweise kann eine erste reaktive Komponente von mindestens einer ersten Düse versprüht werden, und mindestens eine zweite reaktive Komponente von mindestens einer zweiten Düse, wobei Sprühstrahlen der Düsen überlappen können.

Wiederum alternativ oder zusätzlich zu einer oder beiden der vorgenannten Möglichkeiten, können die reaktiven Komponenten jeweils separat auf dem Behälter auftreffen, jedoch zeitlich überlappend oder zeitgleich. Dort können sich diese vermischen und reagieren.

Wiederum alternativ oder zusätzlich zu einer oder beiden der vorgenannten Möglichkeiten können die reaktiven Komponenten jeweils separat nacheinander auf dem Behälter auftreffen und dort reagieren. Beispielsweise kann zunächst die Reinigungskammer mit einem Gas oder Plasma gefüllt werden, welches beispielsweise eine erste reaktive Komponente enthält, und die zweite reaktive Komponente kann beispielsweise in die Reinigungskammer eingesprüht werden. Beispielsweise kann die Reinigungskammer zunächst mit einem Plasma gefüllt werden, welches Wasserstoffperoxid enthält, woraufhin anschließend beispielsweise Natriumnitrat eingesprüht werden kann.

Wiederum alternativ oder zusätzlich zu einer oder mehreren der oben genannten Möglichkeiten, können die reaktiven Komponenten jeweils separat und nacheinander auf dem Behälter auftreffen. Dieser Vorgang kann auch wiederholt durchgeführt werden, so dass beispielsweise alternierend die reaktiven Komponenten jeweils separat und nacheinander auf dem Behälter auftreffen. Dabei können die unterschiedlichen reaktiven Komponenten denselben Aggregatszustand haben oder auch unterschiedliche Aggregatszustände. So kann beispielsweise zunächst eine erste reaktive Komponente aufgetragen werden, beispielsweise Natriumnitrat. Nach dem Auftrag kann dann beispielsweise die zweite reaktive Komponente aufgetragen werden, beispielsweise in Form eines Gases, insbesondere eines Plasmas. So kann beispielsweise die Reinigungskammer mit plasmabehandelter Luft gefüllt werden, welche beispielsweise Ozon und/oder Wasserstoffperoxid enthält, mit welcher der Behälter beaufschlagt wird. Auf der Behälteroberfläche kann dann beispielsweise die erste Komponente mit der zweiten Komponente reagieren. Alternativ können auch beispielsweise die beiden reaktiven Komponenten nacheinander einfach oder mehrfach als Flüssigkeiten auf den Behälter aufgebracht werden.

Die Beaufschlagung des Behälters mit einer oder mehreren der reaktiven Komponenten kann auch mit einem anderen Verfahrensschritt ganz oder teilweise zusammengefasst werden. So kann beispielsweise mindestens eine der reaktiven Komponenten bereits in dem Waschschritt und/oder in dem nachgelagerten, optionalen Klarspülschritt auf den Behälter aufgebracht werden. Beispielsweise könnte Natriumnitrat nach der Klarspülung in einem Wasserfilm auf dem Behälter enthalten sein. Im Rahmen des Desinfektionsschritts, welcher auch ganz oder teilweise beispielsweise mit einem Trocknungsschritt zusammengefasst sein kann, könnte beispielsweise Ozon und/oder Wasserstoffperoxid-haltiges Gas in die Reinigungskammer eingebracht werden, wobei Ozon beispielsweise mit der Luftfeuchtigkeit in der Reinigungskammer reagieren kann. In Kontakt mit dem Natriumnitrat auf dem Behälter könnte die Reaktion ausgelöst werden.

Wenn die reaktiven Komponenten mindestens ein Oxidationsmittel umfassen, so kann dieses Oxidationsmittel beispielsweise Ozon und/oder Wasserstoffperoxid umfassen. Wie oben ausgeführt, kann beispielsweise Ozon mit Wasserdampf oder Wasser reagieren und Wasserstoffperoxid bilden. Ozon kann beispielsweise separat bereitgestellt werden und/oder kann innerhalb des Reinigungs- und Desinfektionsgeräts in situ erzeugt werden, beispielsweise elektrisch und/oder optisch, beispielsweise mittels einer UV-Lampe. Das Reduktionsmittel, beispielsweise Natriumnitrat, kann beispielsweise schon vorher aufgetragen werden, beispielsweise in wässriger Lösung, oder kann in die Reinigungskammer eingesprüht werden.

Wiederum alternativ oder zusätzlich zu einer oder mehreren der oben beschriebenen Möglichkeiten können die reaktiven Komponenten vorgemischt sein, können jedoch in inaktiver Form gehalten werden, um die Bildung des Desinfektionswirkstoffs zu verhindern. Dies kann beispielsweise, wie oben ausgeführt, mittels einer Einstellung des pH-Werts erfolgen. So kann beispielsweise ein alkalischer Zustand eine Reaktion von Wasserstoffperoxid und Nitrit verhindern oder zumindest verlangsamen. Kurz vor der Beaufschlagung, während der Beaufschlagung oder auch kurz nach der Beaufschlagung kann beispielsweise eine Aktivierung erfolgen. Diese Aktivierung kann beispielsweise durch eine Änderung des pH-Werts, durch eine Bestrahlung mit UV-Licht, durch Erwärmung, mittels eines Katalysators oder chemischen Initiators, durch Besprühen mit Säure und/oder Lauge oder auf andere Weise oder mittels einer Kombination der genannten Möglichkeiten und/oder anderer Möglichkeiten erfolgen. Wird mindestens ein chemischer Aktivierungsstoff, beispielsweise eine Säure, eine Lauge, ein Initiator oder ein Katalysator verwendet, so können diese auf verschiedene Weisen aufgebracht werden. So kann eine Beaufschlagung beispielsweise durch Besprühen erfolgen. Alternativ oder zusätzlich können diese Aktivierungsstoffe jedoch auch beispielsweise in Wachskugeln eingemischt werden, die dann beispielsweise mechanisch aufgebrochen werden können, beispielsweise in einer Umwälzpumpe und/oder einer Düse. Verschiedene andere Möglichkeiten sind denkbar.

Wie oben ausgeführt, lassen sich die Verfahrensschritte in verschiedener Weise kombinieren, modifizieren oder in anderer Weise vorteilhaft anpassen. Für Reinigungs- und Desinfektionsgeräte kann insbesondere ein Verfahren eingesetzt werden, welches die folgende Schrittfolge umfasst:

- den Entleerungsschritt a.,
- mindestens einen Vorspülschritt, bei welchem ein kaltes Vorspülen erfolgt,
- den Waschschritt b., welcher beispielsweise ein warmes Spülen umfassen kann,
- den Desinfektionsschritt c.,
- den optionalen Klarspülschritt d.,
- den optionalen Dampfdesinfektionsschritt e.,
- den optionalen Trocknungsschritt, optional auch einschließlich oder in Kombination mit dem mindestens einen optionalen Verdrängungsschritt.

Bei dem Verdrängungsschritt kann Gas aus der Reinigungskammer in den Abfluss stromabwärts des Geruchsverschlusses abgeleitet werden.

Alternativ oder zusätzlich kann das Gas jedoch auch beispielsweise über mindestens einen Filter und/oder Katalysator in eine Umgebung des Reinigungs- und Desinfektionsgeräts abgeleitet werden.

Insgesamt lässt sich mittels der vorliegenden Erfindung ein einfaches, schnelles und dennoch hochwirksames Desinfektionsverfahren realisieren, welches insbesondere im Bereich der Reinigung von Pflegeprodukten aufgrund seiner universellen breitbandigen Wirksamkeit und seiner leichten Integrierbarkeit in bestehende Vorrichtungen und Verfahren erhebliche Vorteile bietet.

Zusammenfassend werden, ohne Beschränkung weiterer möglicher Ausgestaltungen, folgende Ausführungsformen vorgeschlagen:

Ausführungsform 1 Verfahren zur Behandlung mindestens eines Behälters für menschliche Ausscheidungen, umfassend folgende Schritte:

a. mindestens einen Entleerungsschritt, umfassend ein Entleeren des Behälterinhalts innerhalb mindestens einer Reinigungskammer in mindestens einen Abfluss, wobei der Abfluss mindestens einen Geruchsverschluss umfasst;
b. mindestens einen Waschschritt, umfassend mindestens eine Beaufschlagung des Behälters mit mindestens einer Reinigungsflüssigkeit in der Reinigungskammer; und
c. mindestens einen Desinfektionsschritt, umfassend mindestens ein Mischen mindestens zweier reaktiver Komponenten zur Erzeugung mindestens eines Desinfektionswirkstoffs und Beaufschlagung des Behälters mit dem Desinfektionswirkstoff.

Ausführungsform 2 Verfahren nach der vorhergehenden Ausführungsform, weiterhin umfassend:
d. mindestens einen Klarspülschritt, umfassend mindestens eine Beaufschlagung des Behälters mit mindestens einer Klarspülflüssigkeit.

Ausführungsform 3 Verfahren nach einer der vorhergehenden Ausführungsformen, weiterhin umfassend:
e. mindestens einen, insbesondere dem Klarspülschritt und/oder dem Desinfektionsschritt nachgelagerten, Dampfdesinfektionsschritt, umfassend mindestens eine Beaufschlagung des Behälters mit Dampf.

Ausführungsform 4 Verfahren nach einer der vorhergehenden Ausführungsformen, wobei das Mischen in Schritt c. mindestens eine Vermischung der reaktiven Komponenten umfasst, ausgewählt aus der Gruppe bestehend aus:

- die reaktiven Komponenten werden vermischt, wobei der Desinfektionswirkstoff in der Mischung gebildet wird und die Mischung auf den Behälter aufgebracht wird;
- die reaktiven Komponenten werden auf den Behälter aufgebracht und auf dem Behälter vermischt, wobei der Desinfektionswirkstoff in der Mischung auf dem Behälter gebildet wird;
- mindestens eine erste der reaktiven Komponenten wird auf den Behälter aufgebracht, und der Behälter mit der darauf aufgebrachten ersten reaktiven Komponente wird in der Reinigungskammer einer Atmosphäre ausgesetzt, welche mindestens eine zweite der reaktiven Komponenten umfasst, so dass sich die reaktiven Komponenten auf dem Behälter vermischen, wobei der Desinfektionswirkstoff in der Mischung auf dem Behälter entsteht.

Ausführungsform 5 Verfahren nach einer der vorhergehenden Ausführungsformen, wobei der Desinfektionswirkstoff in mindestens einem Trägerstoff enthalten ist, insbesondere in mindestens einem Lösungsmittel, insbesondere Wasser.

Ausführungsform 6 Verfahren nach der vorhergehenden Ausführungsform, wobei der Desinfektionswirkstoff und der Trägerstoff, insbesondere das Lösungsmittel, eine Wirklösung bilden, wobei der Behälter mit der Wirklösung beaufschlagt wird, insbesondere in Verfahrensschritt c.

Ausführungsform 7 Verfahren nach einer der beiden vorhergehenden Ausführungsformen, wobei Schritt c. ein Mischen mindestens einer der reaktiven Komponenten mit dem Trägerstoff umfasst.

Ausführungsform 8 Verfahren nach einer der vorhergehenden Ausführungsformen, wobei die reaktiven Komponenten mindestens ein Oxidationsmittel und mindestens ein Anion einer Säure umfassen.

Ausführungsform 9 Verfahren nach einer der vorhergehenden Ausführungsformen, wobei der Desinfektionswirkstoff mindestens einen Wirkstoff umfasst, ausgewählt aus der Gruppe bestehend aus: einer reaktiven Stickstoffverbindung (reactive nitrogen oxide species, RNOS), insbesondere einer reaktiven Stickstoffverbindung ausgewählt aus der Gruppe bestehend aus: Peroxinitritsäure (ONOOH); Peroxinitrit (ONOO-); einer reaktiven Sauerstoffverbindung (reactive oxygen species, ROS), insbesondere $H_2O_2$; einer Peroxycarbonsäure, insbesondere Peroxyessigsäure ($CH_3COOOH$); einem Anion einer einer Peroxycarbonsäure, insbesondere Peroxyessigsäure ($CH_3COOO^-$); und einer Chlorverbindung, insbesondere einer Chlorverbindung ausgewählt aus der Gruppe bestehend aus hypochloriger Säure (HClO), einem Anion der hypochlorigen Säure (ClO$^-$), chloriger Säure (HClO$_2$), einem Anion der chlorigen Säure (ClO$_2^-$), Chlorsäure (HClO$_3$), einem Anion der Chlorsäure (ClO$_3^-$), einem Chloroxid, insbesondere Chlordioxid.

Ausführungsform 10 Verfahren nach einer der vorhergehenden Ausführungsformen, wobei die reaktiven Komponenten mindestens eine Komponente umfassen, ausgewählt aus der Gruppe bestehend aus: Wasserstoffperoxid ($H_2O$;); Ozon ($O_3$); H$^+$; und eine Säure, insbesondere mindestens eine Säure ausgewählt aus der Gruppe bestehend aus Zitronensäure, Phosphorsäure; Schwefelsäure, Salpetersäure, und Essigsäure.

Ausführungsform 11 Verfahren nach einer der vorhergehenden Ausführungsformen, wobei die reaktiven Komponenten mindestens eine Komponente umfassen, ausgewählt aus der Gruppe bestehend aus: Nitrat (NO$_3^-$), Nitrit (NO$_2^-$); einer Carbonsäure, insbesondere Essigsäure ($CH_3COOH$); einem Anion einer Carbonsäure, insbesondere Essigsäure ($CH_3COO^-$); Hypochlorit (ClO$^-$); Chlorit (ClO$_2^-$); und Chlorat (ClO$_3^-$).

Ausführungsform 12 Verfahren nach einer der vorhergehenden Ausführungsformen, wobei die reaktiven Komponenten mindestens eine Kombination umfassen, ausgewählt aus der Gruppe bestehend aus: Wasserstoffperoxid ($H_2O_2$) und Nitrit (NO$_2^-$); Wasserstoffperoxid ($H_2O_2$) und Nitrat (NO$_3^-$), einer Carbonsäure und einem Oxidationsmittel, insbesondere Wasserstoffperoxid, insbesondere Essigsäure ($CH_3COOH$) und Wasserstoffperoxid ($H_2O_2$); Hypochlorit (ClO$^-$) und einer Säure, insbesondere Hypochlorit (ClO$^-$) und einer Säure ausgewählt aus der Gruppe bestehend aus Essigsäure, Schwefelsäure, Zitronensäure, Phosphorsäure und Salpetersäure; Chlorit (ClO$_2^-$) und einer Säure, insbesondere Chlorit (ClO$_2^-$) und einer Säure ausgewählt aus der Gruppe bestehend aus Essigsäure, Schwefelsäure, Zitronensäure, Phosphorsäure und Salpetersäure; Chlorat (ClO$_3^-$) und einer Säure, insbesondere Chlorat (ClO$_3^-$) und einer Säure ausgewählt aus der Gruppe bestehend aus Essigsäure, Schwefelsäure, Zitronensäure, Phosphorsäure und Salpetersäure.

Ausführungsform 13 Verfahren nach einer der vorhergehenden Ausführungsformen, wobei der Desinfektionswirkstoff mindestens eine Wirkung aufweist, ausgewählt aus der Gruppe bestehend aus: einer sporiziden Wirkung; einer viruziden Wirkung, insbesondere einer vollviruziden Wirkung; einer fungiziden Wirkung; einer bakteriziden Wirkung.

Ausführungsform 14 Verfahren nach einer der vorhergehenden Ausführungsformen, wobei die Beaufschlagung in Verfahrensschritt c. mindestens eine Beaufschlagungsart umfasst, ausgewählt aus der Gruppe bestehend aus: Besprühen; Bestrahlen; Betropfen; Begasen; Bedampfen; Vernebeln, insbesondere Kaltvernebeln.

Ausführungsform 15 Verfahren nach einer der vorhergehenden Ausführungsformen, weiterhin umfassend mindestens einen Verdrängungsschritt, wobei in dem Verdrängungsschritt Gase aus der Reinigungskammer, insbesondere Stickoxid-haltige Gase, abgeleitet werden, insbesondere zwangsweise.

Ausführungsform 16 Verfahren nach der vorhergehenden Ausführungsform, wobei in dem Verdrängungsschritt die Gase aus der Reinigungskammer durch mindestens einen Bypass in den Abfluss stromabwärts des Geruchsverschlusses abgeleitet werden, insbesondere zwangsweise.

Ausführungsform 17 Verfahren nach der vorhergehenden Ausführungsformen, wobei der Verdrängungsschritt zumindest einmal nach dem Desinfektionsschritt und vorzugsweise vor einem optionalen Dampfdesinfektionsschritt durchgeführt wird.

Ausführungsform 18 Verfahren nach einer der vorhergehenden Ausführungsformen, weiterhin umfassend mindestens einen Umwandlungsschritt, wobei bei dem Umwandlungsschritt Gase aus der Reinigungskammer, insbesondere Stickoxid-haltige Gase, mindestens einer Umwandlungsvorrichtung zur chemischen und/oder physikalischen und/oder biologischen Aufbereitung zumindest eines Teils der Gase zugeführt werden.

Ausführungsform 19 Verfahren nach der vorhergehenden Ausführungsform, wobei die Umwandlungsvorrichtung mindestens eine Vorrichtung aufweist, ausgewählt aus der Gruppe bestehend aus einem Katalysator, einem Filter und einer Gaswäsche.

Ausführungsform 20 Verfahren nach einer der beiden vorhergehenden Ausführungsformen, wobei die Gase, nach Aufbereitung durch die Umwandlungsvorrichtung, weitergeleitet werden in einer Weise, ausgewählt aus der Gruppe bestehend aus: die Gase werden zurück in die Reinigungskammer geleitet; die Gase werden in eine Umgebung abgelassen; die Gase werden in ein Abluftsystem abgeleitet; die Gase werden in den Abfluss stromabwärts des Geruchsverschlusses abgeleitet.

Ausführungsform 21 Verfahren nach einer der vorhergehenden Ausführungsformen, wobei das Verfahren weiterhin die Verwendung mindestens eines Sensors umfasst, wobei der Sensor in mindestens einer Weise eingerichtet ist, ausgewählt aus der Gruppe bestehend aus:

a) der Sensor ist eingerichtet, um mindestens eine Eigenschaft mindestens einer Komponente zu detektieren, ausgewählt aus der Gruppe bestehend aus: dem Desinfektionswirkstoff; mindestens einer den Desinfektionswirkstoff enthaltenden Wirklösung; mindestens einer der reaktiven Komponenten; mindestens einer der reaktiven Komponenten, gemischt mit mindestens einem Trägerstoff, insbesondere Wasser; mindestens einem bei einer Reaktion der reaktiven Komponenten entstehenden Nebenprodukt;
b) der Sensor ist eingerichtet, um mindestens eine Eigenschaft mindestens eines Reaktionsprodukts innerhalb der Reinigungskammer oder auf der Oberfläche des Behälters zu detektieren, ausgewählt aus der Gruppe bestehend aus: einer entstehenden Säure; einem aus der Reaktion entstehenden Gas; einem Reaktionsnebenprodukt.

Ausführungsform 22 Verfahren nach der vorhergehenden Ausführungsform, wobei in dem Desinfektionsschritt mindestens ein Parameter des Desinfektionsschritts entsprechend mindestens eines Sensorsignals des Sensors beeinflusst wird.

Ausführungsform 23 Verfahren nach der vorhergehenden Ausführungsform, wobei der Parameter ausgewählt ist aus der Gruppe bestehend aus: einem Mischungsverhältnis der reaktiven Komponenten; einer Konzentration mindestens einer der reaktiven Komponenten; einer Konzentration des Desinfektionswirkstoffs.

Ausführungsform 24 Verfahren nach einem der vorhergehenden Ansprüche, wobei das Verfahren weiterhin eine Verwendung mindestens eines Filterelements umfasst, wobei das Filterelement in mindestens einem von mindestens einer Komponente durchströmten Leitungssystem angeordnet ist, wobei die Komponente ausgewählt ist aus der Gruppe bestehend aus dem Desinfektionswirkstoff; mindestens einer den Desinfektionswirkstoff enthaltenden Wirklösung; mindestens einer der reaktiven Komponenten; mindestens einer der reaktiven Komponenten, gemischt mit mindestens einem Trägerstoff, insbesondere Wasser; mindestens einem bei einer Reaktion der reaktiven Komponenten entstehenden Nebenprodukt.

Ausführungsform 25 Verfahren nach dem vorhergehenden Anspruch, wobei das Verfahren weiterhin eine Erfassung mindestens eines Drucks in dem Leitungssystem stromaufwärts und stromabwärts des Filterelements mittels mindestens eines Drucksensors umfasst, insbesondere mindestens einen Differenzdruck.

Ausführungsform 26 Verfahren nach dem vorhergehenden Anspruch, wobei das Verfahren in mindestens einer der folgenden Weisen eingerichtet ist:

- der Desinfektionsschritt wird entsprechend des mindestens einen Drucksignals gesteuert;
- das Drucksignal wird überwacht und bei Abweichungen des Drucksignals von mindestens einem vorgegebenen Normwert, mindestens einem vorgegebenen Normverlauf oder mindestens einem vorgegebenen Normbereich mindestens eine Information an einen Benutzer ausgegeben, insbesondere mindestens eine Warnung.

Ausführungsform 27 Reinigungs- und Desinfektionsgerät zur Behandlung mindestens eines Behälters für menschliche Ausscheidungen, umfassend mindestens eine Reinigungskammer, weiterhin umfassend mindestens einen Abfluss mit mindestens einem Geruchsverschluss, insbesondere einem Siphonbogen, wobei das Reinigungs- und Desinfektionsgerät weiterhin mindestens eine Beaufschlagungsvorrichtung zur Beaufschlagung des Behälters mit mindestens einem Reinigungsfluid in der Reinigungskammer aufweist, wobei das Reinigungs- und Desinfektionsgerät weiterhin mindestens zwei Vorratsbehälter zur Aufnahme reaktiver Komponenten aufweist, wobei das Reinigungs- und Desinfektionsgerät weiterhin mindestens eine Steuerung zum Ansteuern mindestens eines Reinigungsprogramms aufweist, wobei das Reinigungs- und Desinfektionsgerät eingerichtet ist, um das Verfahren nach einer der vorhergehenden Ausführungsformen durchzuführen.

Ausführungsform 28 Reinigungs- und Desinfektionsgerät nach der vorhergehenden Ausführungsform, wobei die Steuerung eingerichtet ist, um zumindest die Verfahrensschritte b. und c., sowie optional die Verfahrensschritte d. und/oder e., durchzuführen, insbesondere als Programmschritte des Reinigungsprogramms.

Ausführungsform 29 Reinigungs- und Desinfektionsgerät nach einer der vorhergehenden, ein Reinigungs- und Desinfektionsgerät betreffenden Ausführungsformen, weiterhin umfassend mindestens eine Mischvorrichtung, wobei die Mischvorrichtung eingerichtet ist, um die reaktiven Komponenten zu mischen.

Ausführungsform 30 Reinigungs- und Desinfektionsgerät nach der vorhergehenden Ausführungsform, wobei die Mischvorrichtung eingerichtet ist, um die reaktiven Komponenten vor der Beaufschlagung des Behälters zu mischen, insbesondere in mindestens einer Vorrichtung ausgewählt aus der Gruppe bestehend aus: einer Mischkammer; einer Mischstrecke; einer Düse insbesondere einer Mischdüse; einer Pumpe, insbesondere einer Kreiselpumpe.

Ausführungsform 31 Reinigungs- und Desinfektionsgerät nach einer der vorhergehenden, ein Reinigungs- und Desinfektionsgerät betreffenden Ausführungsformen, wobei das Reinigungs- und Desinfektionsgerät mindestens einen Aufbereitungsbehälter zur Aufbereitung mindestens einer der reaktiven Komponenten aufweist, wobei der Aufbereitungsbehälter mit mindestens einem der Vorratsbehälter verbunden ist und wobei der Aufbereitungsbehälter weiterhin mit mindestens einem Vorratsbehälter für mindestens einen Trägerstoff verbunden ist, insbesondere einem Vorratsbehälter für Wasser, wobei das Reinigungs- und Desinfektionsgerät eingerichtet ist, um in dem Aufbereitungsbehälter die mindestens eine reaktive Komponente mit dem Trägerstoff zu mischen.

Ausführungsform 32 Reinigungs- und Desinfektionsgerät nach der vorhergehenden Ausführungsform, wobei das Reinigungs- und Desinfektionsgerät mindestens eine Dosierpumpe aufweist, wobei die Dosierpumpe eingerichtet ist, um eine vorgebbare Menge der mindestens einen reaktiven Komponente in den Aufbereitungsbehälter einzubringen.

Ausführungsform 33 Reinigungs- und Desinfektionsgerät nach einer der beiden vorhergehenden Ausführungsformen, wobei der Aufbereitungsbehälter über mindestens eine Zuleitung mit mindestens einem Ventil mit dem Vorratsbehälter verbunden ist, wobei das Reinigungs- und Desinfektionsgerät eingerichtet ist, um den Trägerstoff über die Zuleitung in den Aufbereitungsbehälter einzubringen.

Ausführungsform 34 Reinigungs- und Desinfektionsgerät nach der vorhergehenden Ausführungsform, wobei die Zuleitung in den Aufbereitungsbehälter hineinragt, so dass eine Mündung der Zuleitung innerhalb einer in dem Aufbereitungsbehälter enthaltenen Menge der reaktiven Komponente eintauchbar ist.

Ausführungsform 35 Reinigungs- und Desinfektionsgerät nach einer der vier vorhergehenden Ausführungsformen, weiterhin umfassend mindestens einen Dampferzeuger zur Erzeugung von Dampf, wobei der Vorratsbehälter für den Trägerstoff zumindest teilweise identisch ist mit einem Vorratsbehälter des Dampferzeugers.

Ausführungsform 36 Reinigungs- und Desinfektionsgerät nach einer der fünf vorhergehenden Ausführungsformen, wobei das Reinigungs- und Desinfektionsgerät mindestens zwei der Aufbereitungsbehälter aufweist, wobei in den Aufbereitungsbehältern unterschiedliche reaktive Komponenten aufbereitbar sind.

Ausführungsform 37 Reinigungs- und Desinfektionsgerät nach der vorhergehenden Ausführungsform, wobei das Reinigungs- und Desinfektionsgerät eingerichtet ist, um die mit dem mindestens einen Trägerstoff gemischten reaktiven Komponenten aus den Aufbereitungsbehältern zusammenzuführen und den Desinfektionswirkstoff zu bilden.

Ausführungsform 38 Reinigungs- und Desinfektionsgerät nach der vorhergehenden Ausführungsform, weiterhin umfassend mindestens eine Pumpe, wobei die Pumpe eingerichtet ist, um die mit dem mindestens einen Trägerstoff gemischten reaktiven Komponenten aus den Aufbereitungsbehältern heraus zu pumpen und auf den Behälter aufzubringen.

Ausführungsform 39 Reinigungs- und Desinfektionsgerät nach einer der vorhergehenden, ein Reinigungs- und Desinfektionsgerät betreffenden Ausführungsformen, weiterhin umfassend mindestens einen Sensor, wobei der Sensor in mindestens einer Weise eingerichtet ist, ausgewählt aus der Gruppe bestehend aus:

a) der Sensor ist eingerichtet, um mindestens eine Eigenschaft mindestens einer Komponente zu detektieren, ausgewählt aus der Gruppe bestehend aus: dem Desinfektionswirkstoff; mindestens einer den Desinfektionswirkstoff enthaltenden Wirklösung; mindestens einer der reaktiven Komponenten; mindestens einer der reaktiven Komponenten, gemischt mit mindestens einem Trägerstoff, insbesondere Wasser; mindestens einem bei einer Reaktion der reaktiven Komponenten entstehenden Nebenprodukt;
b) der Sensor ist eingerichtet, um mindestens eine Eigenschaft mindestens eines Reaktionsprodukts innerhalb der Reinigungskammer oder auf der Oberfläche des Behälters zu detektieren, ausgewählt aus der Gruppe bestehend aus: einer entstehenden Säure; einem aus der Reaktion entstehenden Gas; einem Reaktionsnebenprodukt.

Ausführungsform 40 Reinigungs- und Desinfektionsgerät nach der vorhergehenden Ausführungsform, wobei der Sensor mindestens einen Sensor umfasst, ausgewählt aus der Gruppe bestehend aus: einem Leitfähigkeitssensor; einem Füllstandssensor; einem Drucksensor; einem Durchflusssensor; einem optischen Sensor, insbesondere einem optischen Sensor zur Detektion einer Gelbfärbung; einem Sensor zur Detektion spektroskopischer Eigenschaften, beispielsweise ein Spektrometer und/oder eine Hyperspektralkamera; einem Sensor mit einer oder mehreren Leuchtdioden, insbesondere mit definierter Wellenlänge und/oder einer Vielfarb-Leuchtdiode und insbesondere einem oder mehreren Lichtdetektoren, insbesondere entsprechenden Lichtdetektoren; einem Füllstandssensor; einem Gassensor; einem pH-Sensor.

Ausführungsform 41 Reinigungs- und Desinfektionsgerät nach einer der beiden vorhergehenden Ausführungsformen, wobei der Sensor zumindest teilweise in der Weise a) eingerichtet ist, wobei der Sensor in mindestens einem von der Komponente durchströmten Leitungssystem angeordnet ist, insbesondere in mindestens einem Desinfektionsleitungssystem.

Ausführungsform 42 Reinigungs- und Desinfektionsgerät nach einer der drei vorhergehenden Ausführungsformen, wobei der Sensor mindestens einen optischen Absorptionssensor umfasst, insbesondere mindestens einen optischen Absorptionssensor mit mindestens einer Lichtquelle und mindestens einem Fotodetektor, insbesondere einer im ultravioletten Spektralbereich emittierenden Lichtquelle und mindestens einem im ultravioletten Spektralbereich sensitiven Fotodetektor.

Ausführungsform 43 Reinigungs- und Desinfektionsgerät nach einer der vier vorhergehenden Ausführungsformen, wobei der Sensor mit der Steuerung verbunden ist.

Ausführungsform 44 Reinigungs- und Desinfektionsgerät nach der vorhergehenden Ausführungsform, wobei die Steuerung eingerichtet ist, um den Desinfektionsschritt mittels mindestens eines Sensorsignals des Sensors zu steuern, insbesondere zu regeln.

Ausführungsform 45 Reinigungs- und Desinfektionsgerät nach der vorhergehenden Ausführungsform, wobei die Steuerung eingerichtet ist, um mindestens einen Parameter des Desinfektionsschritts entsprechend dem Sensorsignal zu beeinflussen, insbesondere mindestens einen Parameter ausgewählt aus der Gruppe bestehend aus: einem Mischungsverhältnis der reaktiven Komponenten; einer Konzentration mindestens einer der reaktiven Komponenten; einer Konzentration des Desinfektionswirkstoffs.

Ausführungsform 46 Reinigungs- und Desinfektionsgerät nach einer der vorhergehenden, ein Reinigungs- und Desinfektionsgerät betreffenden Ausführungsformen, weiterhin umfassend mindestens ein Filterelement, wobei das Filterelement in mindestens einem von mindestens einer Komponente durchströmten Leitungssystem angeordnet ist, wobei die Komponente ausgewählt ist aus der Gruppe bestehend aus dem Desinfektionswirkstoff; mindestens einer den Desinfektionswirkstoff enthaltenden Wirklösung; mindestens einer der reaktiven Komponenten; mindestens einer der reaktiven Komponenten, gemischt mit mindestens einem Trägerstoff, insbesondere Wasser; mindestens einem bei einer Reaktion der reaktiven Komponenten entstehenden Nebenprodukt.

Ausführungsform 47 Reinigungs- und Desinfektionsgerät nach der vorhergehenden Ausführungsform, weiterhin umfassend mindestens einen Drucksensor, wobei der Drucksensor eingerichtet ist, um einen Druck in dem Leitungssystem stromaufwärts und stromabwärts des Filterelements zu erfassen, insbesondere mindestens einen Differenzdruck.

Ausführungsform 48 Reinigungs- und Desinfektionsgerät nach der vorhergehenden Ausführungsform, wobei die Steuerung eingerichtet ist, um mindestens ein Drucksignal des Drucksensors zu erfassen.

Ausführungsform 49 Reinigungs- und Desinfektionsgerät nach der vorhergehenden Ausführungsform, wobei die Steuerung in mindestens einer der folgenden Weisen eingerichtet ist:

- die Steuerung ist eingerichtet, um den Desinfektionsschritt entsprechend des mindestens einen Drucksignals zu steuern;
- die Steuerung ist eingerichtet, um das Drucksignal zu überwachen und bei Abweichungen des Drucksignals von mindestens einem vorgegebenen Normwert, mindestens einem vorgegebenen Normverlauf oder mindestens einem vorgegebenen Normbereich mindestens eine Information an einen Benutzer auszugeben, insbesondere mindestens eine Warnung.

Ausführungsform 50 Reinigungs- und Desinfektionsgerät nach einer der vorhergehenden, ein Reinigungs- und Desinfektionsgerät betreffenden Ausführungsformen, wobei das Reinigungs- und Desinfektionsgerät eingerichtet ist, um die Beaufschlagungsvorrichtung für den mindestens einen Waschschritt einzusetzen, wobei das Reinigungs- und Desinfektionsgerät weiterhin mindestens eine Desinfektionsbeaufschlagungsvorrichtung aufweist, wobei die Desinfektionsbeaufschlagungsvorrichtung von der Beaufschlagungsvorrichtung getrennt ausgebildet ist und wobei das Reinigungs- und Desinfektionsgerät eingerichtet ist, um die Desinfektionsbeaufschlagungsvorrichtung bei dem Desinfektionsschritt einzusetzen.

Ausführungsform 51 Reinigungs- und Desinfektionsgerät nach der vorhergehenden Ausführungsform, wobei die Desinfektionsbeaufschlagungsvorrichtung mindestens eine Sprühdüse aufweist, wobei das Reinigungs- und Desinfektionsgerät eingerichtet ist, um mittels der Sprühdüse mindestens eine Komponente auf den Behälter aufzubringen, wobei die Komponente ausgewählt ist aus der Gruppe bestehend aus den reaktiven Komponenten, dem Desinfektionswirkstoff und mindestens einem Hilfsstoff, insbesondere einem Trägerstoff.

Ausführungsform 52 Reinigungs- und Desinfektionsgerät nach einer der beiden vorhergehenden Ausführungsformen, wobei die Desinfektionsbeaufschlagungsvorrichtung mindestens einen Vernebler aufweist, wobei der Vernebler eingerichtet ist, um mindestens ein fluides Medium zu erzeugen, ausgewählt aus der Gruppe bestehend aus einem Dampf und einem Aerosol, wobei das fluide Medium mindestens eine Komponente aufweist, ausgewählt aus der Gruppe bestehend aus den reaktiven Komponenten, dem Desinfektionswirkstoff und mindestens einem Hilfsstoff, insbesondere einem Trägerstoff, wobei die Desinfektionsbeaufschlagungsvorrichtung optional weiterhin mindestens ein Gebläse, insbesondere ein Umwälzgebläse, aufweist, um das von dem Vernebler erzeugte fluide Medium umzuwälzen, insbesondere innerhalb der Reinigungskammer.

Ausführungsform 53 Reinigungs- und Desinfektionsgerät nach einer der vorhergehenden, ein Reinigungs- und Desinfektionsgerät betreffenden Ausführungsformen, wobei die Reinigungskammer mindestens eine Klapptür mit mindestens einer Halterung für den Behälter aufweist, wobei das Reinigungs- und Desinfektionsgerät derart eingerichtet ist, dass bei einem Schließen der Klapptür ein Inhalt des Behälters in den Abfluss entleert wird.

Ausführungsform 54 Reinigungs- und Desinfektionsgerät nach einer der vorhergehenden, ein Reinigungs- und Desinfektionsgerät betreffenden Ausführungsformen, weiterhin umfassend mindestens einen Bypass, wobei Gase aus der Reinigungskammer durch den Bypass in den Abfluss stromabwärts des Geruchsverschlusses ableitbar sind.

Ausführungsform 55 Reinigungs- und Desinfektionsgerät nach der vorhergehenden Ausführungsform, weiterhin umfassend mindestens eine Verdrängungsvorrichtung, wobei die Verdrängungsvorrichtung eingerichtet ist, um Gase aus der Reinigungskammer durch den Bypass in den Abfluss zu leiten, insbesondere eine Verdrängungsvorrichtung ausgewählt aus der Gruppe bestehend aus einem Lüfter, einer Absaugung, einem Gebläse und einer Druckgasquelle.

Ausführungsform 56 Reinigungs- und Desinfektionsgerät nach einer der vorhergehenden Ausführungsformen, weiterhin umfassend mindestens eine Umwandlungsvorrichtung, wobei die Umwandlungsvorrichtung eingerichtet ist, um Gase aus der Reinigungskammer, insbesondere Stickoxid-haltige Gase, chemisch und/oder physikalisch und/oder biologisch aufzubereiten.

Ausführungsform 57 Reinigungs- und Desinfektionsgerät nach der vorhergehenden Ausführungsform, wobei die Umwandlungsvorrichtung mindestens eine Vorrichtung aufweist, ausgewählt aus der Gruppe bestehend aus einem Katalysator, einem Filter und einer Gaswäsche.

Ausführungsform 58 Reinigungs- und Desinfektionsgerät nach einer der beiden vorhergehenden Ausführungsformen, wobei das Reinigungs- und Desinfektionsgerät eingerichtet ist, um die Gase, nach Aufbereitung durch die Umwandlungsvorrichtung, weiterzuleiten werden in einer Weise, ausgewählt aus der Gruppe bestehend aus: die Gase werden zurück in die Reinigungskammer geleitet; die Gase werden in eine Umgebung abgelassen; die Gase werden in ein Abluftsystem abgeleitet; die Gase werden in den Abfluss stromabwärts des Geruchsverschlusses abgeleitet.

Kurze Beschreibung der Figuren

Weitere Einzelheiten und Merkmale ergeben sich aus der nachfolgenden Beschreibung von Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.
Im Einzelnen zeigen:

Figur 1    ein Ausführungsbeispiel eines Reinigungs- und Desinfektionsgeräts zur Behandlung mindestens eines Behälters für menschliche Ausscheidungen;

Figur 2    einen Ausschnitt eines weiteren Ausführungsbeispiels eines Reinigungs- und Desinfektionsgeräts;

Figur 3    ein Flussdiagram eines Ausführungsbeispiels eines Verfahrens zur Behandlung mindestens eines Behälters für menschliche Ausscheidungen;

Figur 4    ein zeitlicher Verlauf der Konzentration des Desinfektionswirkstoffs Peroxinitritsäure;

Figur 5    Messergebnisse zur Inaktivierung von C. *difficile* in einem Steckbeckenspüler durch Desinfektion mit *in situ* - erzeugter Peroxinitritsäure; und

Figur 6    ein weiteres Ausführungsbeispiel eines Reinigungs- und Desinfektionsgeräts, mit einem optischen Sensor und einem Filterelement.

Beschreibung der Ausführungsbeispiele

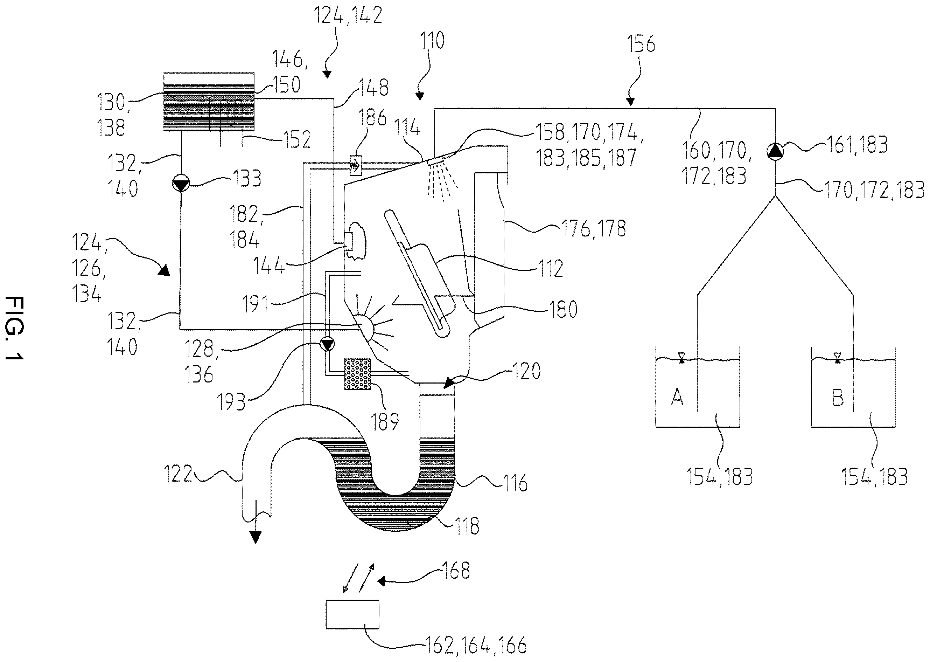

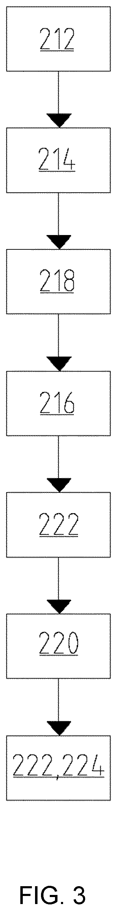

In Figur 1 ist ein Ausführungsbeispiel eines Reinigungs- und Desinfektionsgeräts 110 zur Behandlung mindestens eines Behälters 112 für menschliche Ausscheidungen in einer schematischen Schnittdarstellung gezeigt. Dabei ist das Ausführungsbeispiel des Reinigungs- und Desinfektionsgeräts 110 in einer Reinigungsposition dargestellt. Das Reinigungs- und Desinfektionsgerät 110 ist insbesondere eingerichtet, um ein Verfahren zur Behandlung des mindestens einen Behälters 112 für menschliche Ausscheidungen durchzuführen. Ein mögliches Ausführungsbeispiel eines solchen Verfahrens ist in Figur 3 dargestellt und wird nachfolgend noch näher beschrieben.

Das Reinigungs- und Desinfektionsgerät 110 umfasst mindestens eine Reinigungskammer 114. Im dargestellten Ausführungsbeispiel ist das Reinigungs- und Desinfektionsgerät 110 exemplarisch als Einkammer-Reinigungsgerät ausgestaltet, wobei sämtliche Behandlungsschritte in derselben Reinigungskammer 114 erfolgen.

Weiterhin umfasst das Reinigungs- und Desinfektionsgerät 110 mindestens einen Abfluss 116 mit mindestens einem Geruchsverschluss 118, insbesondere einem Siphonbogen. Wie in Figur 1 dargestellt, kann der Abfluss 116 mindestens eine Öffnung 120 im Bodenbereich der Reinigungskammer 114 umfassen und/oder kann in eine Öffnung 120 münden. Weiterhin kann der Abfluss 116 mindestens ein Abflussrohr 122 umfassen, welches mit der Öffnung 120 verbunden ist. Dieses Abflussrohr 122 kann beispielsweise eine Mündung an der Öffnung 120 in dem Boden der Reinigungskammer 114 aufweisen. Diese Mündung kann insbesondere derart geöffnet sein, dass der Behälterinhalt ohne Hindernisse und allein aufgrund seiner Gewichtskraft in den Abfluss 116 abfließen kann.

Weiterhin umfasst das Reinigungs- und Desinfektionsgerät 110 mindestens eine Beaufschlagungsvorrichtung 124 zur Beaufschlagung des Behälters 112 in der Reinigungskammer 114 mit mindestens einem Reinigungsfluid. Beispielsweise kann die mindestens eine Beaufschlagungsvorrichtung 124 mindestens ein Waschsystem 126 aufweisen, beispielsweise mit mindestens einer Waschdüse 128. Weiterhin kann das Waschsystem 126 mindestens einen Waschtank 130 und mindestens ein Waschleitungssystem 132 aufweisen. Das Waschleitungssystem 132 kann insbesondere die Waschdüse 128 mit dem Waschtank 130 verbinden, so dass ein im Waschtank 130 bevorratetes Reinigungsfluid über das Waschleitungssystem 132 der Waschdüse 128 zugeführt werden kann. Beispielsweise kann weiterhin mindestens eine Pumpe 133 in dem Waschtank 130 und/oder in dem Waschleitungssystem 132 vorgesehen sein, um das Reinigungsfluid der mindestens einen Waschdüse 128 unter Druck zuzuführen. Das Waschsystem 126 kann für einen Waschschritt einsetzbar sein, bei dem beispielsweise der Behälter 112 mit mindestens einer Reinigungsflüssigkeit in Form mindestens einer Waschflüssigkeit beaufschlagt wird.

Für einen optionalen Klarspülschritt kann die Beaufschlagungsvorrichtung 124 beispielsweise optional mindestens ein Klarspülsystem 134 aufweisen, beispielsweise mit mindestens einer Klarspüldüse 136. Das Klarspülsystem 134 kann mindestens einen Klarspültank 138 und mindestens ein Klarspülleitungssystem 140 aufweisen. Das Klarspülleitungssystem 140 kann die Klarspüldüse 136 fluidisch mit dem Klarspültank 138 verbinden, so dass der Behälter 112 über die Klarspüldüse 136 mit einer im Klarspültank 138 bevorrateten Klarspülflüssigkeit beaufschlagt werden kann. Wie in Figur 1 dargestellt, können das Waschsystem 126 und das Klarspülsystem 134 ganz oder teilweise zusammengefasst sein. So kann beispielsweise der Waschtank 130 auch als Klarspültank 138 verwendet werden. Ebenso kann die Waschdüse 128 als Klarspüldüse 136 und das Waschleitungssystem 132 als Klarspülleitungssystem 140 genutzt werden. Auch andere Ausführungen sind jedoch denkbar, beispielsweise Ausführungen, bei denen das Waschsystem 126 und das Klarspülsystem 134 ganz oder teilweise getrennt voneinander ausgebildet sind.

Weiterhin kann die Beaufschlagungsvorrichtung 124 für mindestens einen optionalen Dampfdesinfektionsschritt mindestens ein Dampfsystem 142 aufweisen. Das Dampfsystem 142 kann beispielsweise mindestens eine Dampfdüse 144, mindestens einen Dampferzeuger 146 und mindestens eine Dampfleitung 148 aufweisen. Das Reinigungs- und Desinfektionsgerät 110 kann also weiterhin den mindestens einen Dampferzeuger 146 zur Erzeugung von Dampf umfassen. Der Dampferzeuger 146 kann insbesondere mindestens einen Vorratsbehälter 150, insbesondere für Wasser, und mindestens eine Heizvorrichtung 152 zur Erzeugung des Dampfs, beispielsweise mindestens eine Heizwendel, aufweisen. Der Dampferzeuger 146 kann beispielsweise über die mindestens eine Dampfleitung 148 mit der mindestens einen Dampfdüse 144 an der Reinigungskammer 114 verbunden sein. In diesem Ausführungsbeispiel ist das Dampfsystem 142 zumindest teilweise getrennt von dem Waschsystem 126 und dem Klarspülsystem 134 ausgebildet. Jedoch sind auch hier andere Ausgestaltungen möglich, beispielsweise ein vollständig getrennt von dem Waschsystem 126 und dem Klarspülsystem 134 ausgebildetes Dampfsystem 142 oder eine vollständige Zusammenfassung dieser Systeme. So kann beispielsweise auch das Klarspülleitungssystem 140 ganz oder teilweise auch als Dampfleitung 148 genutzt werden, ebenso wie beispielsweise die Klarspüldüse 136 ganz oder teilweise als Dampfdüse 144 genutzt werden kann.

Das Reinigungs- und Desinfektionsgerät 110 weist weiterhin mindestens zwei Vorratsbehälter 154 zur Aufnahme mindestens zwei reaktiver Komponenten auf. Hierbei kann es sich insbesondere um Vorratsbehälter 154 für Flüssigkeiten handeln. Diese Vorratsbehälter 154 können ganz oder teilweise geschlossen ausgestaltet sein, beispielsweise als Kanister und/oder andere Arten von Vorratsbehältern 154. Insbesondere kann jeweils ein Vorratsbehälter 154 für eine reaktive Komponente A und für eine reaktive Komponente B vorgesehen sein.

Weiterhin kann die Beaufschlagungsvorrichtung 124 für die Durchführung mindestens eines Desinfektionsschritts, insbesondere eines Desinfektionsschritts c. des in Figur 3 näher beschriebenen Verfahrens zur Behandlung des mindestens einen Behälters 112, mindestens ein Desinfektionssystem 156 aufweisen. Das Desinfektionssystem 156 kann beispielsweise die mindestens zwei Vorratsbehälter 154 für die reaktiven Komponenten sowie mindestens eine Desinfektionsdüse 158 zur Beaufschlagung des Behälters 112 mit einer oder beiden der reaktiven Komponenten aufweisen, wobei die Beaufschlagung in flüssiger Form und/oder in gasförmiger Form erfolgen kann. Weiterhin kann das Desinfektionssystem 156 mindestens ein Desinfektionsleitungssystem 160 aufweisen, welches beispielsweise die reaktiven Komponenten sowie optional einen oder mehrere Trägerstoffe der mindestens einen Desinfektionsdüse 158 zuführen kann. So kann das Desinfektionssystem 156 beispielsweise mindestens eine Pumpe 161 aufweisen, die dazu eingerichtet sein kann, eine oder mehrere der reaktiven Komponenten aus den Vorratsbehältern 154 über das Desinfektionsleitungssystem 160 der Desinfektionsdüse 158 unter Druck zuzuführen.

Das Reinigungs- und Desinfektionsgerät 110 weist weiterhin mindestens eine Steuerung 162 zum Ansteuern mindestens eines Reinigungsprogramms auf. Für mögliche Ausgestaltungen des Reinigungsprogramms wird auf das in Figur 3 näher beschriebene Verfahren zur Behandlung des mindestens einen Behälters 112 für menschliche Ausscheidungen verwiesen. Die Steuerung 162 kann zentral oder auch dezentral ausgestaltet sein und/oder kann beispielsweise mindestens eine Datenverarbeitungsvorrichtung 164 umfassen, welche programmtechnisch eingerichtet ist, um das Reinigungsprogramm zu steuern. Beispielsweise kann die Datenverarbeitungsvorrichtung 164 eingerichtet sein, um einen, mehrere oder alle Programmparameter des Reinigungsprogramms einzustellen, beispielsweise in einer vorgegebenen Reihenfolge und/oder mit einem vorgegebenen Zeitschema. Die Steuerung 162 kann zentral und/oder einstückig ausgestaltet sein oder kann auch dezentral ausgestaltet sein und mehrere Steuerungskomponenten umfassen. Beispielsweise kann die Steuerung 162 einen oder mehrere Prozessoren 166 umfassen, die optional drahtlos oder drahtgebunden miteinander verbunden sein können, um Informationen und/oder Befehle auszutauschen. Die mindestens eine Steuerung 162, beispielsweise die mindestens eine optionale Datenverarbeitungsvorrichtung 164, kann allgemein ganz oder teilweise in ein gemeinsames Modul mit der mindestens einen Reinigungskammer 114 integriert sein, beispielsweise in ein gemeinsames Gehäuse. Alternativ oder zusätzlich kann die mindestens eine Steuerung 162, beispielsweise die mindestens eine Datenverarbeitungsvorrichtung 164, auch ganz oder teilweise außerhalb eines die Reinigungskammer 114 umfassenden Moduls des Reinigungs- und Desinfektionsgeräts 110 angeordnet sein, beispielsweise außerhalb eines die Reinigungskammer 114 umschließenden Gehäuses, beispielsweise als externe Steuerung. Die mindestens eine Steuerung 162 kann eine oder mehrere Steuerungskomponenten umfassen, beispielsweise mehrere Datenverarbeitungsvorrichtungen 164, die beispielsweise über mindestens eine Schnittstelle und/oder mindestens eine Datenverbindung 168 miteinander verbunden sein können, beispielsweise um Daten und/oder allgemeine Informationen und/oder Steuerbefehle auszutauschen.

Die Steuerung 162 kann insbesondere eingerichtet sein, um zumindest die Verfahrensschritte b. und c., sowie optional die Verfahrensschritte d. und/oder e. des in Figur 3 näher beschriebenen Verfahrens zur Behandlung des mindestens einen Behälters 112 für menschliche Ausscheidungen, durchzuführen, insbesondere als Programmschritte des Reinigungsprogramms.

Das Reinigungs- und Desinfektionsgerät 110 kann weiterhin mindestens eine Mischvorrichtung 170 aufweisen. Die Mischvorrichtung 170 kann eingerichtet sein, um die reaktiven Komponenten zu mischen. Insbesondere kann die Mischvorrichtung 170 eingerichtet sein, um die reaktiven Komponenten vor der Beaufschlagung des Behälters 112 zu mischen, insbesondere in mindestens einer Vorrichtung ausgewählt aus der Gruppe bestehend aus einer Mischkammer, einer Mischstrecke, einer Pumpe und einer Mischdüse. In diesem Ausführungsbeispiel umfasst die Mischvorrichtung 170 mindestens eine Mischstrecke 172. Die Mischstrecke 172 kann insbesondere mindestens einen fluidischen Leiter umfassen, welcher von den reaktiven Komponenten, alleine oder unter Zusatz beispielsweise von einem oder mehreren Trägerstoffen und/oder Zusatzstoffen, durchströmt werden kann, beispielsweise mindestens ein Strömungsrohr und/oder mindestens eine Strömungsdüse. Alternativ oder zusätzlich kann die Mischvorrichtung 170 auch mindestens eine Mischdüse 174 umfassen. Die Mischdüse 174 kann allgemein mindestens eine Düse sein, welche eingerichtet ist, um mindestens eine der reaktiven Komponenten oder auch die mindestens zwei reaktiven Komponenten, alleine oder auch unter Zusatz beispielsweise eines oder mehrerer Trägerstoffe und/oder Zusatzstoffe, gemeinsam zu verdüsen, so dass diese auf den Behälter 112 auftreffen und beispielsweise auf einer Strecke zwischen der Mischdüse 174 und dem Behälter 112 miteinander in Kontakt kommen und beispielsweise auf dieser Strecke miteinander reagieren können oder auch auf der Oberfläche des Behälters 112 miteinander reagieren können.

Zusätzlich kann das Reinigungs- und Desinfektionsgerät 110 insbesondere mindestens eine Tür 176 zu der Reinigungskammer 114 aufweisen. In diesem Ausführungsbeispiel ist die Tür 176 als Klapptür 178 ausgestaltet. Beispielsweise kann die Klapptür 178 eine horizontale Schwenkachse aufweisen. Die Klapptür 178 kann insbesondere mindestens einer Halterung 180 für den mindestens einen Behälter 112 aufweisen, so dass dieser beispielsweise mit der Tür 176 verbunden sein kann. Das Reinigungs- und Desinfektionsgerät 110 kann insbesondere derart eingerichtet sein, dass bei einem Schließen der Klapptür 178 ein Inhalt des Behälters 112 in den Abfluss 116 entleert wird, beispielsweise durch ein Kippen des Behälters 112.

Weiterhin kann das Reinigungs- und Desinfektionsgerät 110 mindestens einen Bypass 182 aufweisen. Wie in Figur 1 dargestellt, kann der Bypass 182 beispielsweise mindestens eine Rohrleitung 184 aufweisen, welche die Reinigungskammer 114 und den Abfluss 116 stromabwärts des Geruchsverschlusses 118, insbesondere des Siphons, verbindet. Dabei können Gase aus der Reinigungskammer 114 durch den Bypass 182 in den Abfluss 116 stromabwärts des Geruchsverschlusses 118 ableitbar sein. Das Reinigungs- und Desinfektionsgerät 110 kann weiterhin mindestens eine Verdrängungsvorrichtung aufweisen, welche in Figur 1 nicht dargestellt ist. Die Verdrängungseinrichtung kann eingerichtet sein, um Gase aus der Reinigungskammer 114 durch den Bypass 182 in den Abfluss 116 abzuleiten, insbesondere zu verdrängen. Die Verdrängungsvorrichtung kann insbesondere mindestens eine Vorrichtung aufweisen, ausgewählt aus der Gruppe bestehend aus einem Lüfter, einem Gebläse, einer Absaugvorrichtung und einer Druckgasquelle. Der Bypass 182 kann weiterhin mindestens ein Rückschlagventil 186 aufweisen und/oder mindestens eine andere Art von Ventil, welches verhindert, dass Gase aus dem Abfluss 116 zurück in die Reinigungskammer 114 strömen können.

Das Reinigungs- und Desinfektionsgerät 110 kann weiterhin insbesondere mindestens eine Umwandlungsvorrichtung 189 zur chemischen und/oder physikalischen und/oder biologischen Aufbereitung zumindest eines Teils der Gase aus der Reinigungskammer 114 aufweisen. Diese Umwandlungsvorrichtung 189 kann beispielsweise mindestens eine Vorrichtung umfassen, ausgewählt aus der Gruppe bestehend aus einem Katalysator, einem Filter und einer Gaswäsche. Beispielsweise kann die Umwandlungsvorrichtung 189 in einem von der Reinigungskammer 114 abzweigenden Aufbereitungsrohr 191 angeordnet sein, welches getrennt von dem Bypass 182 ausgebildet sein kann oder welches auch ganz oder teilweise mit dem Bypass 182 zusammengefasst sein kann. Es kann beispielsweise eine Pumpe 193 vorgesehen sein, beispielsweise eine Umwälzpumpe, welche Gase aus der Reinigungskammer 114 ansaugt und über die Umwandlungsvorrichtung 189 leitet. Anschließend können die so aufbereiteten Gase zurück in die Reinigungskammer 114 geleitet werden, wie in Figur 1 dargestellt, können in den Abfluss 116 abgeleitet werden oder können auch in die Umgebung oder in ein Abluftsystem abgeleitet werden. Auf diese Weise können beispielsweise Stickoxide in den Gasen reduziert werden. Die in Figur 1 gezeigte Vorrichtung kann beispielsweise in einem Umwälzbetrieb betrieben werden. Die Pumpe 193 kann beispielsweise durch die Steuerung 162 angesteuert werden, sodass eine Aufbereitung der Gase durch die Umwandlungsvorrichtung 189 als einer oder mehrere Umwandlungsschritte in das Verfahren integriert werden können, beispielsweise nach Durchführung des Desinfektionsschritts c..

Das Reinigungs- und Desinfektionsgerät 110 kann mindestens eine Desinfektionsbeaufschlagungsvorrichtung 183 aufweisen. Die Desinfektionsbeaufschlagungsvorrichtung 183 kann Bestandteil der Beaufschlagungsvorrichtung 124 sein. Alternativ kann die Desinfektionsbeaufschlagungsvorrichtung 183 auch getrennt von der Beaufschlagungsvorrichtung 124 ausgebildet sein, wie in dem Ausführungsbeispiel gemäß Figur 1 gezeigt. So kann beispielsweise die Desinfektionsbeaufschlagungsvorrichtung 183 die mindestens eine Desinfektionsdüse 158 aufweisen, welche beispielsweise von der mindestens einen Waschdüse 128 getrennt ausgebildet sein kann. Auf diese Weise kann beispielsweise eine Vermischung von Waschflüssigkeit mit einem oder mehreren Fluiden des Desinfektionsschritts vermieden werden. Das Reinigungs- und Desinfektionsgerät 110 kann insbesondere eingerichtet sein, um die Desinfektionsbeaufschlagungsvorrichtung 183 bei einem Desinfektionsschritt einzusetzen.

Die Desinfektionsbeaufschlagungsvorrichtung 183 kann insbesondere als Bestandteil der mindestens einen Desinfektionsdüse 158 mindestens eine Sprühdüse 185 aufweisen. Das Reinigungs- und Desinfektionsgerät 110 kann eingerichtet sein, um mittels der Sprühdüse 185 mindestens eine Komponente auf den Behälter 112 aufzubringen. Die Komponente kann ausgewählt sein aus der Gruppe bestehend aus den reaktiven Komponenten, einem Desinfektionswirkstoff und mindestens einem Hilfsstoff, insbesondere mindestens einem Trägerstoff. Die Desinfektionsbeaufschlagungsvorrichtung 183 kann insbesondere weiterhin, alternativ oder zusätzlich zu der mindestens einen Sprühdüse 185, mindestens einen Vernebler 187 aufweisen, welcher beispielsweise eingerichtet sein kann, um mindestens ein fluides Medium zu erzeugen, ausgewählt aus der Gruppe bestehend aus einem Dampf und einem Aerosol. Das fluide Medium kann weiterhin durch mindestens ein optionales Gebläse, insbesondere mindestens ein Umwälzgebläse, umgewälzt werden, insbesondere in der Reinigungskammer 114. Das fluide Medium kann insbesondere mindestens eine Komponente aufweisen, ausgewählt aus der Gruppe bestehend aus den reaktiven Komponenten, dem Desinfektionswirkstoff und mindestens einem Hilfsstoff, insbesondere mindestens einem Trägerstoff.

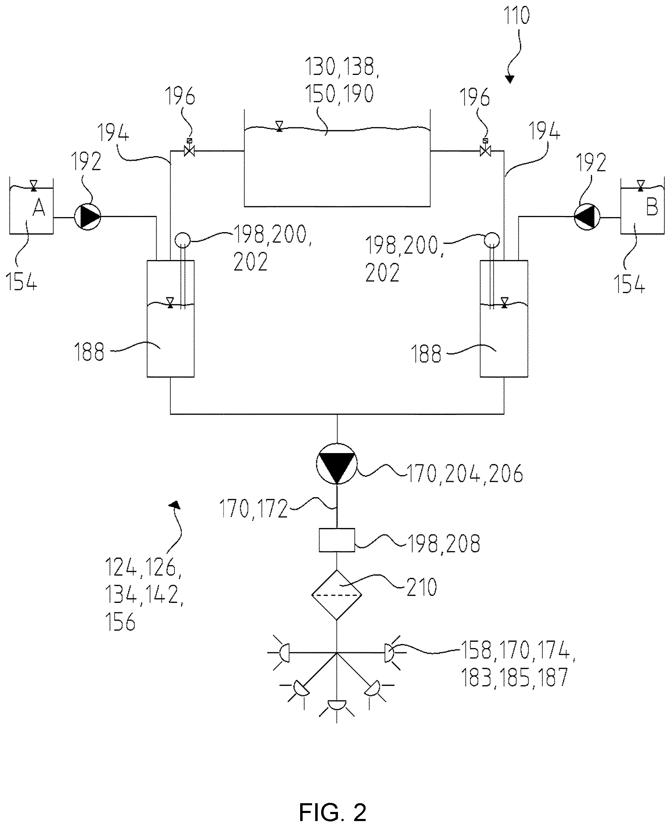

In Figur 2 ist ein Ausschnitt eines weiteren alternativen Ausführungsbeispiels des Reinigungs- und Desinfektionsgeräts 110 in einer schematischen Ansicht gezeigt. In diesem Ausführungsbeispiel ist insbesondere der Aufbau der Beaufschlagungsvorrichtung 124 und der Aufbau der Desinfektionsbeaufschlagungsvorrichtung 183 variiert. Die übrige Ausgestaltung dieses Reinigungs- und Desinfektionsgeräts 110 entspricht weitestgehend dem Ausführungsbeispiel aus Figur 1, so dass an dieser Stelle auf die Beschreibung von Figur 1 verwiesen werden kann.

Wie in Figur 2 dargestellt, kann das Reinigungs- und Desinfektionsgerät 110, insbesondere die Desinfektionsbeaufschlagungsvorrichtung 183, mindestens einen Aufbereitungsbehälter 188 zur Aufbereitung mindestens einer der reaktiven Komponenten aufweisen. Dieser Aufbereitungsbehälter 188 kann allgemein ein Behälter sein, welcher mit mindestens einem der Vorratsbehälter 154 verbunden ist. Beispielsweise kann jeweils ein Aufbereitungsbehälter 188 für die reaktive Komponente A und für die reaktive Komponente B vorgesehen sein, welcher mit dem entsprechenden Vorratsbehälter 154 verbunden sein kann. Der Aufbereitungsbehälter 188 kann weiterhin mit mindestens einem Vorratsbehälter 190 für mindestens einen Trägerstoff verbunden sein, insbesondere einem Vorratsbehälter für Wasser. Dabei kann der Vorratsbehälter 190 für den mindestens einen Trägerstoff auch ganz oder teilweise mit dem Waschtank 130, dem Klarspültank 138 und/oder dem Vorratsbehälter 150 des Dampferzeugers 146 zusammengefasst sein. So können, wie in diesem Ausführungsbeispiel gezeigt, das Waschsystem 126, das Klarspülsystem 134, das Dampfsystem 142 und optional das Desinfektionssystem 156 teilweise oder sogar vollständig zusammengefasst sein.

Das Reinigungs- und Desinfektionsgerät 110, insbesondere die Desinfektionsbeaufschlagungsvorrichtung 183, kann eingerichtet sein, um in dem Aufbereitungsbehälter 188 die mindestens eine reaktive Komponente mit dem Trägerstoff zu mischen. Somit kann der Aufbereitungsbehälter 188 dazu dienen, die reaktive Komponente oder mindestens eine der reaktiven Komponenten in den mindestens einen Trägerstoff einzubringen, wodurch beispielsweise eine Konzentration in gezielter Weise eingestellt werden kann und wodurch auch beispielsweise eine Verdüsung oder Verneblung der mindestens einen reaktiven Komponente erleichtert werden kann. Alternativ oder zusätzlich kann die die mindestens eine reaktive Komponente mit dem mindestens einen Trägerstoff auch auf andere Weise gemischt werden, beispielsweise in mindestens einer gemeinsamen Leitung und/oder Pumpe, beispielsweise in einer gemeinsamen Zuführleitung zu dem Aufbereitungsbehälter 188 und/oder in einer stromabwärts des Aufbereitungsbehälters 188 angeordneten Leitung.

Das Reinigungs- und Desinfektionsgerät 110, insbesondere die Desinfektionsbeaufschlagungsvorrichtung 183, kann insbesondere mindestens eine Dosierpumpe 192 aufweisen, welche eingerichtet ist, um eine vorgebbare Menge der mindestens einen reaktiven Komponente in den Aufbereitungsbehälter 188 einzubringen. Beispielsweise kann die Dosierpumpe 192 durch die Steuerung 162 angesteuert werden, insbesondere auch beispielsweise hinsichtlich des Zeitpunkts der Dosierung und/oder hinsichtlich der vorgegebenen Menge der mindestens einen reaktiven Komponente und/oder des Zusatzstoffs. Die Dosierpumpe 192 kann beispielsweise zeitgesteuert sein.

Der Aufbereitungsbehälter 188 kann insbesondere über mindestens eine Zuleitung 194, die optional mindestens ein Ventil 196 aufweist, mit dem Vorratsbehälter 190 verbunden sein. Das Ventil 196 kann beispielsweise ein 2/2-Wege Ventil umfassen. Alternativ zur Verwendung des Ventils 196 sind auch beispielsweise Konstruktionen ohne Ventil denkbar, beispielsweise mit einem Überlauf, beispielsweise indem der Aufbereitungsbehälter 188 mittels eines Überlaufs aus dem Waschtank 130 gefüllt wird.

Das Reinigungs- und Desinfektionsgerät 110 kann insbesondere eingerichtet sein, um den Trägerstoff über die Zuleitung 194 in den Aufbereitungsbehälter 188 einzubringen. Dieses Einbringen kann beispielsweise gravitationsgetrieben oder auch angetrieben durch mindestens eine weitere Dosierpumpe sein. Beispielsweise kann das Reinigungs- und Desinfektionsgerät 110 eingerichtet sein, insbesondere mittels der Steuerung 162, um das Ventil 196 entsprechend anzusteuern, um beispielsweise eine vorgegebene Menge des Trägerstoffs über die Zuleitung 194 in den Aufbereitungsbehälter 188 einzubringen. Beispielsweise kann dementsprechend eine Zeitsteuerung vorgesehen sein, um die Menge an Trägerstoff zu definieren.

In diesem Ausführungsbeispiel kann das Reinigungs- und Desinfektionsgerät 110 also mindestens zwei der Aufbereitungsbehälter 188 aufweisen, wobei in den Aufbereitungsbehältern 188 unterschiedliche reaktive Komponenten aufbereitbar sind, indem diese in mindestens einen Trägerstoff eingebracht werden.

Weiterhin kann das Reinigungs- und Desinfektionsgerät 110 mindestens einen Sensor 198 umfassen. Der Sensor 198 kann beispielsweise in dem mindestens einen Aufbereitungsbehälter 188 angeordnet sein. Wie in Figur 2 dargestellt, kann insbesondere jeweils ein Sensor 198 in jedem der Aufbereitungsbehälter 188 angeordnet sein. Der Sensor 198 in dem Aufbereitungsbehälter 188 kann in diesem Ausführungsbeispiel einen Füllstandssensor 200 umfassen. Beispielsweise kann der Sensor 198 einen Leitfähigkeitssensor 202 umfassen, der dazu eingerichtet ist, einen Füllstand in dem Aufbereitungsbehälter 188 zu detektieren. Zusätzliche Sensoren und/oder andere Ausgestaltungen des Sensors 198 sind jedoch auch möglich.

Das Reinigungs- und Desinfektionsgerät 110 kann weiterhin mindestens eine Pumpe 204 aufweisen, welche eingerichtet ist, um die mit dem mindestens einen Trägerstoff gemischten reaktiven Komponenten aus den Aufbereitungsbehältern 188 heraus zu pumpen und auf den Behälter 112 aufzubringen. Die Pumpe 204 kann auch selbst als Mischvorrichtung 170 dienen oder Bestandteil der Mischvorrichtung 170 sein. Die Pumpe 204 kann insbesondere als Kreiselpumpe ausgestaltet sein, um eine ausreichende Mischungswirkung zu erzielen. Die Pumpe 204 kann insbesondere Bestandteil der Beaufschlagungsvorrichtung 124 sein. Hierbei kann eine gesonderte Desinfektionspumpe 206 vorgesehen sein, welche beispielsweise einen erforderlichen Druck generiert und/oder welche die aufbereiteten reaktiven Komponenten der mindestens einen Desinfektionsdüse 158 zuführt.

Nach der Aufbereitung einer oder mehrerer der reaktiven Komponenten können diese gemischt werden. So kann das Reinigungs- und Desinfektionsgerät 110 insbesondere eingerichtet sein, um die mit dem mindestens einen Trägerstoff gemischten reaktiven Komponenten aus den Aufbereitungsbehältern 188 zusammenzuführen und einen Desinfektionswirkstoff zu bilden. Dieses Zusammenführen kann ein Mischen der reaktiven Komponenten umfassen. Das Zusammenführen kann außerhalb des Behälters 112 erfolgen, beispielsweise in der Mischvorrichtung 170 oder auch beispielsweise auf dem Behälter 112, beispielsweise indem auf der Behälteroberfläche die aufbereiteten reaktiven Komponenten zusammengeführt werden. Auch in diesem Ausführungsbeispiel kann die Mischvorrichtung 170 die Mischstrecke 172 und/oder die Mischdüse 174 und/oder die Pumpe 204 umfassen.

Das Reinigungs- und Desinfektionsgerät 110 kann zusätzlich einen oder mehrere weitere Sensoren 198 umfassen, welche beispielsweise, wie in Figur 2 gezeigt, an der Mischvorrichtung 170, insbesondere an der Mischstrecke 172, angeordnet sind. Auch andere Anordnungen des Sensors 198 sind denkbar, beispielsweise in einem Bypass zur Mischstrecke 172 und/oder einem Abzweig zur Mischstrecke 172. Der Sensor 198 kann hier insbesondere einen optischen Sensor 208 umfassen. Der optische Sensor 208 kann dazu eingerichtet sein, eine Gelbfärbung zu detektieren. Eine Gelbfärbung von Wasser bei Anwesenheit von Nitrat, Nitrit, Stickoxiden, Peroxinitritsäure oder Peroxinitrit kann somit qualitativ und/oder quantitativ optisch erfasst werden. Auch andere Sensoren 198 können hier angeordnet sein, beispielsweise ein Drucksensor und/oder ein Durchflusssensor. Der Sensor 198 kann, alternativ oder zusätzlich, auch direkt in einer Kammerwand der Reinigungskammer 114 eingebaut sein, beispielsweise um den Zustand des Desinfektionsprozesses zu analysieren und/oder zu überwachen. In Abhängigkeit von den Inhaltsstoffen der Reinigungskammer 114 kann beispielsweise ein Öffnen der Tür 176 freigegeben werden, beispielsweise indem die Steuerung 162 ein Sensorsignal des Sensors 198, insbesondere des Sensors 198 in der Wand der Reinigungskammer 114, überwacht und, wenn eine oder mehrere Freigabebedingungen erfüllt sind, die Tür 176 freigibt. So kann beispielsweise verhindert werden, dass Schadstoffe aus der Reinigungskammer 114 in die Umgebung gelangen. Alternativ oder zusätzlich kann die Steuerung 162 auch den Ablauf des Desinfektionsschritts in der Reinigungskammer 114 überwachen. Andere Ausgestaltungen des Sensors 198 sind jedoch ebenfalls möglich.

Wie in Figur 2 exemplarisch gezeigt, kann das Reinigungs- und Desinfektionsgerät 110 weiterhin optional mindestens einen Filter 210 aufweisen. Der Filter 210 kann dazu eingerichtet sein, das Reinigungsfluid, die Klarspülflüssigkeit, den Trägerstoff mit den reaktiven Komponenten und/oder das Desinfektionsmittel zu filtern. Der Filter 210 kann insbesondere Bestandteil der Beaufschlagungsvorrichtung 124 und/oder der Desinfektionsbeaufschlagungsvorrichtung 183 sein.

In Figur 3 ist ein Flussdiagram eines Ausführungsbeispiels eines Verfahrens zur Behandlung des mindestens einen Behälters 112 für menschliche Ausscheidungen gezeigt. Das Verfahren kann allgemein in mindestens einem Reinigungs- und Desinfektionsgerät 110 durchgeführt werden, insbesondere gemäß einem oder mehreren der in den Figuren 1 und 2 beschriebenen Ausführungsbeispiele. Andere Ausführungen des Reinigungs- und Desinfektionsgerät 110 sind jedoch ebenfalls möglich.

Das Verfahren umfasst die nachfolgend näher genannten Schritte. Diese Schritte können in der genannten Reihenfolge durchgeführt werden. Auch eine andere Reihenfolge ist jedoch grundsätzlich möglich. Weiterhin können zwei oder mehr der genannten Verfahrensschritte zeitlich überlappend oder gleichzeitig durchgeführt werden. Weiterhin können einer oder mehrere der genannten Verfahrensschritte einfach oder auch wiederholt durchgeführt werden. Das Verfahren kann über die genannten Schritte hinaus weitere Verfahrensschritte umfassen, welche nicht genannt sind.

Das Verfahren umfasst die folgenden Schritte:

a. (gekennzeichnet durch Bezugszeichen 212) mindestens einen Entleerungsschritt, umfassend ein Entleeren des Behälterinhalts innerhalb der mindestens einen Reinigungskammer 114 in den mindestens einen Abfluss 116, wobei der Abfluss 116 den mindestens einen Geruchsverschluss 118 umfasst;

b. (gekennzeichnet durch Bezugszeichen 214) mindestens einen Waschschritt, umfassend mindestens eine Beaufschlagung des Behälters 112 mit mindestens einer Reinigungsflüssigkeit in der Reinigungskammer 114; und

c. (gekennzeichnet durch Bezugszeichen 216) mindestens einen Desinfektionsschritt, umfassend mindestens ein Mischen mindestens zweier reaktiver Komponenten zur Erzeugung mindestens eines Desinfektionswirkstoffs und Beaufschlagung des Behälters 112 mit dem Desinfektionswirkstoff.

Dabei kann das Entleeren des Behälters 112 in Verfahrensschritt a. insbesondere durch eine Änderung einer Lage und/oder Orientierung des Behälters 112 erfolgen, beispielsweise indem der Inhalt des Behälters 112 vollständig oder teilweise in den Abfluss 116 gekippt wird. Diese Änderung der Lage und/oder Orientierung kann beispielsweise dadurch erfolgen, dass der Behälter 112 an der Halterung 180 der Tür 176 des Reinigungs- und Desinfektionsgeräts 110 befestigt ist, welche schwenkbar ist, so dass beim Schwenken die Lageänderung erfolgt. Das Entleeren kann somit durch die eigene Gewichtskraft des Behälterinhalts erfolgen.

Weiterhin kann die Beaufschlagung des Behälters 112 in Verfahrensschritt b. mittels der mindestens einen Beaufschlagungsvorrichtung 124 des Reinigungs- und Desinfektionsgeräts 110 erfolgen. Dabei kann insbesondere ein im Waschtank 130 bevorratetes Reinigungsfluid über die Beaufschlagungsvorrichtung 124 auf den Behälter 112 aufgebracht werden.

In Verfahrensschritt c. kann das Mischen der mindestens zwei reaktiven Komponenten insbesondere mittels der Mischvorrichtung 170 des Reinigungs- und Desinfektionsgeräts 110 erfolgen. Der so erzeugte Desinfektionswirkstoff kann über die Beaufschlagungsvorrichtung 124, insbesondere über die Desinfektionsbeaufschlagungsvorrichtung 183, auf den Behälter 112 aufgebracht werden.

Das Verfahren kann weiterhin folgenden Schritt umfassen:
d. (gekennzeichnet durch Bezugszeichen 218) mindestens einen Klarspülschritt, umfassend mindestens eine Beaufschlagung des Behälters 112 mit mindestens einer Klarspülflüssigkeit.

In diesem Ausführungsbeispiel erfolgt der Klarspülschritt d. vorzugsweise vor dem Desinfektionsschritt c.. Es ist jedoch ebenfalls möglich, dass Klarspülschritt d. nach dem Desinfektionsschritt c. erfolgen kann. Auch mehrere Klarspülschritte sind möglich, beispielsweise jeweils ein Klarspülschritt d. vor und nach dem Desinfektionsschritt c..

Das Verfahren kann weiterhin folgenden Verfahrensschritt umfassen:

e. (gekennzeichnet durch Bezugszeichen 220) mindestens einen, insbesondere dem Klarspülschritt und/oder dem Desinfektionsschritt nachgelagerten, Dampfdesinfektionsschritt, umfassend mindestens eine Beaufschlagung des Behälters 112 mit Dampf.

Insbesondere kann das Verfahren weiterhin mindestens einen Verdrängungsschritt (gekennzeichnet durch Bezugszeichen 222) umfassen. In dem Verdrängungsschritt können Gase aus der Reinigungskammer 114, insbesondere Stickoxid-haltige Gase, abgeleitet werden, insbesondere zwangsweise. Der Verdrängungsschritt kann nach Beendigung des Verfahrensschritts c. durchgeführt werden, beispielsweise jedoch vor Durchführung des optionalen Verfahrensschritts e.

Der Verdrängungsschritt kann jedoch auch mehrfach durchgeführt werden, beispielsweise einmal nach Durchführung des Verfahrensschritts c. und mindestens ein weiteres Mal nach Durchführung des optionalen Verfahrensschritts e. So können beispielsweise in einem ersten Verdrängungsschritt, welcher dem Verfahrensschritt c. nachgelagert ist, Gase aus der Reinigungskammer 114 abgeleitet werden, welche Stickoxide (NOx) enthalten, wohingegen in einem zweiten Verdrängungsschritt, welcher dem Verfahrensschritt e. nachgelagert ist, Dampf aus der Reinigungskammer 114 abgeleitet wird.

In diesem Ausführungsbeispiel kann dementsprechend das Verfahren beispielsweise die Verfahrensschritte a. bis c. sowie optional d. umfassen, gefolgt von einem ersten Verdrängungsschritt, auf welchen wiederum der Verfahrensschritt e. folgt, auf welchen wiederum der zweite Verdrängungsschritt folgt. Auf diese Weise können der Desinfektionsschritt c. und der optionale Dampfdesinfektionsschritt e. klar getrennt werden, und es kann zwischen diesen Desinfektionsschritten c. und e. sowie optional auch nach dem Dampfdesinfektionsschritt e. jeweils mindestens ein Verdrängungsschritt erfolgen. Damit können beispielsweise schädliche Gase aus Verfahrensschritt c. sowie auch optional für das Bedienungspersonal unangenehme Dämpfe und Feuchtigkeitsschwaden vor einem Öffnen der Tür 176 der Reinigungskammer 114 in den Verdrängungsschritten abgeleitet werden, und es kann verhindert werden, dass diese in die Raumluft abgegeben werden. In dem Verdrängungsschritt können die Gase aus der Reinigungskammer 114 insbesondere zwangsweise über den Bypass 182 des Reinigungs- und Desinfektionsgeräts 110 in den Abfluss 116 stromabwärts des Geruchsverschlusses 118 abgeleitet werden.

Wie oben ausgeführt, kann, alternativ oder zusätzlich zu dem Verdrängungsschritt, auch der mindestens eine optionale Umwandlungsschritt durchgeführt werden, beispielsweise mittels der Umwandlungsvorrichtung 189 dieser kann entsprechend auch zu den bezüglich des Verdrängungsschritts beschriebenen Zeitpunkten in dem Verfahren durchgeführt werden. Auch andere Ausgestaltungen sind jedoch möglich.

Weiterhin kann das Verfahren mindestens einen Trocknungsschritt (gekennzeichnet durch Bezugszeichen 224) umfassen. Dieser Trocknungsschritt kann beispielsweise dem Desinfektionsschritt und/oder dem Dampfdesinfektionsschritt nachgelagert sein. Wie in Figur 3 dargestellt, kann das Verfahren beispielsweise zunächst die Verfahrensschritte a. bis c. sowie optional den Verfahrensschritt d. umfassen, wobei Verfahrensschritt d. vorzugsweise dem Verfahrensschritt c. vorgelagert ist. Anschließend kann, nach Durchführung des Verfahrensschritts c. ein erster Verdrängungsschritt durchgeführt werden, gefolgt optional von dem Dampfdesinfektionsschritt e. sowie optional dem mindestens einen zweiten Verdrängungsschritt und dem Trocknungsschritt. Dabei können der zweite Verdrängungsschritt und der Trocknungsschritt ganz oder teilweise als gemeinsame Verfahrensschritte ausgestaltet sein. Auch andere Ausgestaltungen sind möglich, beispielsweise eine Ausgestaltung, bei der der zweite Verdrängungsschritt und der Trocknungsschritt als getrennte Verfahrensschritte ausgestaltet sind.

Ausführungsbeispiel 1: Messung des Konzentrationsverlaufs des Desinfektionswirkstoffs:

Für mikrobiologische Versuche wurde als Desinfektionswirkstoff Peroxinitritsäure verwendet. Als Trägerstoff und Lösungsmittel wurde dabei Wasser eingesetzt. Als Wirklösung wurde somit eine wässrige Lösung von Peroxinitritsäure eingesetzt.

Zum Test der Bildung von Peroxinitritsäure wurde eine Vorrichtung analog zu dem in Figur 1 gezeigten Reinigung- und Desinfektionsgerät 110 verwendet, mit dem Desinfektionssystem 156 und der Desinfektionsbeaufschlagungsvorrichtung 183. Zwischen der Pumpe 161 und den Vorratsbehältern 154 können dabei allgemein optional jeweils Rückschlagventile vorgesehen sein. Weiterhin wurde für den Test zwischen der Pumpe 161 und der Desinfektionsdüse 158 ein Teil der gemischten Flüssigkeit abgeleitet und über ein Drosselventil und eine Verzögerungsstrecke einem in Figur 1 nicht dargestellten optischen Sensor, beispielsweise einem UV-VIS-Spektrometer, zugeführt, um die Konzentration zu überwachen. Da eine direkte, zeitabhängige Messung der Konzentration des Desinfektionswirkstoffs allgemein nur schwer möglich ist, insbesondere die Konzentration auf der Oberfläche des zu reinigenden Behälters 112, dient der optische Sensor, welcher beispielsweise spektroskopisch in einer Durchflussküvette eine Gelbfärbung erfasst, zur Ermittlung eines Nehrungswerts dieser Konzentration. Hierdurch wird eine Quantifizierung der Konzentration des Desinfektionswirkstoffs, beispielsweise von ONOOH, ermöglicht, insbesondere mittels UV-Spektroskopie ermöglicht. Hierbei ist optional durch die Verwendung eines zusätzlichen Ventils oder weiterer Bauelemente dafür Sorge zu tragen, dass die Düsen im Messaufbau mit demselben Druck sowie mit demselben Durchfluss betrieben werden, wie im üblichen Spülprozess. Sofern die Flugzeit der Desinfektionsflüssigkeit von der Düse bis auf das Spülgut nicht vernachlässigbar ist, kann diese im Messaufbau durch eine Verzögerungsstrecke simuliert werden. Die zeitabhängige Absorbanz A ist mit Hilfe der UV/VIS-Durchflussküvette mit einer für den jeweiligen Prozess geeigneten Absorptionslänge L sowie mit einer für den jeweiligen Prozess ausreichend feinen Zeitauflösung $\Delta t$ zu messen.

Die Quantifizierung des Desinfektionswirkstoffs, beispielsweise die Konzentration von ONOOH auf der Oberfläche des Behälters 112, kann anschließend durch eine Ausgleichungsrechnung erfolgen, bei welcher die Konzentration des Desinfektionswirkstoffs, beispielsweise von ONOOH, und gegebenenfalls anderer absorbierender Spezies i als freie Parameter genutzt werden. Hierbei kann beispielsweise eine Modellfunktion

$$A_{\mathrm{Mod}} = ([ONOOH]\epsilon_{ONOOH} + \textstyle\sum_i [i]\epsilon_i)L \qquad\qquad (3)$$

für jeden Messzeitpunkt an die Absorbanz A angepasst werden. Hierbei sind $\varepsilon_{ONOOH}$ bzw. $\varepsilon_i$ die jeweiligen Extinktionskoeffizienten für den Desinfektionswirkstoff, hier beispielsweise ONOOH, bzw. für die Spezies i, welche beispielsweise der Literatur zu entnehmen sind oder für den jeweiligen Versuchsaufbau durch entsprechende Kalibrationsmessungen bestimmt werden können.

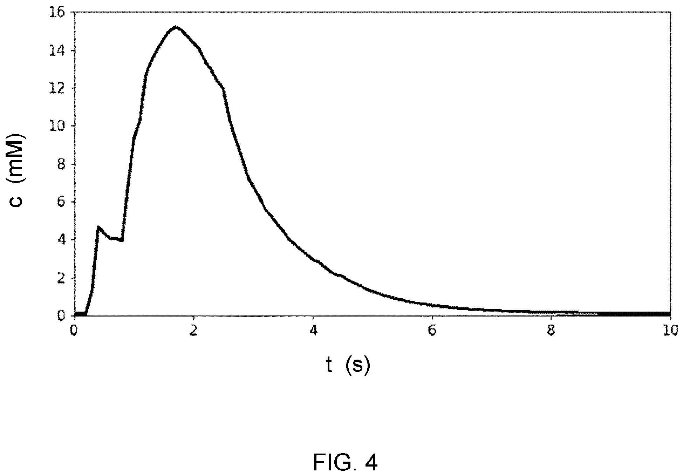

In Figur 4 ist exemplarisch ein Reaktionsverlauf dargestellt, welcher sich bei Verwendung der Ausgangsflüssigkeit A (110 mM $H_2O_2$ und 140 mM $H_3PO_4$ in destilliertem Wasser) und Ausgangsflüssigkeit B (100 mM $NaNO_2$ in destilliertem Wasser) sowie einer Einspritzzeit von 0,8 s ergibt. Dargestellt ist in dieser Figur der Verlauf der Konzentration c von ONOOH über die Messzeit t, wobei die Konzentration c gemäß der obigen Beschreibung optisch ermittelt wurde. Die Einspritzzeit ist in Figur 4 anhand des Konzentrationsplateaus zu Beginn des Prozesses zu erkennen. In diesem Beispiel beträgt der Wirksamkeitsparameter H entsprechend Gleichung (2) oben und der in Figur 4 dargestellten Messung 37,2 mM *s. Zur* weiteren Erhöhung des Wirksamkeitsparameters kann bei Verwendung der gleichen Ausgangsflüssigkeiten die Einspritzzeit verlängert werden und/oder die Reaktionszeit im Schlauchsystem so angepasst werden, dass sich das Plateau bei einer höheren ONOOH-Konzentration bildet.

Ausführungsbeispiel 2: Quantifizierung der Wirksamkeit:

Für mikrobiologische Versuche wurde zur Herstellung der Wirklösung als eine erste reaktive Komponente eine wässrige Lösung A von 300 mM $H_2O_2$ (entsprechend $300\times0.001$ mol/l) eingesetzt. Als Trägerstoff und Lösungsmittel für die erste reaktive Komponente wurde somit ebenfalls Wasser eingesetzt. Als Zusatzstoff wurde der Lösung der ersten reaktiven Komponente weiterhin Phosphorsäure ($H_3PO_4$) in einer Konzentration von 60 mM (entsprechend $60\times0.001$ mol/l) beigefügt, sodass insgesamt eine Lösung mit einem pH-Wert von 1,8 entstand. Weiterhin wurden der ersten Lösung 0.1% eines leicht sauren Klarspülers und Enthärters (Doyen SK22E®, Hersteller etol Eberhard Tripp GmbH, 77728 Oppenau, Deutschland) beigefügt. So entstand eine erste reaktive Lösung.

Als zweite reaktive Komponente wurde eine wässrige Lösung von 100 mM $NaNO_2$ verwendet, mit einem berechneten pH-Wert von 8.

Als Wirksamkeitsparameter für die durchgeführten Versuche wurde ein Wert von 144 mM·s verwendet.

Für die mikrobiologischen Versuche wurden Prüfkörper in Form von Steckbecken mit C. *difficile* - Sporen inokuliert. Hierfür wurden an 5 verschiedenen Positionen jeweils 20 $\mu$l der Sporen-Lösung aufgetragen, gefolgt von einer an Trocknung über 60 Minuten.

Anschließend wurde in der oben beschriebenen Vorrichtung analog zu Figur 1 der Desinfektionsschritt mit der Wirklösung durchgeführt. Über das T-Stück vor der Pumpe 161 wurden die Saugschläuche zusammengeführt und mit der Pumpe 161 verbunden. Im T-Stück wurden die beiden Ausgangsstoffe zum ersten Mal vermischt. Ab diesem Zeitpunkt wurde die Reaktion gestartet. Das restliche Schlauchsystem war dabei exemplarisch so ausgelegt, dass ab dem Zeitpunkt des ersten Kontakts beider Komponenten bis zum Auftreffen auf dem Spülgut näherungsweise 2 s vergingen.

Die Mikroorganismen auf dem Spülgut wurden kontinuierlich über eine Zeitdauer T mit der Wirklösung eingesprüht. Je nach applizierter Menge lag die Zeitdauer T im Experiment entweder bei 6 s bei einem Einsprühvolumen von 90 ml Wirklösung oder bei 12 s bei einem Einsprühvolumen von 180 ml Wirklösung.

Der Wirksamkeitsparameter wurde, ohne Beschränkung anderer Möglichkeiten, in folgender Weise ermittelt. So erreicht, aus der Perspektive der Mikroorganismen, über die Einsprühdauer T zunächst immer ein Flüssigkeitselement mit einer konstanten ONOOH-Konzentration die Oberfläche des Spülguts. Die Konzentration bestimmt sich aus dem Zeitverzug zwischen erstem Kontakt der Desinfektionskomponenten und dem Auftreffzeitpunkt auf der Oberfläche. In dem untersuchten Beispiel trifft das vermischte Desinfektionsmittel nach 2 s auf der Oberfläche auf. Die ONOOH-Konzentration beträgt nach 2 s ca. 12 mM. Damit ergeben sich für die zwei getesteten Einsprühzeiten Habersche Wirksamkeitsparameter von 12 mM x 6 s = 72 mM · s und 12 x 12 s = 144 mM · s.

Nach dem Desinfektionsschritt wurde die Reinigungskammer 114 für 10 Sekunden mit Frischluft befüllt, und anschließend wurde das Steckbecken aus der Reinigungskammer 114 entfernt.

Zur Kontrolle erfolgte eine Einsprühung mit 180 ml destilliertem Wasser.

In einem nächsten Schritt wurden die Sporen unmittelbar nach Entnahme des Steckbeckens aus der Reinigungskammer 114 mit 30 ml Neutralisationsmittel überschichtet und abgelöst. Es folgte eine Ablösung und eine Abdeckung mit einem Deckel, gefolgt von einer Agitation über 30 Minuten auf einem Horizontalschüttler mit 200 rpm.

Anschließend erfolgte eine Quantifizierung des Reduktionsfaktors. Hierfür wurde die wie oben beschrieben gewonnene Rückgewinnungslösung auf einem Nährmedium für 5 Tage bei 36 °C inkubiert. Abschließend wurden die koloniebildenden Einheiten KBE ausgezählt bei den verschiedenen Verdünnungsstufen, und die Reduktionsfaktoren wurden bestimmt.

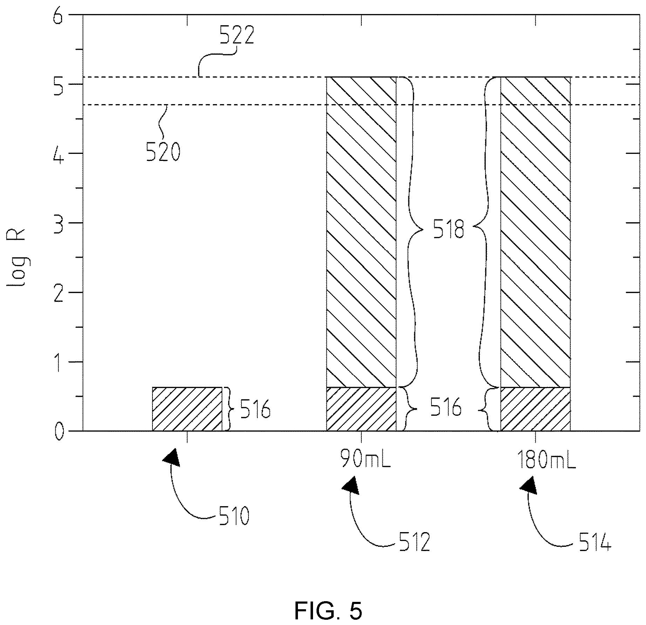

Die Ergebnisse dieser Versuche zur Inaktivierung von *C. difficile* - Sporen in dem Reinigung- und Desinfektionsgerät 110 mit Peroxinitritsäure sind in Figur 5 als Balkendiagramm dargestellt. Dort ist die logarithmische Reduktion log R aufgetragen für drei verschiedene Bedingungen: Bezugsziffer 510 zeigt Ergebnisse von Versuchen, bei denen nur ein Abspülen der Proben erfolgte, Bezugsziffer 512 zeigt Ergebnisse von Versuchen mit einem Einsprühvolumen der oben beschriebenen Wirklösung von 90 ml und einer Einwirkzeit von 6 s, und Bezugsziffer 514 zeigt die entsprechenden Ergebnisse bei einem Einsprühvolumen von 180 ml Wirklösung und einer Einwirkzeit von 12 s.

Dabei sind die Reduktionen jeweils unterteilt in einen reinen Abreicherungsanteil 516 und einen Desinfektionsanteil 518. Beide Anteile summieren sich zum Wirkungsanteil.

Weiterhin sind in Figur 5 zwei Grenzwerte als gestrichelte Linien eingezeichnet. So bezeichnet die Linie 520 die Anforderungen an die Abreicherung gemäß der europäischen Normen EN 17126 und EN 13697. Die Linie 522 bezeichnet die Nachweisgrenze der Reduktion.

Die Ergebnisse zeigen deutlich, dass auch der hartnäckige Krankenhauskeim C. difficile durch das beschriebene Verfahren der *in situ* - Erzeugung des Desinfektionswirkstoffs ONOOH effizient abgetötet werden kann, wobei die entsprechenden Normen übererfüllt werden.

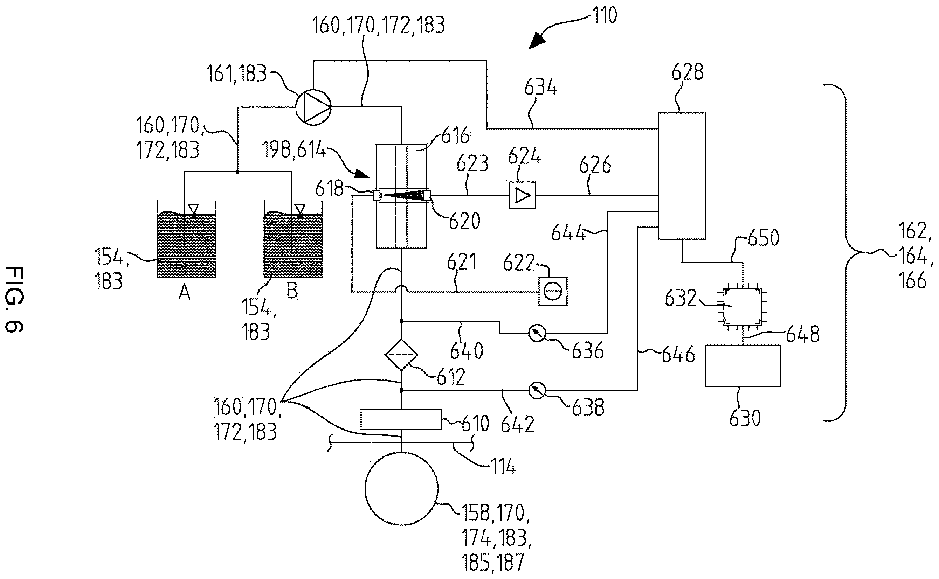

In Figur 6 ist schematisch ein weiteres Ausführungsbeispiel eines Reinigungs- und Desinfektionsgeräts 110 dargestellt. Die Darstellung ist lediglich teilweise, wobei bezüglich der übrigen Teile des Reinigungs- und Desinfektionsgeräts 110 beispielsweise auf andere Ausführungsbeispiele verwiesen werden kann, insbesondere auf das Beispiel gemäß Figur 1 oder auch das Beispiel gemäß Figur 2. Wiederum umfasst das Reinigungs- und Desinfektionsgerät 110 eine Desinfektionsbeaufschlagungsvorrichtung 183 mit Vorratsbehältern 154. Diese können über ein Desinfektionsleitungssystem 160 und optional eine Pumpe 161, sowie, wie auch in den anderen Ausführungsbeispielen, über mindestens einen optionalen Verteiler 610, mit mindestens einer Desinfektionsdüse 158 des Reinigungs- und Desinfektionsgeräts 110 verbunden sein. Bezüglich der möglichen Optionen der Mischung der Komponenten aus den Vorratsbehältern 154 kann auf die obige Beschreibung der Figuren 1 und 2 verwiesen werden. Grundsätzlich sind jegliche der oben genannten Optionen der Mischung der Komponenten auch in dem vorliegenden Ausführungsbeispiel realisierbar, und/oder das vorliegende Ausführungsbeispiel kann beispielsweise auch in die Ausführungsbeispiele der Figuren 1 und/oder 2 integriert werden.

In dem in Figur 6 gezeigten Ausführungsbeispiel des Reinigungs- und Desinfektionsgeräts 110 sind zwei optionale Merkmale verwirklicht, welche kumulativ oder auch jeweils einzeln in dem dargestellten Beispiel des Reinigungs- und Desinfektionsgeräts 110 oder auch in anderen erfindungsgemäßen Reinigungs- und Desinfektionsgeräten 110 zum Einsatz kommen können. So ist als erste Option wiederum ein Sensor 198 vorgesehen, und als zweite, alternativ oder zusätzlich einsetzbare Option, ist bei dem Ausführungsbeispiel gemäß Figur 6 ein Filterelement 612 vorgesehen. Beide dieser Elemente 198, 612 sind in dem dargestellten Ausführungsbeispiel in dem Desinfektionsleitungssystem 160 vorgesehen, wobei in dem dargestellten Ausführungsbeispiel eine Positionierung zwischen der Pumpe 161 und dem Verteiler 610 gezeigt ist. Alternativ oder zusätzlich ist jedoch auch eine Positionierung einer oder beider dieser Elemente 198, 612 an einer anderen Stelle denkbar, beispielsweise an einer anderen Stelle in dem Desinfektionsleitungssystem 160.

Der Sensor 198 ist in dem dargestellten Ausführungsbeispiel beispielsweise eingerichtet, um mindestens eine Eigenschaft der Wirklösung, die den Desinfektionswirkstoff umfasst, zu detektieren, insbesondere in dem Desinfektionsleitungssystem 160.

Der Sensor 198 ist in dem dargestellten Ausführungsbeispiel exemplarisch als optischer Sensor 614 ausgestaltet, beispielsweise als Absorptionssensor, welcher beispielsweise Absorptionseigenschaften der Wirklösung in dem Desinfektionsleitungssystem 160 erfassen kann. Zu diesem Zweck kann der optische Sensor 614 beispielsweise mindestens eine Durchflusszelle 616 umfassen, welche beispielsweise fluidisch in das Desinfektionsleitungssystem 160 integriert sein kann und welche von der Wirklösung durchströmt werden kann. Die Durchflusszelle kann beispielsweise ganz oder teilweise transparent ausgestaltet sein. Weiterhin kann der optische Sensor 614 mindestens eine Lichtquelle 618 und mindestens einen Fotodetektor 620 umfassen. Eine Verbindungslinie zwischen der Lichtquelle 618 und dem Fotodetektor 620 kann die Durchflusszelle 616 kreuzen, sodass von der Lichtquelle 618 emittiertes Licht die Durchflusszelle 616 passiert, dort mit der Wirklösung wechselwirkt und schließlich von dem Fotodetektor 620 detektiert wird. Die Wellenlänge der Lichtquelle 618 und/oder deren spektrale Eigenschaften können auf die Art der Wirklösung und/oder die Einsprechende zu detektierende Komponente angepasst werden. Beispielsweise kann für den oben beschriebenen Wirkstoff Peroxinitritsäure (ONOOH) bzw. Peroxinitrit (ONOO-) eine ultraviolette Lichtquelle 618 verwendet werden, beispielsweise eine im ultravioletten Spektralbereich emittierende Leuchtdiode. Alternativ oder zusätzlich zu Leuchtdioden können jedoch auch andere Arten von Lichtquellen zum Einsatz kommen, beispielsweise Laser, Glühlampen oder andere Arten von Lichtquellen. Der Fotodetektor 620 kann grundsätzlich mindestens ein beliebiges lichtempfindliches Element umfassen, welches beispielsweise auf die spektralen Eigenschaften der Lichtquelle 618 angepasst ist. Beispielsweise kann es sich hierbei um einen fotoempfindlichen Halbleiterdetektor handeln, beispielsweise eine Fotodiode, einen Fotowiderstand oder ähnliche fotoempfindliche Halbleiterdetektoren. Auch andere Arten von lichtempfindlichen Elementen können alternativ oder zusätzlich eingesetzt werden.

Die Lichtquelle 618 kann beispielsweise über mindestens eine elektrische Leitung 621 elektrisch mit einer Konstantstromquelle 622 verbunden sein und von dieser mit einem konstanten elektrischen Strom beaufschlagt werden. Die Art und Weise der elektrischen Ansteuerung der Lichtquelle 618 kann jedoch an den Typ der Lichtquelle 618 angepasst werden.

Der Fotodetektor 620 kann beispielsweise, wie ebenfalls in Figur 6 exemplarisch gezeigt, über mindestens eine elektrische Leitung 623 mit mindestens einem Signalwandler verbunden sein, beispielsweise mit mindestens einem Signalverstärker 624. Alternativ oder zusätzlich sind auch andere Arten der Signalwandlung möglich, beispielsweise Analog-Digital-Wandlungen oder ähnliche Arten der Signalwandlung.

Mindestens ein Sensorsignal des Sensors 198, beispielsweise des optischen Sensors 614, kann beispielsweise an die Steuerung 162 übermittelt werden. In dem exemplarischen Ausführungsbeispiel gemäß Figur 6 kann beispielsweise der optische Sensor 614 direkt oder auch über den Signalverstärker 624 drahtlos oder über mindestens eine elektrische Leitung 626 mit der Steuerung 162 verbunden sein.

Beispielsweise kann die Steuerung 162, wie in Figur 6 exemplarisch gezeigt, mehrteilig ausgebildet sein. So kann die Steuerung 162 beispielsweise mindestens eine Desinfektionssteuerung 628 umfassen, beispielsweise einen Mikrocontroller, welcher für die Steuerung des Desinfektionsschritts eingerichtet ist. Diese Desinfektionssteuerung 628 kann ganz oder teilweise als Softwarekomponente und/oder ganz oder teilweise auch als Hardwarekomponente ausgestaltet sein. Optional kann diese von einer, ebenfalls der Steuerung 162 zugehörigen, zentralen Maschinensteuerung 630 getrennt ausgebildet sein, welche beispielsweise die übrigen Funktionen des Reinigungs- und Desinfektionsgeräts 110 steuert, beispielsweise den Waschschritt. Die zentrale Maschinensteuerung 630, welche auch als CPU (Central Processing Unit) bezeichnet werden kann, kann beispielsweise über eine oder mehrere Zwischenkomponenten 632 mit der Desinfektionssteuerung 628 verbunden sein, beispielsweise über mindestens eine Eingabe-Ausgabe-Platine (E/A-Platine, auch als I/O-Platine bezeichnet).

Die Steuerung 162 kann, wie oben ausgeführt, eingerichtet sein, um entsprechend dem mindestens einen Sensorsignal des mindestens einen Sensors 198 entsprechende Steuer- und/oder Regelsignale zu generieren. So kann die Steuerung 162 beispielsweise drahtlos und/oder über mindestens eine Steuerleitung 634 elektrisch mit der Pumpe 161 und/oder mit anderen Komponenten verbunden sein. Auf diese Weise kann beispielsweise eine Regelstrecke erzeugt werden, um einen oder mehrere Parameter des Desinfektionsschritts anzusteuern, zu regeln oder anzupassen.

Wie weiterhin oben ausgeführt, ist in Figur 6 eine alternativ oder zusätzlich zu dem Sensorelement 198 realisierbare Option verwirklicht, bei welcher mindestens ein Filterelement 612 vorgesehen ist. Wie in der Figur erkennbar, sind weiterhin in diesem Ausführungsbeispiel stromaufwärts und stromabwärts des Filterelements 612 Drucksensoren 636, 638 vorgesehen, welche beispielsweise über Druckleitungen 640, 642 fluidisch mit dem Desinfektionsleitungssystem 160 stromaufwärts bzw. stromabwärts des Filterelements 612 verbunden sein können. Anstelle zweier Drucksensoren 636, 638 kann auch beispielsweise ein einzelner Differenzdrucksensor verwendet werden.

Sensorsignale der Drucksensoren 636, 638 können beispielsweise wiederum an die Steuerung 162 übermittelt werden, wie ebenfalls in Figur 6 dargestellt. Beispielsweise kann dies drahtlos erfolgen oder auch beispielsweise über elektrische Leitungen 644, 646. Wiederum kann dies beispielsweise an die spezielle Desinfektionssteuerung 628 erfolgen, welche für die Steuerung des Desinfektionsschritts zuständig ist.

Auf diese Weise kann durch die Steuerung 162 beispielsweise eine Überwachung der Funktion des mindestens einen Filterelements 612 erfolgen. Mit zunehmender Alterung des Filterelements 612 und/oder bei Verstopfung des Filterelements 612 kann beispielsweise der Differenzdruck, welcher über das Filterelement 612 abfällt, ansteigen, sodass mittels der Steuerung 162 beispielsweise diese Situation erkannt und beispielsweise eine entsprechende Warnung ausgegeben werden kann. Alternativ oder zusätzlich kann der Differenzdruck auch beispielsweise eine Information über die Zusammensetzung der Wirklösung in dem Desinfektionsleitungssystem 160 und/oder der mindestens einen Komponente in diesem Desinfektionsleitungssystem 160 liefern. So kann sich die Zusammensetzung beispielsweise auf die Viskosität auswirken, welche sich wiederum in dem Differenzdruck niederschlägt. Auch auf diese Weise kann beispielsweise mittels der Steuerung 162 mindestens ein entsprechendes Steuersignal erzeugt werden, beispielsweise um, durch eine entsprechende Anpassung der Zusammensetzung der Wirklösung, die Viskosität an einen Sollwert anzupassen.

Verschiedene Arten der Steuerung sind möglich.

Das in Figur 6 gezeigte Reinigungs- und Desinfektionsgerät 110 in einer oder mehreren der genannten Varianten lässt sich beispielsweise zur Behandlung oder Vermeidung von Sporen oder anderen Erregern einsetzen, wie oben diskutiert. Hierbei sind verschiedene Einsatzstrategien denkbar.

Die in Figur 6 gezeigte mehrteilige Ausgestaltung der Steuerung 162 kann insbesondere zu einer vorteilhaften Kommunikation genutzt werden. So kann die Steuerung beispielsweise als Hauptrechner-Nebenrechner-System (Master-Slave-System) ausgestaltet werden. Dabei kann beispielsweise die Desinfektionssteuerung 628, beispielsweise der Mikrocontroller, als Nebenrechner eingesetzt werden, während die zentrale Maschinensteuerung 630 beispielsweise als Hauptrechner fungieren kann. Die Desinfektionssteuerung 628 kann dann von der zentralen Maschinensteuerung 630 gesteuert werden. Die Kommunikation kann beispielsweise jedoch nicht direkt erfolgen, sondern, wie oben ausgeführt, optional über mindestens einen weiteren Baustein, in diesem Fall beispielsweise die mindestens eine Zwischenkomponente 632, beispielsweise die sogenannte Eingangs/Ausgangs (E/A) - Platine. Dies kann beispielsweise durch den Aufbau des Gesamtsystems des Reinigungs- und Desinfektionsgeräts 110, beispielsweise des Krankenhaus-Desinfektionsgeräts, bedingt sein.

Für die Kommunikation zwischen der Zwischenkomponente 632, insbesondere der E/A - Platine, und der zentralen Maschinensteuerung 630 oder CPU kann beispielsweise ein Bussystem 648 eingesetzt werden. Die Kommunikation zwischen der Zwischenkomponente 632, insbesondere der E/A - Platine, und der Desinfektionssteuerung 628, beispielsweise dem Mikrocontroller, kann beispielsweise über eine drahtlose Kommunikation und/oder über mindestens eine digitale Signalleitung 650 realisiert werden.

Für eine Kommunikationsrichtung von der Zwischenkomponente 632, insbesondere der E/A - Platine, hin zu der Desinfektionssteuerung 628, insbesondere dem Mikrocontroller, können insbesondere mindestens zwei separate Datenleitungen zur Verfügung stehen. Diese können beispielsweise an definierten Digitaleingängen der Desinfektionssteuerung 628, beispielsweise des Mikrocontrollers, eingelesen werden. Beispielsweise kann durch eine Umschaltung der Signalpegel (0 oder 1) auf den Signalleitungen und einer binären Auswertung in der Desinfektionssteuerung 628 unterschiedliche Zustände erfasst und/oder verarbeitet werden, beispielsweise vier oder zweiundzwanzig unterschiedliche Zustände. Die unterschiedlichen Zustände können beispielsweise als Startfreigabe, Stopp, Fehler oder ähnliche Befehle oder Informationen erfasst und/oder verarbeitet werden. Die Entscheidung über die Zustände der Ausgänge der Zwischenkomponente 632, insbesondere der E/A - Platine, und somit der Datenleitungen kann beispielsweise durch die zentrale Maschinensteuerung 630 oder CPU erfolgen.

Die umgekehrte Kommunikationsrichtung, also von der Desinfektionssteuerung 628, insbesondere dem Mikrocontroller, hin zu der Zwischenkomponente 632, insbesondere der E/A - Platine, kann analog oder nach demselben Prinzip erfolgen. Hierfür können jedoch beispielsweise mindestens vier separate Signalleitungen zur Verfügung stehen. Diese ermöglichen die Signalisierung von 16 oder 24 unterschiedlichen Zuständen, Ergebnissen, Füllständen, Fehlerzuständen oder ähnlichen Informationen. Damit die zentrale Maschinensteuerung 630 oder CPU über das weitere Vorgehen entscheiden kann, kann insbesondere eine Auswertung der an den Eingängen der Zwischenkomponente 632, insbesondere der E/A - Platine, anstehenden Signalpegel erfolgen. Das Ergebnis kann insbesondere anschließend mittels des Bussystems 648 an die zentrale Maschinensteuerung 630 oder CPU übertragen werden. Das Ergebnis kann dann insbesondere die Grundlage für weitere Entscheidungen bilden.

Mittels des in Figur 6 beschriebenen Aufbaus kann beispielsweise ein Messverfahren realisiert werden, welches beispielsweise der Prozessvalidierung des in dem Reinigungs- und Desinfektionsgerät 110 ablaufenden Verfahrens zur Behandlung des mindestens einen Behälters 112 dienen kann. Allgemein kann die Prozessvalidierung auch beispielsweise Bestandteil einer Dokumentation sein, beispielsweise in einem elektronischen Logbuch des Reinigungs- und Desinfektionsgeräts 110.

Zur Prozessvalidierung kann dabei insbesondere der Sensor 198, insbesondere der optische Sensor 614, eingesetzt werden. Dieser kann, wie oben ausgeführt, beispielsweise auf dem Einsatz einer Lichtquelle 618 in Form einer UV-Leuchtdiode (UV-LED) beruhen, einschließlich eines entsprechenden optoelektronischen Sensors oder Fotodetektors 620. Durch den Einsatz des Fotodetektors 620 kann das einfallende Licht in eine elektrische Größe umgewandelt werden.

Beispielsweise kann die Desinfektionssteuerung 628, insbesondere der Mikrocontroller, zu einem definierten Zeitpunkt, welcher beispielsweise von der zentralen Maschinensteuerung 630 oder CPU vorgegeben werden kann, eine Messung mittels des Sensors 198, insbesondere mittels des optischen Sensors 614, starten. Hierbei kann es sich insbesondere um eine Intensitätsmessung und/oder Absorptionsmessung handeln. Beispielsweise durch die sich zeitlich ändernden Absorptionseigenschaften der Wirkstofflösung, beispielsweise der Chemiebestandteile im Fluidgemisch, kann sich am Ausgang des Fotodetektors 620 ein bestimmter Ausgangsspannungsverlauf einstellen. Dieser Spannungsverlauf kann beispielsweise abhängig sein von der Zusammensetzung des Inhalts der Durchflusszelle 616 und/oder der Wirkstofflösung. Dies kann insbesondere bedeuten, dass sich in dem Fall, dass eine oder beide der Komponenten A und/oder B fehlen und/oder die Konzentration einer oder beider dieser Komponenten inkorrekt ist, insbesondere von Vorgaben abweicht, dieser Fehler in der Ausgangsspannung oder dem Signal des Fotodetektors 620 oder in dessen Verlauf widerspiegelt. Durch den Einsatz des optionalen Signalverstärkers 624, welcher beispielsweise das Ausgangssignal des Fotodetektors 620 um ein Vielfaches verstärkt, können beispielsweise bereits geringe Abweichungen sicher erkannt werden.

Die Steuerung 162, beispielsweise die Desinfektionssteuerung 628, kann beispielsweise überprüfen, ob das Sensorsignal des mindestens einen Sensors 198, beispielsweise das Signal des Fotodetektors 620, eine oder mehrere Vorgaben erfüllt und/oder kann dieses Signal als Eingangssignal für eine Steuerung oder Regelung einsetzen. Beispielsweise können IstWerte des Sensorsignals mit entsprechenden Werten verglichen werden, die in mindestens einer Datenbank hinterlegt sind. Aus der Auswertung des Sensorsignals können dann beispielsweise von der Steuerung 162 Entscheidungen über den Prozessablauf getroffen werden. Beispielsweise können entsprechende Steuersignale oder Regelsignale generiert werden, die beispielsweise über die Steuerleitung 634 übertragen werden können. Alternativ oder zusätzlich können Informationen und/oder Entscheidungen auch an die zentrale Maschinensteuerung 630 übermittelt werden, beispielsweise über die mindestens eine Zwischenkomponente 632. Dort können diese beispielsweise eingesetzt werden, um den Prozessablauf des Verfahrens zu beeinflussen.

Wie oben ausgeführt, können verschiedene Arten von Sensoren 198 in dem Reinigungs- und Desinfektionsgerät 110 vorgesehen sein. Weiterhin kann die Desinfektionssteuerung 628, insbesondere der Mikrocontroller, zusätzliche Aufgaben übernehmen, insbesondere Aufgaben im Zusammenhang mit dem Desinfektionsschritt. So kann beispielsweise, insbesondere aufgrund der typischerweise begrenzten Anzahl an Analogeingängen der Zwischenkomponente 632, beispielsweise an der mindestens einen E/A - Platine, die Desinfektionssteuerung 628 weitere Auswerteaufgaben übernehmen. So kann die Desinfektionssteuerung 628 beispielsweise mindestens einen Füllstand überwachen, beispielsweise in mindestens einem der Vorratsbehälter 154. Hierbei kann beispielsweise mindestens ein Füllstand in mindestens einem der Vorratsbehälter 154 und/oder in mindestens einem Aufbereitungsbehälter 188 überwacht werden, wobei beispielsweise auf das Beispiel in der Figur 2 verwiesen werden kann. Hierfür kann beispielsweise mindestens ein Füllstandssensor 200 zum Einsatz kommen, beispielsweise in mindestens einem Vorratsbehälter 154 und/oder in mindestens einem Aufbereitungsbehälter 188. Der Füllstandssensor 200 kann beispielsweise mindestens einen Leitfähigkeitssensor umfassen, sodass der Füllstand beispielsweise mittels einer oder mehrerer Leitwertelektroden erfasst werden kann. Diese können den Füllstand innerhalb des jeweiligen überwachten Behälters signalisieren. Auf diese Weise kann sichergestellt werden, dass für jeden Reinigungsprozess die benötigten Füllmengen vorhanden sind. Die Überwachung kann mittels der Desinfektionssteuerung 628 erfolgen.

Wie oben ausgeführt, können auch einer oder mehrere Drucksensoren, beispielsweise die Drucksensoren 636, 638, von der Desinfektionssteuerung 628 überwacht werden. Bei diesen Drucksensoren 636, 638 kann es sich beispielsweise um piezoresistive Drucksensoren handeln. Diese können ganz oder teilweise separat von dem Sensor 198 ausgestaltet sein oder können auch ganz oder teilweise in den Sensor 138, insbesondere den optischen Sensor 614, integriert sein, beispielsweise in ein Sensorgehäuse. Wie oben ausgeführt, können mittels der Drucksensoren 636, 638 vor und nach dem Filterelement 612 Druckveränderungen in dem Desinfektionsleitungssystem 160 erkannt und ausgewertet werden, beispielsweise in einem Schlauchsystem. Diese Druckverhältnisse können beispielsweise Aufschluss über verschiedene Systemzustände geben, beispielsweise über einen Zustand des Filterelements 612 selbst, über Eigenschaften der Wirkstofflösung oder auch über einen Zustand der Desinfektionsbeaufschlagungsvorrichtung 183, beispielsweise der Sprühdüsen 185. Steigt beispielsweise der Druck an, so kann dies beispielsweise ein Anzeichen sein für eine zunehmende Verunreinigung innerhalb der Desinfektionsbeaufschlagungsvorrichtung 183 und/oder innerhalb des Filterelements 612. Alternativ oder zusätzlich können, beispielsweise aus einem Differenzdruck, wie oben ausgeführt, Aussagen über eine Viskosität und/oder eine Zusammensetzung der Wirkstofflösung getroffen werden. Weiterhin können auch Vorzeichen des Differenzdrucks ausgewertet werden, da diese beispielsweise Ausschluss geben können über die Stelle einer Verunreinigung, beispielsweise stromaufwärts des Filterelements 612 oder stromabwärts desselben, insbesondere an den Sprühdüsen 185. Wird eine zunehmende Verunreinigung frühzeitig erkannt, so können, beispielsweise durch die Desinfektionssteuerung 628, entsprechende Maßnahmen ergriffen werden. Beispielsweise kann auf diese Weise eine unzureichende Reinigung und/oder eine unzureichende Desinfektion des Behälters 112 verhindert werden. So kann insbesondere dauerhaft sichergestellt werden, dass die verwendeten Wirkstoffe in der geforderten Menge an den Behälter 112 gelangen.

Bezugszeichenliste

110     Reinigungs- und Desinfektionsgerät
112     Behälter
114     Reinigungskammer
116     Abfluss
118     Geruchsverschluss

| 120 | Öffnung |
|---|---|
| 122 | Abflussrohr |
| 124 | Beaufschlagungsvorrichtung |
| 126 | Waschsystem |
| 128 | Waschdüse |
| 130 | Waschtank |
| 132 | Waschleitungssystem |
| 133 | Pumpe |
| 134 | Klarspülsystem |
| 136 | Klarspüldüse |
| 138 | Klarspültank |
| 140 | Klarspülleitungssystem |
| 142 | Dampfsystem |
| 144 | Dampfdüse |
| 146 | Dampferzeuger |
| 148 | Dampfleitung |
| 150 | Vorratsbehälter |
| 152 | Heizvorrichtung |
| 154 | Vorratsbehälter |
| 156 | Desinfektionssystem |
| 158 | Desinfektionsdüse |
| 160 | Desinfektionsleitungssystem |
| 161 | Pumpe |
| 162 | Steuerung |
| 164 | Datenverarbeitungsvorrichtung |
| 166 | Prozessor |
| 168 | Datenverbindung |
| 170 | Mischvorrichtung |
| 172 | Mischstrecke |
| 174 | Mischdüse |
| 176 | Tür |
| 178 | Klapptür |
| 180 | Halterung |
| 182 | Bypass |
| 183 | Desinfektionsbeaufschlagungsvorrichtung |
| 184 | Rohrleitung |
| 185 | Sprühdüse |
| 186 | Rückschlagventil |
| 187 | Vernebler |
| 188 | Aufbereitungsbehälter |
| 189 | Umwandlungsvorrichtung |
| 190 | Vorratsbehälter |
| 191 | Aufbereitungsrohr |
| 192 | Dosierpumpe |
| 193 | Pumpe |
| 194 | Zuleitung |
| 196 | Ventil |
| 198 | Sensor |
| 200 | Füllstandssensor |
| 202 | Leitfähigkeitssensor |
| 204 | Pumpe |
| 206 | Desinfektionspumpe |
| 208 | optische Sensor |
| 210 | Filter |
| 212 | Entleerungsschritt |
| 214 | Waschschritt |
| 216 | Desinfektionsschritt |
| 218 | Klarspülschritt |

220 Dampfdesinfektionsschritt

222 Verdrängungsschritt

224 Trocknungsschritt

510 nur Abspülen

512 90 ml Wirklösung und einer Einwirkzeit von 6 s

514 180 ml Wirklösung und einer Einwirkzeit von 12 s

516 Abreicherungsanteil

518 Desinfektionsanteil

520 Anforderung für EN 17126 und EN 13697

522 Nachweisgrenze

610 Verteiler

612 Filterelement

614 optischer Sensor

616 Durchflusszelle

618 Lichtquelle

620 Fotodetektor

621 elektrische Leitung

622 Konstantstromquelle

623 elektrische Leitung

624 Signalverstärker

626 elektrische Leitung

628 Desinfektionssteuerung

630 zentrale Maschinensteuerung

632 Zwischenkomponente

634 Steuerleitung

636 Drucksensor

638 Drucksensor

640 Druckleitung

642 Druckleitung

644 elektrische Leitung

646 elektrische Leitung

648 Bussystem

650 digitale Signalleitung

**Patentansprüche**

1. Verfahren zur Behandlung mindestens eines Behälters (112) für menschliche Ausscheidungen, umfassend folgende Schritte:

    a. mindestens einen Entleerungsschritt, umfassend ein Entleeren des Behälterinhalts innerhalb mindestens einer Reinigungskammer (114) in mindestens einen Abfluss (116), wobei der Abfluss (116) mindestens einen Geruchsverschluss (118) umfasst;
    b. mindestens einen Waschschritt, umfassend mindestens eine Beaufschlagung des Behälters (112) mit mindestens einer Reinigungsflüssigkeit in der Reinigungskammer (114); und
    c. mindestens einen Desinfektionsschritt, umfassend mindestens ein Mischen mindestens zweier reaktiver Komponenten zur Erzeugung mindestens eines Desinfektionswirkstoffs und Beaufschlagung des Behälters (112) mit dem Desinfektionswirkstoff;

    wobei das Verfahren eine *in-situ*-Bildung des Desinfektionswirkstoffs aus den mindestens zwei reaktiven Komponenten innerhalb eines Reinigungs- und Desinfektionsgeräts (110) beinhaltet, wobei der Desinfektionswirkstoff in dem Reinigungs- und Desinfektionsgerät (110) selbst gebildet wird.

2. Verfahren nach dem vorhergehenden Anspruch, weiterhin umfassend:
    d. mindestens einen Klarspülschritt, umfassend mindestens eine Beaufschlagung des Behälters (112) mit mindestens einer Klarspülflüssigkeit.

3. Verfahren nach einem der vorhergehenden Ansprüche, weiterhin umfassend:
    e. mindestens einen Dampfdesinfektionsschritt, umfassend mindestens eine Beaufschlagung des Behälters (112) mit Dampf.

4. Verfahren nach einem der vorhergehenden Ansprüche, wobei das Mischen in Schritt c. mindestens eine Vermischung der reaktiven Komponenten umfasst, ausgewählt aus der Gruppe bestehend aus:

- die reaktiven Komponenten werden vermischt, wobei der Desinfektionswirkstoff in der Mischung gebildet wird und die Mischung auf den Behälter (112) aufgebracht wird;
- die reaktiven Komponenten werden auf den Behälter (112) aufgebracht und auf dem Behälter (112) vermischt, wobei der Desinfektionswirkstoff in der Mischung auf dem Behälter (112) gebildet wird;
- mindestens eine erste der reaktiven Komponenten wird auf den Behälter (112) aufgebracht, und der Behälter (112) mit der darauf aufgebrachten ersten reaktiven Komponente wird in der Reinigungskammer (114) einer Atmosphäre ausgesetzt, welche mindestens eine zweite der reaktiven Komponenten umfasst, so dass sich die reaktiven Komponenten auf dem Behälter (112) vermischen, wobei der Desinfektionswirkstoff in der Mischung auf dem Behälter (112) entsteht.

5. Verfahren nach einem der vorhergehenden Ansprüche, wobei der Desinfektionswirkstoff in mindestens einem Trägerstoff enthalten ist, wobei der Desinfektionswirkstoff und der Trägerstoff eine Wirklösung bilden, wobei der Behälter (112) mit der Wirklösung beaufschlagt wird.

6. Verfahren nach einem der vorhergehenden Ansprüche, wobei die reaktiven Komponenten mindestens ein Oxidationsmittel und mindestens ein Anion einer Säure umfassen.

7. Verfahren nach einem der vorhergehenden Ansprüche, wobei der Desinfektionswirkstoff mindestens einen Wirkstoff umfasst, ausgewählt aus der Gruppe bestehend aus: einer reaktiven Stickstoffverbindung, insbesondere einer reaktiven Stickstoffverbindung ausgewählt aus der Gruppe bestehend aus: Peroxinitritsäure (ONOOH); Peroxinitrit (ONOO-); einer reaktiven Sauerstoffverbindung, insbesondere $H_2O_2$; einer Peroxycarbonsäure, insbesondere Peroxyessigsäure ($CH_3COOOH$); einem Anion einer einer Peroxycarbonsäure, insbesondere Peroxyessigsäure ($CH_3COOO^-$); und einer Chlorverbindung, insbesondere einer Chlorverbindung ausgewählt aus der Gruppe bestehend aus hypochloriger Säure (HClO), einem Anion der hypochlorigen Säure ($ClO^-$), chloriger Säure ($HClO_2$), einem Anion der chlorigen Säure ($ClO_2^-$), Chlorsäure ($HClO_3$), einem Anion der Chlorsäure ($ClO_3^-$), einem Chloroxid, insbesondere Chlordioxid.

8. Verfahren nach einem der vorhergehenden Ansprüche, wobei die reaktiven Komponenten mindestens eine Komponente umfassen, ausgewählt aus der Gruppe bestehend aus: Wasserstoffperoxid ($H_2O_2$); Ozon ($O_3$); $H^+$; und einer Säure, insbesondere mindestens eine Säure ausgewählt aus der Gruppe bestehend aus Zitronensäure, Phosphorsäure; Schwefelsäure, Salpetersäure, und Essigsäure.

9. Verfahren nach einem der vorhergehenden Ansprüche, wobei die reaktiven Komponenten mindestens eine Komponente umfassen, ausgewählt aus der Gruppe bestehend aus: Nitrat ($NO_3^-$); Nitrit ($NO_2^-$); einer Carbonsäure, insbesondere Essigsäure ($CH_3COOH$); einem Anion einer Carbonsäure, insbesondere Essigsäure ($CH_3COO^-$); Hypochlorit ($ClO^-$); Chlorit ($ClO_2^-$); und Chlorat ($ClO_3^-$).

10. Verfahren nach einem der vorhergehenden Ansprüche, weiterhin umfassend mindestens einen Verdrängungsschritt, wobei in dem Verdrängungsschritt Gase aus der Reinigungskammer (114) abgeleitet werden, wobei in dem Verdrängungsschritt die Gase aus der Reinigungskammer (114) durch mindestens einen Bypass (182) in den Abfluss (116) stromabwärts des Geruchsverschlusses (118) abgeleitet werden.

11. Verfahren nach einem der vorhergehenden Ansprüche, weiterhin umfassend mindestens einen Umwandlungsschritt, wobei in dem Umwandlungsschritt Gase aus der Reinigungskammer (114) mindestens einer Umwandlungsvorrichtung (189) zur chemischen und/oder physikalischen und/oder biologischen Aufbereitung zumindest eines Teils der Gase zugeführt werden.

12. Verfahren nach einem der vorhergehenden Ansprüche, wobei das Verfahren weiterhin die Verwendung mindestens eines Sensors umfasst, wobei der Sensor in mindestens einer Weise eingerichtet ist, ausgewählt aus der Gruppe bestehend aus:

a) der Sensor (198) ist eingerichtet, um mindestens eine Eigenschaft mindestens einer Komponente zu detektieren, ausgewählt aus der Gruppe bestehend aus: dem Desinfektionswirkstoff; mindestens einer den Desinfektionswirkstoff enthaltenden Wirklösung; mindestens einer der reaktiven Komponenten; mindestens einer der reaktiven Komponenten, gemischt mit mindestens einem Trägerstoff; mindestens einem bei einer Reaktion der reaktiven Komponenten entstehenden Nebenprodukt;

b) der Sensor (198) ist eingerichtet, um mindestens eine Eigenschaft mindestens eines Reaktionsprodukts innerhalb der Reinigungskammer (114) oder auf der Oberfläche des Behälters (112) zu detektieren, ausgewählt aus der Gruppe bestehend aus: einer entstehenden Säure; einem aus der Reaktion entstehenden Gas; einem Reaktionsnebenprodukt.

13. Verfahren nach dem vorhergehenden Anspruch, wobei in dem Desinfektionsschritt mindestens ein Parameter des Desinfektionsschritts entsprechend mindestens eines Sensorsignals des Sensors (198) beeinflusst wird.

14. Reinigungs- und Desinfektionsgerät (110) zur Behandlung mindestens eines Behälters (112) für menschliche Ausscheidungen, umfassend mindestens eine Reinigungskammer (114), weiterhin umfassend mindestens einen Abfluss (116) mit mindestens einem Geruchsverschluss (118), wobei das Reinigungs- und Desinfektionsgerät (110) weiterhin mindestens eine Beaufschlagungsvorrichtung (124) zur Beaufschlagung des Behälters (112) mit mindestens einem Reinigungsfluid in der Reinigungskammer (114) aufweist, wobei das Reinigungs- und Desinfektionsgerät (110) weiterhin mindestens zwei Vorratsbehälter (154) zur Aufnahme reaktiver Komponenten aufweist, wobei das Reinigungs- und Desinfektionsgerät (110) weiterhin mindestens eine Steuerung (162) zum Ansteuern mindestens eines Reinigungsprogramms aufweist, weiterhin umfassend mindestens eine Mischvorrichtung (170), wobei die Mischvorrichtung (170) eingerichtet ist, um die reaktiven Komponenten zu mischen, wobei das Reinigungs- und Desinfektionsgerät (110) eingerichtet ist, um das Verfahren nach einem der vorhergehenden Ansprüche durchzuführen.

15. Reinigungs- und Desinfektionsgerät (110) nach dem vorhergehenden Anspruch, weiterhin umfassend mindestens einen Sensor (198), wobei der Sensor (198) in mindestens einer Weise eingerichtet ist, ausgewählt aus der Gruppe bestehend aus:

a) der Sensor (198) ist eingerichtet, um mindestens eine Eigenschaft mindestens einer Komponente zu detektieren, ausgewählt aus der Gruppe bestehend aus: dem Desinfektionswirkstoff; mindestens einer den Desinfektionswirkstoff enthaltenden Wirklösung; mindestens einer der reaktiven Komponenten; mindestens einer der reaktiven Komponenten, gemischt mit mindestens einem Trägerstoff; mindestens einem bei einer Reaktion der reaktiven Komponenten entstehenden Nebenprodukt;

b) der Sensor (198) ist eingerichtet, um mindestens eine Eigenschaft mindestens eines Reaktionsprodukts innerhalb der Reinigungskammer (114) oder auf der Oberfläche des Behälters (112) zu detektieren, ausgewählt aus der Gruppe bestehend aus: einer entstehenden Säure; einem aus der Reaktion entstehenden Gas; einem Reaktionsnebenprodukt.

16. Reinigungs- und Desinfektionsgerät (110) nach dem vorhergehenden Anspruch, wobei der Sensor (198) zumindest teilweise in der Weise a) eingerichtet ist, wobei der Sensor (198) in mindestens einem von der Komponente durchströmten Leitungssystem (160) angeordnet ist.

17. Reinigungs- und Desinfektionsgerät (110) nach einem der beiden vorhergehenden Ansprüche, wobei der Sensor (198) mindestens einen optischen Absorptionssensor (614) umfasst.

18. Reinigungs- und Desinfektionsgerät (110) nach einem der drei vorhergehenden Ansprüche, wobei die Steuerung (162) eingerichtet ist, um den Desinfektionsschritt mittels mindestens eines Sensorsignals des Sensors (198) zu steuern.

19. Reinigungs- und Desinfektionsgerät (110) nach einem der vorhergehenden, ein Reinigungs- und Desinfektionsgerät (110) betreffenden Ansprüche, wobei das Reinigungs- und Desinfektionsgerät (110) eingerichtet ist, um die Beaufschlagungsvorrichtung (124) für den mindestens einen Waschschritt einzusetzen, wobei das Reinigungs- und Desinfektionsgerät (110) weiterhin mindestens eine Desinfektionsbeaufschlagungsvorrichtung (183) aufweist, wobei die Desinfektionsbeaufschlagungsvorrichtung (183) von der Beaufschlagungsvorrichtung (124) getrennt ausgebildet ist und wobei das Reinigungs- und Desinfektionsgerät (110) eingerichtet ist, um die Desinfektionsbeaufschlagungsvorrichtung (183) bei dem Desinfektionsschritt einzusetzen.

**Claims**

1. Method for treating at least one container (112) for human waste, comprising the following steps:

   a. at least one emptying step, comprising emptying of the container contents within at least one cleaning chamber (114) into at least one drain (116), wherein the drain (116) comprises at least one odour trap (118);
   b. at least one washing step, comprising at least one impingement of at least one cleaning liquid on the container (112) in the cleaning chamber (114); and
   c. at least one disinfection step, comprising at least one mixing of at least two reactive components to produce at least one disinfectant and impingement of the disinfectant on the container (112);

   wherein the method involves in situ formation of the disinfectant from the at least two reactive components within a washer-disinfector (110), wherein the disinfectant is formed in the washer-disinfector (110) itself.

2. Method according to the preceding claim, further comprising:
   d. at least one final-rinsing step, comprising at least one impingement of at least one rinse aid liquid on the container (112).

3. Method according to either of the preceding claims, further comprising:
   e. at least one steam disinfection step, comprising at least one impingement of steam on the container (112).

4. Method according to any of the preceding claims, wherein the mixing in step c. comprises at least one mixing of the reactive components selected from the group consisting of:

   - the reactive components are mixed, wherein the disinfectant is formed in the mixture and the mixture is applied to the container (112);
   - the reactive components are applied to the container (112) and mixed on the container (112), wherein the disinfectant is formed in the mixture on the container (112) ;
   - at least one first reactive component of the reactive components is applied to the container (112), and the container (112) with the first reactive component applied thereto is exposed in the cleaning chamber (114) to an atmosphere which comprises at least one second reactive component of the reactive components, such that the reactive components are mixed on the container (112), wherein the disinfectant is formed in the mixture on the container (112).

5. Method according to any of the preceding claims, wherein the disinfectant is contained in at least one carrier, wherein the disinfectant and the carrier form an active solution, wherein the container (112) is impinged on by the active solution.

6. Method according to any of the preceding claims, wherein the reactive components comprise at least one oxidizing agent and at least one anion of an acid.

7. Method according to any of the preceding claims, wherein the disinfectant comprises at least one active ingredient selected from the group consisting of: a reactive nitrogen compound, in particular a reactive nitrogen compound selected from the group consisting of: peroxynitric acid (ONOOH); peroxynitrite (ONOO-); a reactive oxygen compound, in particular $H_2O_2$; a peroxycarboxylic acid, in particular peroxyacetic acid ($CH_3COOOH$); an anion of a peroxycarboxylic acid, in particular peroxyacetic acid ($CH_3COOO^-$); and a chlorine compound, in particular a chlorine compound selected from the group consisting of hypochlorous acid (HClO), an anion of hypochlorous acid ($ClO^-$), chlorous acid ($HClO_2$), an anion of chlorous acid ($ClO_2^-$), chloric acid ($HClO_3$), an anion of chloric acid ($ClO_3^-$), a chlorine oxide, in particular chlorine dioxide.

8. Method according to any of the preceding claims, wherein the reactive components comprise at least one component selected from the group consisting of: hydrogen peroxide ($H_2O_2$); ozone ($O_3$); H+; and an acid, in particular at least one acid selected from the group consisting of citric acid, phosphoric acid, sulfuric acid, nitric acid and acetic acid.

9. Method according to any of the preceding claims, wherein the reactive components comprise at least one component selected from the group consisting of: nitrate ($NO_3^-$); nitrite ($NO_2^-$); a carboxylic acid, in particular acetic acid ($CH_3COOH$); an anion of a carboxylic acid, in particular acetic acid ($CH_3COO^-$); hypochlorite ($ClO^-$); chlorite ($ClO_2^-$); and chlorate ($ClO_3^-$).

10. Method according to any of the preceding claims, further comprising at least one displacement step, wherein the displacement step comprises discharging gases from the cleaning chamber (114), wherein the displacement step comprises discharging the gases from the cleaning chamber (114) through at least one bypass (182) into the drain (116) downstream of the odour trap (118).

11. Method according to any of the preceding claims, further comprising at least one conversion step, wherein the conversion step comprises supplying gases from the cleaning chamber (114) to at least one conversion device (189) for chemical and/or physical and/or biological processing of at least a portion of the gases.

12. Method according to any of the preceding claims, wherein the method further comprises the use of at least one sensor, wherein the sensor is arranged in at least one way selected from the group consisting of:

   a) the sensor (198) is arranged to detect at least one property of at least one component selected from the group consisting of: the disinfectant; at least one active solution containing the disinfectant; at least one of the reactive components; at least one of the reactive components mixed with at least one carrier; at least one by-product formed in a reaction of the reactive components;
   b) the sensor (198) is arranged to detect at least one property of at least one reaction product within the cleaning chamber (114) or on the surface of the container (112) selected from the group consisting of: an acid which is formed; a gas formed from the reaction; a reaction by-product.

13. Method according to the preceding claim, wherein the disinfection step comprises influencing at least one parameter of the disinfection step in accordance with at least one sensor signal of the sensor (198).

14. Washer-disinfector (110) for treating at least one container (112) for human waste, comprising at least one cleaning chamber (114), further comprising at least one drain (116) having at least one odour trap (118), wherein the washer-disinfector (110) further comprises at least one impingement device (124) for impingement of at least one cleaning fluid on the container (112) in the cleaning chamber (114), wherein the washer-disinfector (110) further comprises at least two reservoirs (154) for accommodation of reactive components, wherein the washer-disinfector (110) further comprises at least one controller (162) for control of at least one cleaning program, further comprising at least one mixing device (170), wherein the mixing device (170) is arranged to mix the reactive components, wherein the washer-disinfector (110) is arranged to carry out the method according to any of the preceding claims.

15. Washer-disinfector (110) according to the preceding claim, further comprising at least one sensor (198), wherein the sensor (198) is arranged in at least one way selected from the group consisting of:

   a) the sensor (198) is arranged to detect at least one property of at least one component selected from the group consisting of: the disinfectant; at least one active solution containing the disinfectant; at least one of the reactive components; at least one of the reactive components mixed with at least one carrier; at least one by-product formed in a reaction of the reactive components;
   b) the sensor (198) is arranged to detect at least one property of at least one reaction product within the cleaning chamber (114) or on the surface of the container (112) selected from the group consisting of: an acid which is formed; a gas formed from the reaction; a reaction by-product.

16. Washer-disinfector (110) according to the preceding claim, wherein the sensor (198) is at least partly arranged in the way a), wherein the sensor (198) is disposed in at least one line system (160) through which the component flows.

17. Washer-disinfector (110) according to either of the two preceding claims, wherein the sensor (198) comprises at least one optical absorption sensor (614).

18. Washer-disinfector (110) according to any of the three preceding claims, wherein the controller (162) is arranged to control the disinfection step by means of at least one sensor signal of the sensor (198).

19. Washer-disinfector (110) according to any of the preceding claims relating to a washer-disinfector (110), wherein the washer-disinfector (110) is arranged to use the impingement device (124) for the at least one washing step, wherein the washer-disinfector (110) further comprises at least one disinfection impingement device (183), wherein the disinfection impingement device (183) is separate from the impingement device (124) and wherein the washer-disinfector (110) is arranged to use the disinfection impingement device (183) in the disinfection step.

**Revendications**

1. Procédé destiné à traiter au moins un récipient (112) pour des excrétions humaines, comprenant les étapes suivantes :

   a. au moins une étape de vidage, comprenant un vidage du contenu du récipient à l'intérieur d'au moins une chambre de nettoyage (114) dans au moins une évacuation (116), l'évacuation (116) comprenant au moins siphon (118) ;
   b. au moins une étape de lavage, comprenant au moins une sollicitation du récipient (112) par au moins un liquide de nettoyage dans la chambre de nettoyage (114) ; et
   c. au moins une étape de désinfection, comprenant au moins un mélange d'au moins deux composants réactifs afin de générer au moins un agent actif de désinfection et une sollicitation du récipient (112) par l'agent actif de désinfection ;

   le procédé comportant une formation in situ de l'agent actif de désinfection à partir desdits au moins deux composants réactifs à l'intérieur d'un appareil de nettoyage et de désinfection (110), l'agent actif de désinfection étant formé dans l'appareil de nettoyage et de désinfection (110) lui-même.

2. Procédé selon la revendication précédente, comprenant en outre :
   d. au moins une étape de rinçage, comprenant au moins une sollicitation du récipient (112) par au moins un liquide de rinçage.

3. Procédé selon l'une des revendications précédentes, comprenant en outre :
   e. au moins une étape de désinfection à la vapeur, comprenant au moins une sollicitation du récipient (112) par de la vapeur.

4. Procédé selon l'une des revendications précédentes, le mélange dans l'étape c. comprenant au moins un mélange des composants réactifs, choisi dans le groupe constitué par :

   - les composants réactifs sont mélangés, l'agent actif de désinfection étant formé dans le mélange et le mélange étant appliqué sur le récipient (112) ;
   - les composants réactifs sont appliqués sur le récipient (112) et mélangés sur le récipient (112), l'agent actif de désinfection étant formé dans le mélange sur le récipient (112) ;
   - au moins un premier des composants réactifs est appliqué sur le récipient (112) et le récipient (112) présentant le premier composant réactif appliqué sur celui-ci est soumis, dans la chambre de nettoyage (114), à une atmosphère qui comprend au moins un deuxième des composants réactifs de telle sorte que les composants réactifs se mélangent sur le récipient (112), l'agent actif de désinfection se formant dans le mélange sur le récipient (112).

5. Procédé selon l'une des revendications précédentes, l'agent actif de désinfection étant contenu dans au moins une substance support, l'agent actif de désinfection et la substance support formant une solution active, le récipient (112) étant sollicité par la solution active.

6. Procédé selon l'une des revendications précédentes, les composants réactifs comprenant au moins un agent d'oxydation et au moins un anion d'un acide.

7. Procédé selon l'une des revendications précédentes, l'agent actif de désinfection comprenant au moins une substance active choisie dans le groupe constitué par : un composé azoté réactif, en particulier un composé azoté réactif choisi dans le groupe constitué par : l'acide peroxynitrique (ONOOH) ; le peroxynitrite (ONOO⁻) ; un composé réactif de l'oxygène, en particulier $H_2O_2$ ; un acide peroxycarboxylique, en particulier l'acide peroxyacétique ($CH_3COOOH$) ; un anion d'un acide peroxycarboxylique, en particulier de l'acide peroxyacétique ($CH_3COOO^-$) ; et un composé chloré, en particulier un composé chloré choisi dans le groupe constitué par l'acide hypochloreux (HClO), un anion de l'acide hypochloreux (ClO⁻), l'acide chloreux ($HClO_2$), un anion de l'acide chloreux ($ClO_2^-$), l'acide chlorique ($HClO_3$), un anion de l'acide chlorique ($ClO_3^-$), un oxyde de chlore, en particulier le dioxyde de chlore.

8. Procédé selon l'une des revendications précédentes, les composants réactifs comprenant au moins un composant choisi dans le groupe constitué par : le peroxyde d'hydrogène ($H_2O_2$) ; l'ozone ($O_3$) ; H⁺ ; et un acide, en particulier un acide choisi dans le groupe constitué par l'acide citrique, l'acide phosphorique, l'acide sulfurique, l'acide nitrique et l'acide acétique.

9. Procédé selon l'une des revendications précédentes, les composants réactifs comprenant au moins un composant choisi dans le groupe constitué par : le nitrate ($NO_3^-$) ; le nitrite ($NO_2^-$) ; un acide carboxylique, en particulier l'acide acétique ($CH_3COOH$) ; un anion d'un acide carboxylique, en particulier de l'acide acétique ($CH_3COO^-$) ; l'hypochlorite ($ClO^-$) ; le chlorite ($ClO_2^-$) ; et le chlorate ($ClO_3^-$).

10. Procédé selon l'une des revendications précédentes, comprenant en outre au moins une étape de déplacement, dans lequel, dans l'étape de déplacement, des gaz sont évacués de la chambre de nettoyage (114), dans lequel, dans l'étape de déplacement, les gaz sont évacués de la chambre de nettoyage (114) à travers au moins une dérivation (182) dans l'évacuation (116) en aval du siphon (118).

11. Procédé selon l'une des revendications précédentes, comprenant en outre au moins une étape de transformation, dans lequel, dans l'étape de transformation, des gaz provenant de la chambre de nettoyage (114) sont introduits au moins dans un dispositif de transformation (189) pour le traitement chimique et/ou physique et/ou biologique d'au moins une partie des gaz.

12. Procédé selon l'une des revendications précédentes, le procédé comprenant en outre l'utilisation d'au moins un capteur, le capteur étant conçu d'au moins une manière choisie dans le groupe constitué par :

   a) le capteur (198) est conçu pour détecter au moins une propriété d'au moins un composant, choisi dans le groupe constitué par : l'agent actif de désinfection ; au moins une solution active contenant l'agent actif de désinfection ; au moins l'un des composants réactifs ; au moins l'un des composants réactifs mélangé avec au moins une substance support ; au moins un produit secondaire formé lors d'une réaction des composants réactifs ;
   b) le capteur (198) est conçu pour détecter au moins une propriété d'au moins un produit de réaction à l'intérieur de la chambre de nettoyage (114) ou sur la surface du récipient (112), choisi dans le groupe constitué par : un acide qui se forme ; un gaz qui se forme à partir de la réaction ; un produit secondaire de la réaction.

13. Procédé selon la revendication précédente, dans lequel, dans l'étape de désinfection, au moins un paramètre de l'étape de désinfection est influencé conformément à au moins un signal de capteur du capteur (198).

14. Appareil de nettoyage et de désinfection (110) destiné à traiter au moins un récipient (112) pour des excréments humains, comprenant au moins une chambre de nettoyage (114), comprenant en outre au moins une évacuation (116) présentant au moins un siphon (118), l'appareil de nettoyage et de désinfection (110) présentant en outre au moins un dispositif de sollicitation (124) destiné à solliciter le récipient (112) par au moins un fluide de nettoyage dans la chambre de nettoyage (114), l'appareil de nettoyage et de désinfection (110) présentant en outre au moins deux réservoirs (154) destinés à recevoir des composants réactifs, l'appareil de nettoyage et de désinfection (110) présentant en outre au moins une commande (162) destinée à commander au moins un programme de nettoyage, comprenant en outre au moins un dispositif de mélange (170), le dispositif de mélange (170) étant conçu pour mélanger les composants réactifs, l'appareil de nettoyage et de désinfection (110) étant conçu pour mettre en œuvre le procédé selon l'une des revendications précédentes.

15. Appareil de nettoyage et de désinfection (110) selon la revendication précédente, comprenant en outre au moins un capteur (198), le capteur (198) étant conçu d'au moins une manière choisie dans le groupe constitué par :

   a) le capteur (198) est conçu pour détecter au moins une propriété d'au moins un composant, choisi dans le groupe constitué par : l'agent actif de désinfection ; au moins une solution active contenant l'agent actif de désinfection ; au moins l'un des composants réactifs ; au moins l'un des composants réactifs mélangé avec au moins une substance support ; au moins un produit secondaire formé lors d'une réaction des composants réactifs ;
   b) le capteur (198) est conçu pour détecter au moins une propriété d'au moins un produit de réaction à l'intérieur de la chambre de nettoyage (114) ou sur la surface du récipient (112), choisi dans le groupe constitué par : un acide qui se forme ; un gaz qui se forme à partir de la réaction ; un produit secondaire de la réaction.

16. Appareil de nettoyage et de désinfection (110) selon la revendication précédente, le capteur (198) étant conçu au moins partiellement de la manière a), le capteur (198) étant agencé dans au moins un système de conduits (160) traversé par les composants.

**17.** Appareil de nettoyage et de désinfection (110) selon l'une des deux revendications précédentes, le capteur (198) comprenant au moins un capteur d'absorption optique (614).

**18.** Appareils de nettoyage et de désinfection (110) selon l'une des trois revendications précédentes, la commande (162) étant conçue pour commander l'étape de désinfection au moyen d'au moins un signal de capteur du capteur (198).

**19.** Appareil de nettoyage et de désinfection (110) selon l'une des revendications précédentes concernant un appareil de nettoyage et de désinfection (110), l'appareil de nettoyage et de désinfection (110) étant conçu pour utiliser le dispositif de sollicitation (124) pour ladite au moins une étape de lavage, l'appareil de nettoyage et de désinfection (110) présentant en outre au moins un dispositif de sollicitation pour la désinfection (183), le dispositif de sollicitation pour la désinfection (183) étant réalisé séparément du dispositif de sollicitation (124) et l'appareil de nettoyage et de désinfection (110) étant conçu pour utiliser le dispositif de sollicitation pour la désinfection (183) lors de l'étape de désinfection.

FIG. 1

FIG. 2

FIG. 3

FIG. 4

FIG. 5

FIG. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10348344 A1
- WO 2013037723 A1
- WO 2019219220 A1
- WO 2019219959 A1
- US 20180058053 A1
- WO 2019036828 A1
- US 20170260728 A1